# EUROPÄISCHE PATENTSCHRIFT

(11) **EP 2 121 597 B1**
(45) Veröffentlichungstag und Bekanntmachung des Hinweises auf die Patenterteilung: **21.09.2011**
(21) Anmeldenummer: 07818979.2
(22) Anmeldetag: 13.10.2007
(51) Int. Cl.: C07D 207/38, A01N 43/36

(54) **TRIFLUORMETHOXY-PHENYLSUBSTITUIERTE TETRAMSÄURE-DERIVATE ALS SCHÄDLINGSBEKÄMPFUNGSMITTEL UND/ODER HERBIZIDE**
TRIFLUOROMETHOXYPHENYL-SUBSTITUTED TETRAMIC ACID DERIVATIVES AS PESTICIDES AND/OR HERBICIDES
DÉRIVÉS D'ACIDE TÉTRAMIQUE TRIFLUOROMÉTHOXY-PHÉNYLSUBSTITUÉS EN TANT QUE PESTICIDES ET/OU HERBICIDES

(30) Priorität: 25.10.2006 DE 102006050148
(43) Veröffentlichungstag der Anmeldung: 25.11.2009
(73) Patentinhaber: Bayer CropScience AG, 40789 Monheim (DE)
(72) Erfinder: FISCHER, Rainer, 40789 Monheim (DE); LEHR, Stefan, 65835 Liederbach (DE); ARNOLD, Christian, 40764 Langenfeld (DE); AULER, Thomas, 42799 Leichlingen (DE); DITTGEN, Jan, 60316 Frankfurt/M. (DE); FEUCHT, Dieter, 65750 Eschborn (DE); FRANKEN, Eva-Maria, 42799 Leichlingen (DE); HEMPEL, Waltraud, 65835 Liederbach (DE); HILLS, Martin Jeffrey, 65510 Idstein (DE); KEHNE, Heinz, 65719 Hofheim (DE); LÖSEL, Peter, 51371 Leverkusen (DE); MALSAM, Olga, 51503 Rösrath (DE); ROSINGER, Christopher Hugh, 65719 Hofheim (DE); SANWALD, Erich, 24159 Kiel (DE); GÖRGENS, Ulrich, 40882 Ratingen (DE); ANTONS, Stefan, 51373 Leverkusen (DE); EBENBECK, Wolfgang, 51373 Leverkusen (DE); PLESCHKE, Axel, 79238 Ehrenkirchen 1 (DE); SCHNEIDER, Marielouise, 51375 Leverkusen (DE); WISCHNAT, Ralf, 51467 Bergisch Gladbach (DE)
(86) Internationale Anmeldenummer: PCT/EP2007/008908
(87) Internationale Veröffentlichungsnummer: WO 2008/067873

(56) Entgegenhaltungen:
- EP-A- 0 854 134
- WO-A-2006/007998
- WO-A-2006/056281

## Beschreibung

Die vorliegende Erfindung betrifft neue Trifluormethoxy-phenylsubstituierte Tetramsäure-Derivate, mehrere Verfahren zu ihrer Herstellung und ihre Verwendung als Schädlingsbekämpfungsmittel und/oder Herbizide. Auch Gegenstand der Erfindung sind selektiv herbizide Mittel, die Trifluormethoxy-phenylsubstituierte Tetramsäure-Derivate einerseits und eine die Kulturpflanzen-verträglichkeit verbessernde Verbindung andererseits enthalten.

Die vorliegende Erfindung betrifft weiterhin die Steigerung der Wirkung von Pflanzenschutzmitteln enthaltend insbesondere Trifluormethoxy-phenylsubstituierte Tetramsäure-Derivate, durch die Zugabe von Ammonium- oder Phosphoniumsalzen und gegebenenfalls Penetrationsförderem, die entsprechenden Mittel, Verfahren zu ihrer Herstellung und ihre Anwendung im Pflanzenschutz als Insektizide und/oder Akarizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Winkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01997, WO 95/26954, WO 95/20572, EP-A-0 668 267, WO 96/25395, WO 96/35664, WO 97/01535, WO 97/02243, WO 97/36868, WO 97/43275, WO 98/05638, WO 98/06721, WO 98/25928, WO 99/16748, WO 99/24437, WO 99/43649, WO 99/48869, WO 99/55673, WO 01/17972, WO 01/23354, WO 01/74770, WO 03/013249, WO 04/007448, WO 04/024688, WO 04/065366, WO 04/080962, WO 04/111042, WO 05/044791, WO 05/044796, WO 05/048710, WO 05/049596, WO 05/066125, WO 05/092897, WO 06/000355 WO 06/029799, WO 06/056281, WO 06/056282, WO 06/089633,WO 07/048545. WO 07/073856, DE-A-05/059892, DE-A-06/007882, DE-A-06/018828, DE-A-06/025874.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen gegenüber manchen Kulturpflanzen nicht immer ausreichend.

Es wurden nun neue Verbindungen der Formel (I) gefunden 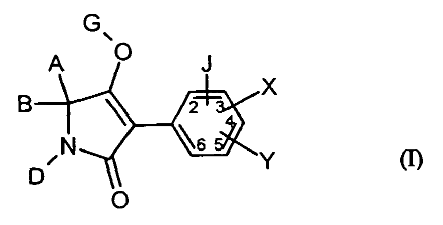 in welcher
- J: für Trifluormethoxy steht,
- X: für Wasserstoff, Alkyl, Halogen, Halogenalkyl, Alkoxy oder Halogenalkoxy steht,
- Y: für Wasserstoff, Alkyl, Alkoxy oder Halogen steht,
mit der Maßgabe, dass mindestens einer der Reste J, X oder Y in der 2-Position des Phenylrestes steht und dabei ungleich Wasserstoff ist,
- A: für Wasserstoff, jeweils gegebenenfalls durch Halogen substituiertes Alkyl, Alkenyl, Alkoxyalkyl, Alkylthioalkyl, gesättigtes oder ungesättigtes, gegebenen- falls substituiertes Cycloalkyl, in welchem gegebenenfalls mindestens ein Ring- atom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind, für einen ge- sättigten oder ungesättigten, gegebenenfalls mindestens ein Heteroatom ent- haltenden unsubstituierten oder substituierten Cyclus stehen,
- D: für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
- A und D: gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen.
- G: für Wasserstoff (a) oder für eine der Gruppen 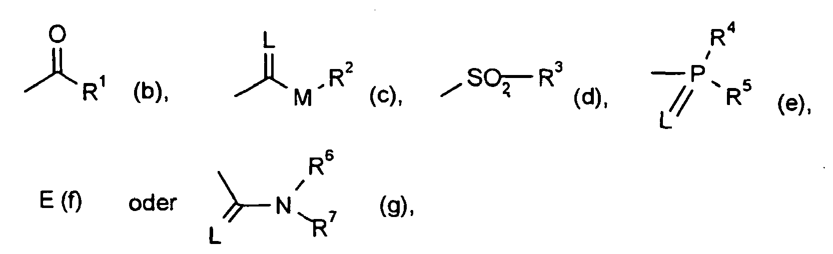
steht,
worin
- E: für ein Metallionäquivalent oder ein Ammoniumion steht,
- L: für Sauerstoff oder Schwefel steht,
- M: für Sauerstoff oder Schwefel steht,
- R¹: für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryl- oxyalkyl steht,
- R²: für jeweils gegebenenfalls durch Halogen substituiertes Alkyl, Alkenyl, Alkoxyalkyl, Polyalkoxyalkyl oder für jeweils gegebenenfalls sub- stituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alke- nylthio, Cycloalkylthio oder für jeweils gegebenenfalls substituiertes Phe- nyl, Benzyl, Phenoxy oder Phenylthio stehen,
- R⁶ und R⁷: unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbro- chenen Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g), 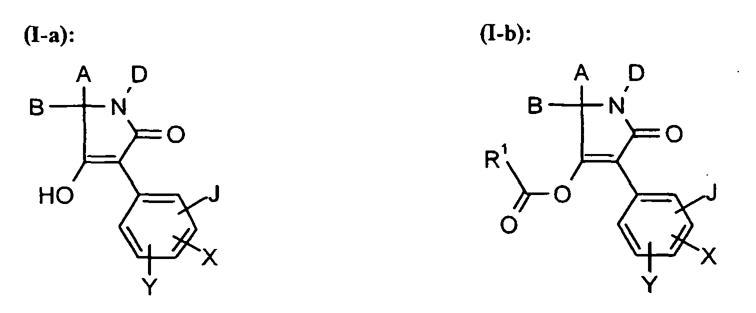 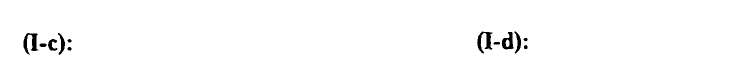 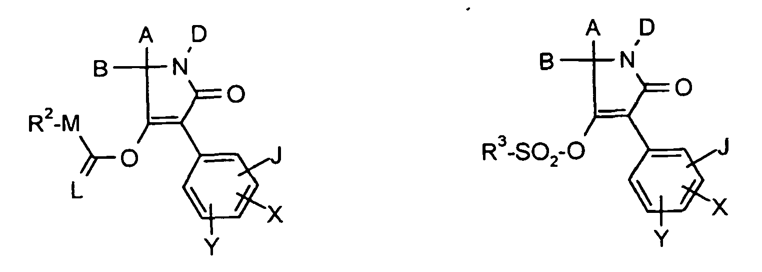 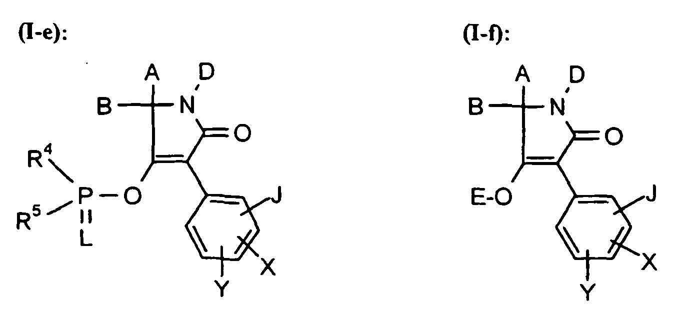 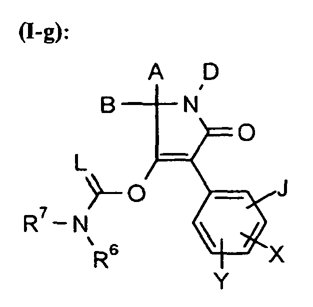 worin
A, B, D, E, J, L, M, X, Y, R¹, R², R³, R⁴, R⁵, R⁶ und R⁷ die oben angegebenen Bedeutungen besitzen.
Weiterhin wurde gefunden, dass man die neuen Verbindungen der Formel (I) nach einem der im folgenden beschriebenen Verfahren erhält:

### (A) Man erhält Verbindungen der Formel (I-a)

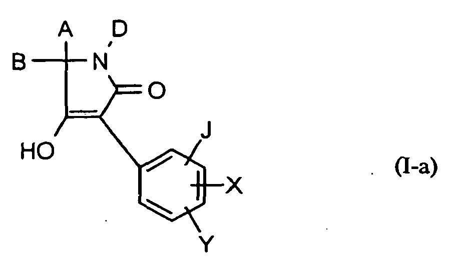

in welcher
- A, B, D, J, X und Y: die oben angegebenen Bedeutungen haben,
wenn man
N-Acylaminosäureester der Formel (II) 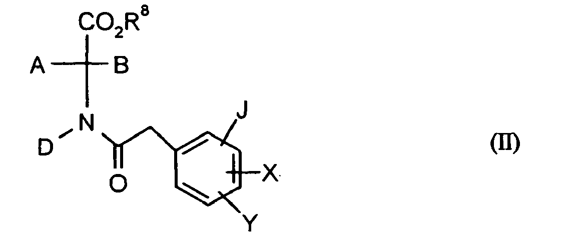 in welcher
- A, B, D, J, X und Y: die oben angegebenen Bedeutungen haben,
und
- R⁸: für Alkyl (bevorzugt C₁-C₆-Alkyl) steht,
in Gegenwart eines Verdünnungsmittels und in Gegenwart einer Base intramolekular kondensiert.

Weiterhin wurde gefunden,
(B) dass man die Verbindungen der oben gezeigten Formel (I-b), in welcher A, B, D, J, R¹, X, und Y die oben angegebenen Bedeutungen haben, erhält, wenn man Verbindungen der oben gezeigten Formel (I-a), in welcher A, B, D, J, X und Y die oben angegebenen Bedeutungen haben, jeweils

### (α) mit Säurehalogeniden der Formel (III)

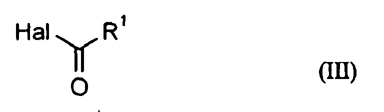

in welcher
- R¹: die oben angegebene Bedeutung hat und

- Hal: für Halogen (insbesondere Chlor oder Brom) steht
oder

### (β) mit Carbonsäureanhydriden der Formel (IV)

R¹-CO-O-CO-R¹ (IV)

in welcher
- R¹: die oben angegebene Bedeutung hat,
gegebenenfalls in Gegenwart eines Verdünnungsmittels und gegebenenfalls in Gegenwart eines Säurebindemittels umsetzt;
(C) dass man die Verbindungen der oben gezeigten Formel (I-c), in welcher A, B, D, J, R², M, X und Y die oben angegebenen Bedeutungen haben und L für Sauerstoff steht, erhält, wenn man Verbindungen der oben gezeigten Formel (I-a), in welcher A, B, D, J, X und Y die oben angegebenen Bedeutungen haben, jeweils
mit Chlorameisensäureestern oder Chlorameisensäurethioestem der Formel (V)

R²-M-CO-Cl (V)

in welcher
R² und M die oben angegebenen Bedeutungen haben,
gegebenenfalls in Gegenwart eines Verdünnungsmittels und gegebenenfalls in Gegenwart eines Säurebindemittels umsetzt;
(D) dass man Verbindungen der oben gezeigten Formel (I-c), in welcher A, B, D, J, R², M, X und Y die oben angegebenen Bedeutungen haben und L für Schwefel steht, erhält, wenn man Verbindungen der oben gezeigten Formel (I-a), in welcher A, B, D, J, X und Y die oben angegebenen Bedeutungen haben, jeweils
mit Chlormonothioameisensäureestem oder Chlordithioameisensäureestem der Formel (VI) 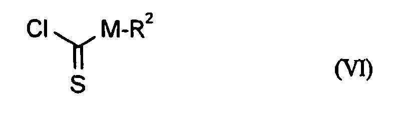 in welcher
- M und R²: die oben angegebenen Bedeutungen haben,
gegebenenfalls in Gegenwart eines Verdünnungsmittels und gegebenenfalls in Gegenwart eines Säurebindemittels umsetzt
und
(E) dass man Verbindungen der oben gezeigten Formel (I-d), in welcher A, B, D, J, R³, X und Y die oben angegebenen Bedeutungen haben, erhält, wenn man Verbindungen der oben gezeigten Formel (I-a), in welcher A, B, D, J, X und Y die oben angegebenen Bedeutungen haben, jeweils
mit Sulfonsäurechloriden der Formel (VII)

R³-SO₂-Cl (VII)

in welcher
- R³: die oben angegebene Bedeutung hat,
gegebenenfalls in Gegenwart eines Verdünnungsmittels und gegebenenfalls in Gegenwart eines Säurebindemittels umsetzt,
(F) dass man Verbindungen der oben gezeigten Formel (I-e), in welcher A, B, D, J, L, R⁴, R⁵, X und Y die oben angegebenen Bedeutungen haben, erhält, wenn man Verbindungen der oben gezeigten Formel (I-a), in welcher A, B, D, J, X und Y die oben angegebenen Bedeutungen haben, jeweils
mit Phosphorverbindungen der Formel (VIII) 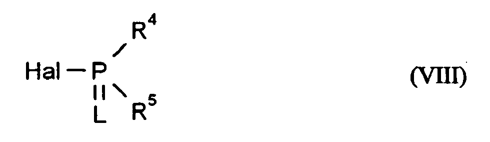 in welcher
- L, R⁴ und R⁵: die oben angegebenen Bedeutungen haben und
- Hal: für Halogen (insbesondere Chlor oder Brom) steht, gegebenenfalls in Gegenwart eines Verdünnungsmittels und gegebenenfalls in Gegenwart eines Säurebindemittels umsetzt,
- (G): dass man Verbindungen der oben gezeigten Formel (I-f), in welcher A, B, D, E, J, X und Y die oben angegebenen Bedeutungen haben, erhält, wenn man Verbindungen der Formel (I- a), in welcher A, B, D, J, X und Y die oben angegebenen Bedeutungen haben, jeweils
mit Metallverbindungen oder Aminen der Formeln (IX) oder (X)

Me(OR¹⁰)ₜ (IX)

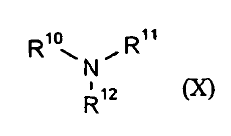

in welchen
- Me: für ein ein- oder zweiwertiges Metall (bevorzugt ein Alkali- oder Erdalkalimetall wie Lithium, Natrium, Kalium, Magnesium oder Calcium), oder für ein Ammoniumion

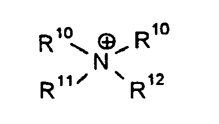

- t: für die Zahl 1 oder 2 und
- R¹⁰, R¹¹, R¹²: unabhängig voneinander für Wasserstoff oder Alkyl (bevorzugt C₁-C₈- Alkyl) stehen,
gegebenenfalls in Gegenwart eines Verdünnungsmittels umsetzt,
(H) dass man Verbindungen der oben gezeigten Formel (I-g), in welcher A, B, D, J, L, R⁶, R⁷, X und Y die oben angegebenen Bedeutungen haben, erhält, wenn man Verbindungen der oben gezeigten Formel (I-a), in welcher A, B, D, J, X und Y die oben angegebenen Bedeutungen haben, jeweils

### (α) mit Isocyanaten oder Isothiocyanaten der Formel (XI)

R⁶-N=C=L (XI)

in welcher
- R⁶ und L: die oben angegebenen Bedeutungen haben,

gegebenenfalls in Gegenwart eines Verdünnungsmittels und gegebenenfalls in Gegenwart eines Katalysators umsetzt oder

### (ß) mit Carbamidsäurechloriden oder Thiocarbamidsäurechloriden der Formel (XII)

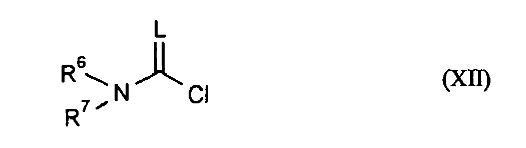

in welcher
- L, R⁶ und R⁷: die oben angegebenen Bedeutungen haben,
gegebenenfalls in Gegenwart eines Verdünnungsmittels und gegebenenfalls in Gegenwart eines Säurebindemittels, umsetzt.

Folgende Verbindungen der Formel (I) sind im Rahmen des europäischen Patentprüfungsverfahren zu EP-A-809629 und EP-A-915846 bekannt geworden: 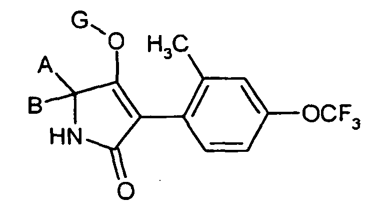 aus EP-A-809629

| Bsp.-Nr. | A B | G | Isomer |
|---|---|---|---|
| I-1-a-13 | -(CH₂)₂-CHCH₃-(CH₂)₂- | H | β |
| I-1-a-14 | -(CH₂)₂-CHOCH₃-(CH₂)₂ | H | β |
| I-1-b-14 | -(CH₂)₂-CHCH₃-(CH₂)₂ | CO-i-C₃H₇ | β |
| I-1-b-15 | -(CH₂)₂-CHCH₃-(CH₂)₂- | CO-CH₂-t-C₄H₉ | β |
| I-1-b-16 | -(CH₂)₂-CHOCH₃-(CH₂)₂- | CO-i-C₃H₇ | β |
| I-1-b-17 | -(CH₂)₂-CHOCH₃-(CH₂)₂ | CO-CH₂-t-C₄H₉ | β |
| I-1-c-9 | -(CH₂)₂-CHCH₃-(CH₂)₂- | CO₂C₂H₅ | β |
| I-1-c-10 | -(CH₂)₂-CHOCH₃-(CH₂)₂ | CO₂C₂H₅ | β |

aus EP-A-915846
Bsp.- I-1-a-29

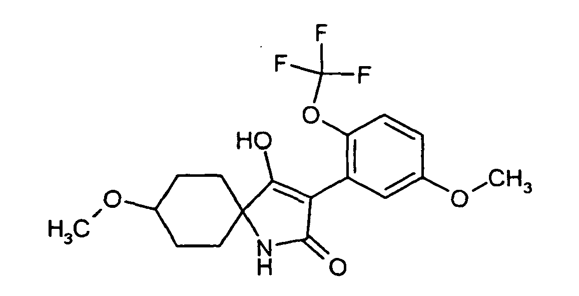

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein trifluormethoxy-phenylsubstituiertes Tetramsäure-Derivat der Formel (I), in welcher A, B, D, G, J, X und Y die oben angegebene Bedeutung haben
und
(b') zumindest eine die Kulturpflanzen-Verträglichkeit verbessernde Verbindung aus der folgenden Gruppe von Verbindungen: 4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloraceryl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dirnethyl-ethyl)-1H-pyrazo1-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinol-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, I-Brom-4-chlor-methylsulfonyl-benzol, 1-[4-(N-2-Methoxybenzoylsulfamoyl)-phenyl]-3-methyl-hanstoff (alias N-(2-Methoxy-benzoyl)-4-[(methylamino-carbonyl)-amino]-bemolsulfonamid), 1-[4-(N-2-Methoxy-benzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen der allgemeinen Formel (IIa) 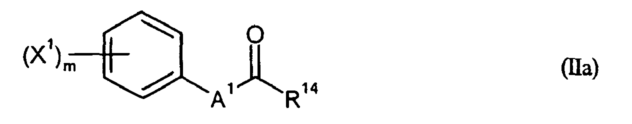 oder der allgemeinen Formel (IIb) 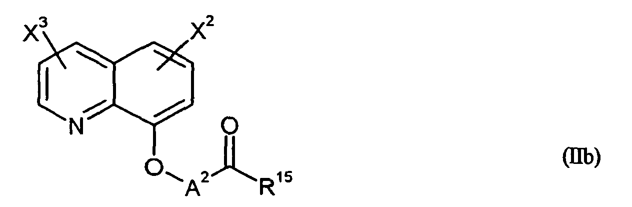 oder der Formel (IIc) 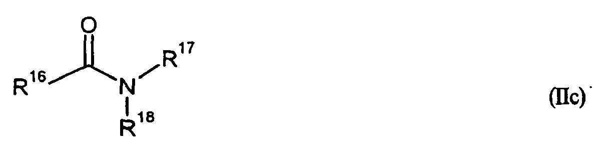 wobei
- m: für eine Zahl 0, 1, 2, 3, 4 oder 5 steht,
- A¹: für eine der nachstehend skizzierten divalenten heterocyclischen Gruppierungen steht,

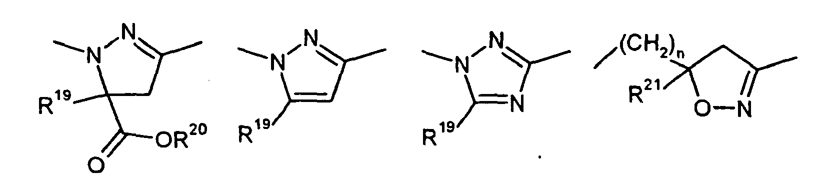

- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄- Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alklamino oder Di- (C₁C₄-alkyl)-amino steht,
- R¹⁵: für Hydroxy, Mercapto, Amino, C₁-C₇-Alkoxy, C₁-C₆-Akenyloxy, C₁-C₆-Alkenyloxy-C₁-C₆- alkoxy, C₁-C₆-Alkylthio, C₁-C₆-Alkylamino oder Di-(C₁-C₄-alkyl)-amino steht,
- R¹⁶: für jeweils gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆- Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆- Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
- R¹⁷ und R¹⁸: auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Akandiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloallryl oder Phenyl steht,
- R²⁰: für Wasserstoff, gegebenenfalls durch Hydroxy, Cyano, Halogen oder C₁-C₄-Alkoxy substitu- iertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl C₁-C₄-Alkoxy oder C₁-C₄- Halogenalkoxy steht,
- X²: für Wasserstoff, Cyano, Nitro, Halogen, C₁-C₄-Alkyl C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁₋C₄-Halogenalkoxy steht,
und/oder die folgenden durch allgemeine Formeln definierten Verbindungen der allgemeinen Formel (IId) 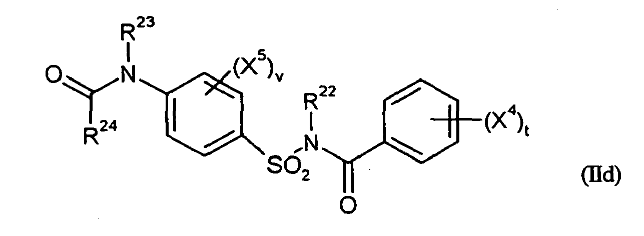 oder der allgemeinen Formel (IIe) 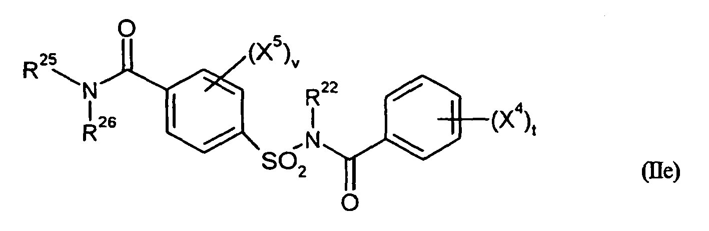 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cyclo- alkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substitu- iertes C₁-C₆Alkyl, jeweils gegebenenfalls durch Cyano oder Halogen substituiertes C₃-C₆- Alkenyl oder C₃-C₆-Akinyl, oder gegebenenfalls durch Cyano, Halogen oder C₁-C₄-Alkyl sub- stituiertes C₃-C₆-Cycloalkyl steht,
- R²⁶: für Wasserstoff, gegebenenfalls durch Cyano, Hydroxy, Halogen oder C₁-C₄-Alkoxy substitu- iertes C₁-C₆-Alkyl, jeweils gegebenenfalls durch Cyano oder Halogen substituiertes C₃-C₆- Alkenyl oder C₃-C₆-Alkinyl, gegebenenfalls durch Cyano, Halogen oder C₁-C₄-Alkyl substitu- iertes C₃-C₆-Cycloalkyl, oder gegebenenfalls durch Nitro, Cyano, Halogen, C₁-C₄-Alkyl, C₁-C₄- Halogenalkyl, C₁-C₄-Alkoxy oder C₁-C₄-Halogenalkoxy substituiertes Phenyl steht, oder zusam- men mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- J: steht bevorzugt für Trifluormethoxy,
- X: steht bevorzugt für Wasserstoff, Halogen, C₁-C₆-Alkyl, C₁-C₄-Halogenalkyl, C₁-C₆- Alkoxy oder C₁-C₄-Halogenalkoxy,
- Y: steht bevorzugt für Wasserstoff, C₁-C₆-Alkyl, C₁-C₆-Alkoxy oder Halogen,
mit der Maßgabe, dass mindestens einer der Reste J, X oder Y in der 2-Position des Phenylrestes steht und dabei ungleich Wasserstoff ist.

Bevorzugt stehen dabei die Reste J, X und Y mit ihren bevorzugten Bedeutungen in folgenden Phenylsubstitutionsmustern 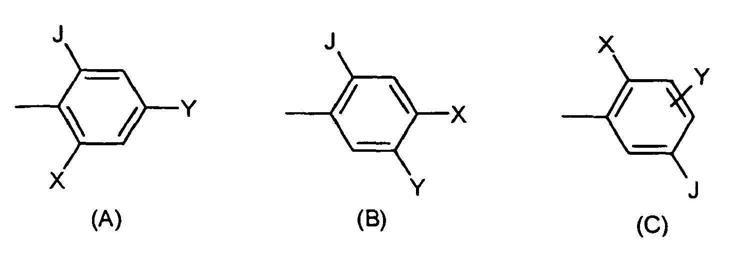 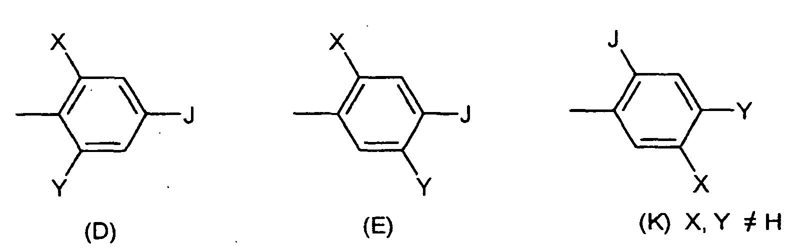 wobei in den Phenylsubstitutionsmustern (C), (D) und (E) X und Y gleichzeitig ungleich Wasserstoff stehen,
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₈-C₆- alkyl, gegebenenfalls durch Halogen, C₁-C₆-Alkyl oder C₁-C₆-Alkoxy substituiertes C₃- C₈-Cycloalkyl, in welchem gegebenenfalls ein oder zwei nicht direkt benachbarte Ring- glieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogen- alkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-C₁-C₆-alkyl oder Naphthyl-C₁-C₆-alkyl,
- B: steht bevorzugt für Wasserstoff, C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes C₃- C₁₀-Cycloalkyl oder ungesättigtes C₅-C ₁₀()-Cycloalkyl, worin gegebenenfalls ein Ringglied durch Sauerstoff oder Schwefel ersetzt ist und welche gegebenenfalls einfach oder zwei- fach durch C₁-C₈-Alkyl, C₁-C₆-Alkoxy-C₁-C₄-Alkyl, C₁-C₆-Alkoxy-C₁-C₄-alkoxy, C₃-C₆-Cycloalkyl-C₁-C₂-alkoxy, C₃-C₁₀-Cycloalkyl, C₁-C₈-Halogenalkyl C₁-C₈- Alkoxy, C₁-C₈-Alkylthio, Halogen oder Phenyl substituiert sind oder
- A, B: und das Kohlenstoffatom, an das sie gebunden sind, stehen bevorzugt für C₃-C₆-Cyclo- alkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cyclo- alkyl oder C₅-C₈-Cycloalkenyl, in welchen zwei Substituenten gemeinsam mit den Kohlen- stoffatomen, an die sie gebunden sind, für jeweils gegebenenfalls durch C₁-C₆-Alkyl, C₁- C₆-Alkoxy oder Halogen substituiertes C₂-C₆-Alkandiyl, C₂-C₆-Alkendiyl oder C₄-C₆- Alkandiendiyl stehen, worin gegebenenfalls eine Methylengruppe durch Sauerstoff oder Schwefel ersetzt ist,
- D: steht bevorzugt für Wasserstoff, jeweils gegebenenfalls durch Halogen substituiertes C₁- C₁₂-Alkyl, C₃-C₈-Alkenyl, C₃-C₈-Alkinyl, C₁-C₁₀-Alkoxy-C₂-C₈-alkyl, gegebenenfalls durch Halogen, C₁-C₄-Alkyl, C₁-C₄-Alkoxy oder C₁-C₄-Halogenalkyl substituiertes C₃- Cg-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazo- lyl, Pyrimidyl, Pyrrolyl, Thienyl oder Triazolyl), Phenyl-C₁-C₆-alkyl oder Hetaryl-C₁-C₆- alkyl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl) oder
- A und: D stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonyl- gruppe, Sauerstoff oder Schwefel ersetzt ist und
wobei als Substituenten jeweils in Frage kommen: Halogen, Hydroxy, Mercapto oder jeweils gegebenenfalls durch Halogen substituiertes C₁- C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ring- atomen bilden (und stehen dann beispielsweise für die weiter unten genannten Gruppen AD-1 bis AD-10), der Sauerstoff oder Schwefel enthalten kann, oder worin gegebenenfalls eine der folgenden Gruppen 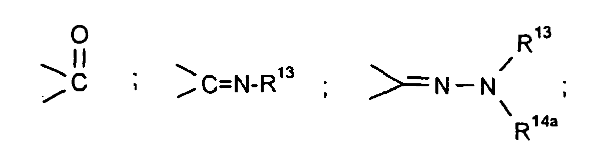 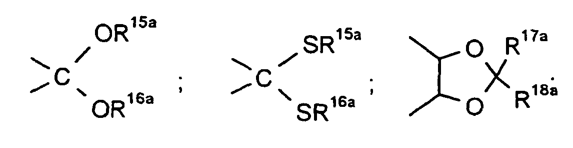 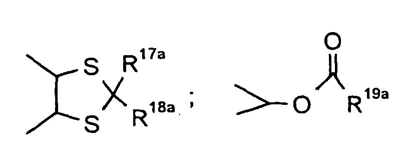 oder 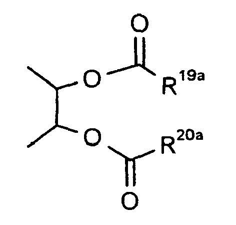 enthalten ist,
- G: steht bevorzugt für Wasserstoff (a) oder für eine der Gruppen 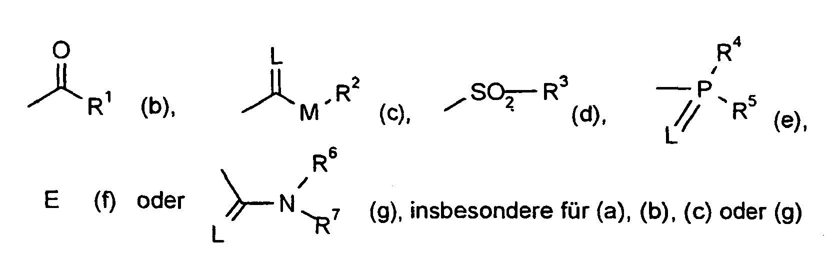
in welchen
- E: für ein Metallionäquivalent oder ein Ammoniumion steht,
- L: für Sauerstoff oder Schwefel steht und
- M: für Sauerstoff oder Schwefel steht,
- R¹: steht bevorzugt für jeweils gegebenenfalls durch Halogen substituiertes C₁-C₂₀-Alkyl, C₂- C₂₀-Alkenyl, C₁-C₈-Alkoxy-C₁-C₈-alkyl, C₁-C₈-Alkylthio-C₁-C₈-alkyl, Poly-C₁-C₈- alkoxy-C₁-C₈-alkyl oder gegebenenfalls durch Halogen, C₁-C₆-Alkyl oder C ₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind, für gegebenenfalls durch Halogen, Cyano, Nitro, C₁-C₆-Alkyl, C₁-C₆-Alkoxy, C₁-C₆- Halogenalkyl, C₁-C₆-Halogenalkoxy, C₁-C₆-Alkylthio oder C₁-C₆-Alkylsulfonyl substituiertes Phenyl, für gegebenenfalls durch Halogen, Nitro, Cyano, C₁-C₆-Alkyl, C₁-C₆-Alkoxy, C₁-C₆- Halogenalkyl oder C₁-C₆-Halogenalkoxy substituiertes Phenyl-C₁-C₆-alkyl, für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl), für gegebenenfalls durch Halogen oder C₁-C₆-Alkyl substituiertes Phenoxy-C₁-C₆-alkyl oder für gegebenenfalls durch Halogen, Amino oder C₁-C₆-Alkyl substituiertes 5- oder 6- gliedriges Hetaryloxy-C₁-C₆-alkyl (beispielsweise Pyridyloxy-C₁-C₆-alkyl, Pyrimidyloxy- C₁-C₆-alkyl oder Thiazolyloxy-C₁-C₆-alkyl),
- R²: steht bevorzugt für jeweils gegebenenfalls durch Halogen substituiertes C₁-C₂₀-Alkyl, C₂- C₂₀-Alkenyl, C₁-C₈-Alkoxy-C₂-C₈-alkyl, Poly-C₁-C₈-alkoxy-C₂-C₈-alkyl, für gegebenenfalls durch Halogen, C₁-C₆-Alkyl oder C₁-C₆-Alkoxy substituiertes C₃-C₈- Cycloalkyl oder für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenen- falls durch Halogen, Nitro, Cyano, C₁-C₄-Alkoxy, C₁-C₄-Halogenalkoxy, C₁-C₄-Alkyl- thio, C₁-C₄-Halogenalkylthio, C₁-C₄-Alkyl oder C₁-C₄-Halogenalkyl substituiertes Phenyl, Phenoxy oder Phenylthio,
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈- Alkenyl, C₁-C₈-Alkoxy-C₁-C₈-alkyl, für gegebenenfalls durch Halogen, C₁-C₈- Halogenalkyl, C₁-C₈-Alkyl oder C₁-C₈-Alkoxy substituiertes Phenyl, gegebenenfalls durch Halogen, C₁-C₈-Alkyl,C₁-C₈-Halogenalkyl oder C₁-C₈-Alkoxy substituiertes Benzyl oder zusammen für einen gegebenenfalls durch C₁-C₄-Alkyl substituierten C₃-C₆- Alkylenrest, in welchem gegebenenfalls ein Kohlenstoffatom durch Sauerstoff oder Schwefel ersetzt ist,
- R¹³: steht bevorzugt für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄- Halogenalkoxy, Nitro oder Cyano substituiertes Phenyl, Phenyl-C₁-C₄-alkyl oder Phenyl- C₁-C₄-alkoxy,
- R^{14a}: steht bevorzugt für Wasserstoff oder C₁-C₈-Alkyl oder
- R¹³ und R^{14a}: stehen gemeinsam bevorzugt für C₄-C₆-Alkandiyl,
- R^{15a} und R^{16a}: sind gleich oder verschieden und stehen bevorzugt für C₁-C₆-Alkyl oder
- R^{15a} und R^{16a}: stehen gemeinsam bevorzugt für einen C₂-C₄-Alkandiylrest, der gegebenenfalls durch C₁-C₆-Alkyl, C₁-C₆-Halogenalkyl oder durch gegebenenfalls durch Halogen, C₁- C₆-Alkyl, C₁-C₄-Halogenalkyl, C₁-C₆-Alkoxy, C₁-C₄-Halogenalkoxy, Nitro oder Cyano substituiertes Phenyl substituiert ist,
- R^{17a} und R^{18a}: stehen unabhängig voneinander bevorzugt für Wasserstoff, für gegebenenfalls durch Halogen substituiertes C₁-C₈-Alkyl oder für gegebenenfalls durch Halogen, C₁-C₆- Alkyl, C₁-C₆-Alkoxy, C₁-C₄-Halogenalkyl, C₁-C₄-Halogenalkoxy, Nitro oder Cyano substituiertes Phenyl oder
- R^{17a} und R^{18a}: stehen gemeinsam mit dem Kohlenstoffatom, an das sie gebunden sind, bevorzugt für eine Carbonylgruppe oder für gegebenenfalls durch Halogen, C₁-C₄-Alkyl oder C₁- C₄-Alkoxy substituiertes C₅-C₇-Cycloalkyl, in dem gegebenenfalls eine Methylengruppe durch Sauerstoff oder Schwefel ersetzt ist,
- R^{19a} und R^{20a}: stehen unabhängig voneinander bevorzugt für C₁-C₁₀-Alkyl, C₂-C₁₀-Alkenyl C₁C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino.

In den als bevorzugt genannten Restedefinitionen steht Halogen für Fluor, Chlor, Brom und Jod, insbesondere für Fluor, Chlor und Brom.
- J: steht besonders bevorzugt für Trifluormethoxy,
- X: steht besonders bevorzugt für Wasserstoff, Fluor, Chlor, Brom, C₁-C₄-Alkyl, Trifluormethyl oder C₁-C₄-Alkoxy,
- Y: steht besonders bevorzugt für Wasserstoff, Fluor, Chlor, Brom, C₁-C₄-Alkoxy oder C₁- C₄-Alkyl
mit der Maßgabe, dass mindestens einer der Reste J, X oder Y in der 2-Position des Phenylrestes steht und dabei ungleich Wasserstoff ist.

Besonders bevorzugt stehen dabei die Reste J, X und Y mit ihren besonders bevorzugten Bedeutungen in folgenden Phenylsubstitutionsmustern 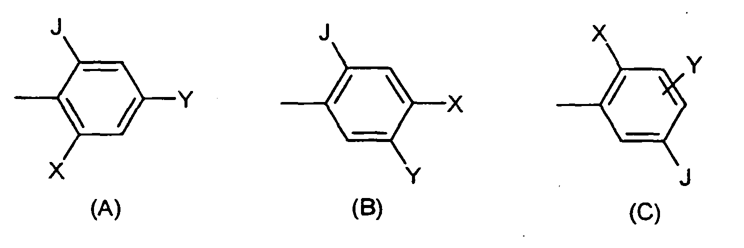 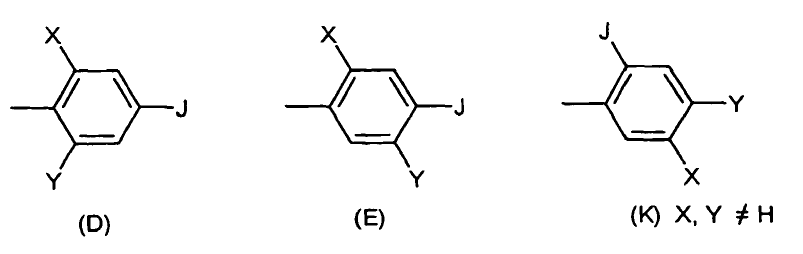 wobei in den Phenylsubstitutionsmustern (C), (D) und (E) X und Y gleichzeitig ungleich Wasserstoff stehen,
- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebe- nenfalls einfach bis zweifach durch C₁-C₂-Alkyl oder C₁-C₂-Alkoxy substituiertes C₃- C₆-Cycloalkyl, welches gegebenenfalls durch ein Sauerstoffatom unterbrochen sein kann, für jeweils einfach bis zweifach gegebenenfalls durch Fluor, Chlor, Brom, C₁-C₄-Alkyl, C_{I}-C₂-Halogenalkyl, C₁-C₄-Alkoxy, C₁-C₂-Halogenalkoxy, Cyano oder Nitro substitu- iertes Phenyl oder Benzyl,
- B: steht besonders bevorzugt für Wasserstoff, C₁-C₄-Alky oder C₁-C₂-Alkoxyl-C₁-C₂-alkyl oder
- A, B: und das Kohlenstoffatom an das sie gebunden sind, stehen besonders bevorzugt für ge- sättigtes oder ungesättigtes C₅-C₇-Cycloalkyl, worin gegebenenfalls ein Ringglied durch Sauerstoff oder Schwefel ersetzt ist und welches gegebenenfalls einfach bis zweifach durch C₁-C₆-Alkyl, C₁₋C₄-Alkoxy-C₁-C₂-alkyl, C₁-C₄-Alkoxy-C₁-C₂-alkoxy, C₃-C₆- Cycloalkyl-methoxy, Trifluormethyl oder C₁-C₆-Alkoxy, substituiert ist, oder
- A, B: und das Kohlenstoffatom, an das sie gebunden sind, stehen besonders bevorzugt für C₅- C₆-Cycloalkyl, welches durch eine gegebenenfalls ein oder zwei nicht direkt benachbarte Sauerstoff- oder Schwefelatome enthaltende gegebenenfalls durch Methyl oder Ethyl sub- stituierte Alkylendiyl- oder durch eine Alkylendioxyl- oder durch eine Alkylendithiol- Gruppe substituiert ist, die mit dem Kohlenstoffatom, an das sie gebunden ist, einen weiteren fünf- oder sechsgliedrigen Ring bildet, oder
- A, B: und das Kohlenstoffatom, an das sie gebunden sind, stehen besonders bevorzugt für C₃- C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂- Alkyl oder C₁-C₂-Alkoxy substituiertes C₂-C₄-Alkandiyl, C₂-C₄-Alkendiyl oder Buta- diendiyl stehen,
- D: steht besonders bevorzugt für Wasserstoff, für jeweils gegebenenfalls einfach bis dreifach durch Fluor substituiertes C₁-C₆-Alkyl, C₃-C₆-Alkenyl, C₁-C₄-Alkoxy-C₂-C₃-alkyl, für gegebenenfalls einfach bis zweifach durch C₁-C₄-Alkyl, C₁-C₄-Alkoxy oder C₁-C₂- Halogenalkyl substituiertes C₃-C₆-Cycloalkyl, in welchem gegebenenfalls eine Methylen- gruppe durch Sauerstoff ersetzt ist, oder
- A und D: stehen gemeinsam besonders bevorzugt für gegebenenfalls einfach bis zweifach sub- stituiertes C₃-C₅-Alkandiyl, in welchem eine Methylengruppe durch eine Carbonylgruppe, Sauerstoff oder Schwefel ersetzt sein kann, wobei als Substituenten C₁-C₂-Alkyj oder C₁- C₂-Alkoxy in Frage kommen oder
- A und D: stehen gemeinsam mit den Atomen, an die sie gebunden sind, für eine der Gruppen AD-1 bis AD-10:

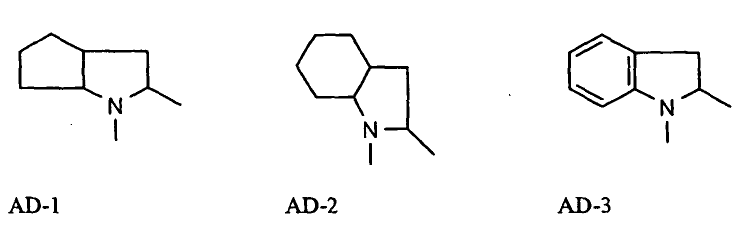

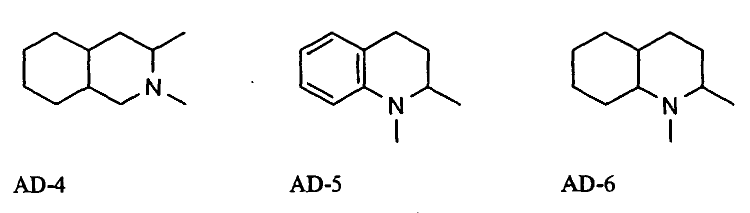

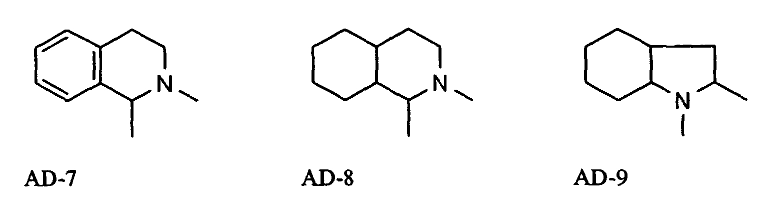

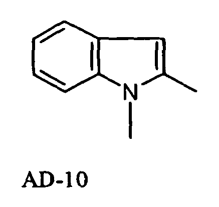

- G: steht besonders bevorzugt für Wasserstoff (a) oder für eine der Gruppen 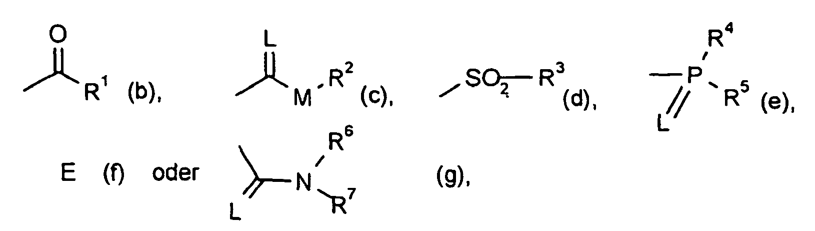
in welchen
- E: für ein Metallionäquivalent oder ein Ammoniumion steht,
- L: für Sauerstoff oder Schwefel steht und
- M: für Sauerstoff oder Schwefel steht,

- R¹: steht besonders bevorzugt für jeweils gegebenenfalls einfach bis dreifach durch Fluor oder Chlor substituiertes C₁-C₈-Alkyl, C₂-C₁₈-Alkenyl, C₁-C₄-Alkoxy-C₁-C₂-alkyl, C₁-C₄- Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenen- falls ein oder zwei nicht direkt benachbarte Ringglieder durch Sauerstoff ersetzt sind, für gegebenenfalls einfach bis zweifach durch Fluor, Chlor, Brom, Cyano, Nitro, C₁-C₄- Alkyl, C₁-C₄-Alkoxy, C₁-C₂-Halogenalkyl oder C₁-C₂-Halogenalkoxy substituiertes Phenyl,
- R²: steht besonders bevorzugt für jeweils gegebenenfalls einfach bis dreifach durch Fluor sub- stituiertes C₁-C₈-Alkyl C₂-C₈-Alkenyl oder C₁-C₄-Alkoxy-C₂-C₄-alkyl, für gegebenenfalls einfach durch C₁-C₂-Alkyl oder C₁-C₂-Alkoxy substituiertes C₃-C₆- Cycloalkyl oder für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- R³: steht besonders bevorzugt für gegebenenfalls einfach bis dreifach durch Fluor substi- tuiertes C₁-C₆-Alkyl oder für jeweils gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl,
- R⁴: steht besonders bevorzugt für C₁-C₆-Alkyl, C₁-C₆-Alkoxy, C₁-C6-_{A}lkylamino, Di-(C₁- C₆-alkyl)amino_{,} C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Allcoxy, C₁-C₃-Halogenalkoxy, C₁-C₃-Alkylthio, C₁-C₃-Halogenalkylthio, C₁-C₃-Alkyl oder Tri- fluormethyl substituiertes Phenyl, Phenoxy oder Phenylthio,
- R⁵: steht besonders bevorzugt für C₁C₆-Alkoxy oder C₁₋-Alkylthio,
- R⁶: steht besonders bevorzugt für Wasserstoff, C₁-C₆-Alkyl, C₃-C₆-Cycloalkyl, C₁-C₆- Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phe- nyl, für gegebenenfalls einfach durch Fluor, Chlor, Brom, C₁-C₄-Alkyl, Trifluormethyl oder C₁-C₄-Alkoxy substituiertes Benzyl,
- R⁷: steht besonders bevorzugt für C₁-C₆-Alkyl, C₃-C₆-Alkenyl oder C₁-C₆-Alkoxy-C₁-C₄-alkyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- J: steht ganz besonders bevorzugt für Trifluormethoxy,
- X: steht ganz besonders bevorzugt für Wasserstoff, Fluor, Chlor, Brom, Methyl, Ethyl, Propyl, Methoxy oder Ethoxy,
- Y: steht ganz besonders bevorzugt für Wasserstoff, Chlor, Brom, Methyl, Ethyl oder Methoxy,
mit der Maßgabe, dass mindestens einer der Reste J, X oder Y in der 2-Position des Phenylrestes steht und ungleich Wasserstoff ist.

Ganz besonders bevorzugt stehen dabei die Reste J, X und Y mit ihren ganz besonders bevorzugten Bedeutungen in folgenden Phenylsubstitutionsmustern 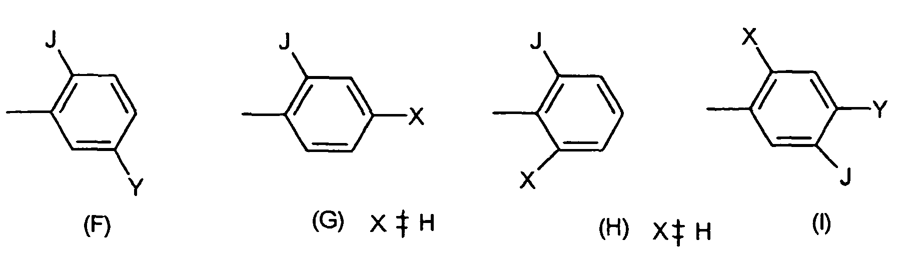 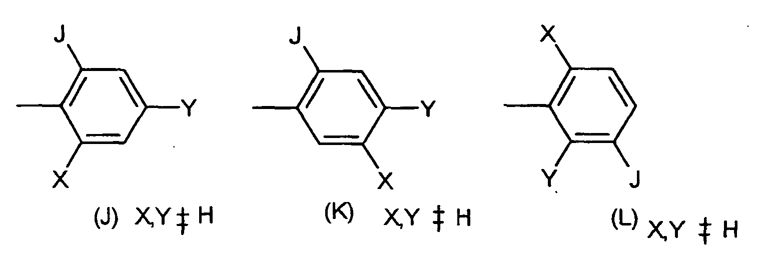 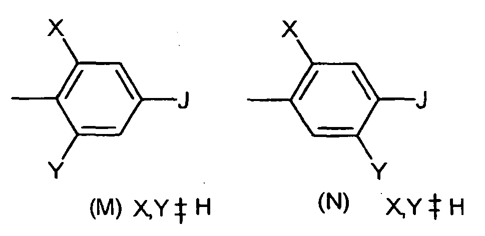
- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl,
- B: steht ganz besonders bevorzugt für Wasserstoff, Methyl oder Ethyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Methoxymethyl, Ethoxymethyl, Propoxymethyl, Trifluormethyl, Methoxy, Ethoxy, Propoxy, Butoxy, Methoxyethyl, Ethoxyethyl, Methoxyethoxy, Ethoxyethoxy, Cyclopropylmethoxy, Cyclopentylmethoxy oder Cyclohexylmethoxy substituiert ist, oder
- A, B: und das Kohlenstoffatom, an das sie gebunden sind, stehen ganz besonders bevorzugt für C₆-Cycloalkyl, welches gegebenenfalls durch mit zwei nicht direkt benachbarten Sauer- stoffatomen enthaltende Alkylendioxyl-Gruppe substituiert ist, oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
- D: steht ganz besonders bevorzugt für Wasserstoff, für jeweils gegebenenfalls einfach bis dreifach durch Fluor substituiertes C₁-C₄-Alkyl, C₃-C₄-Alkenyl, C₁-C₄-Alkoxy-C₂-C₃- alkyl, für Cyclopropyl, Cyclopentyl oder Cyclohexyl,
oder
- A und D: stehen gemeinsam ganz besonders bevorzugt für gegebenenfalls einfach durch Methyl oder Methoxy substituiertes C₃-C₅-Alkandiyl, worin gegebenenfalls ein Kohlenstoffatom durch Sauerstoff oder Schwefel ersetzt ist oder für die Gruppe AD-1,
- G: steht ganz besonders bevorzugt für Wasserstoff (a) oder für eine der Gruppen 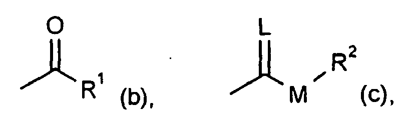
-SO₂-R³ (d) oder E (f), insbesondere für (a), (b), (c) oder (f)
in welchen
- L: für Sauerstoff oder Schwefel steht,
- M: für Sauerstoff oder Schwefel steht und
- E: für ein Ammoniumion steht,

- R¹: steht ganz besonders bevorzugt für C₁-C₆-Alkyl, C₂-C₁₇-Alkenyl, C₁-C₂-Alkoxy-C₁- alkyle, C₁-C₂-Alkylthio-C₁-alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Tri- fluormethyl oder Trifluormethoxy substituiertes Phenyl,
- R²: steht ganz besonders bevorzugt für jeweils gegebenenfalls einfach durch Fluor sub- stituiertes C₁-C₈-Alkyl, C₂-C₆-Alkenyl oder C₁-C₄-Alkoxy-C₂-C₃-alkyl, Phenyl oder Benzyl,
- R³: steht ganz besonders bevorzugt für C₁-C₈-Alkyl.
- J: steht hervorgehoben für Trifluormethoxy,
- X: steht hervorgehoben für Wasserstoff, Chlor, Brom, Methyl, Ethyl, oder Methoxy,
- Y: steht hervorgehoben für Wasserstoff, Chlor, Brom, Methyl oder Methoxy,
mit der Maßgabe, dass mindestens einer der Reste J, X oder Y in der 2-Position des Phenylrestes steht und ungleich Wasserstoff ist.

Hervorgehoben stehen dabei die Reste J, X und Y mit ihren ganz besonders bevorzugten Bedeutungen in folgenden Phenylsubstitationsmustem 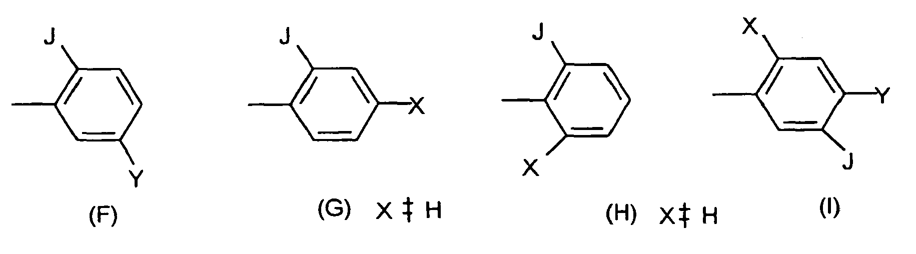 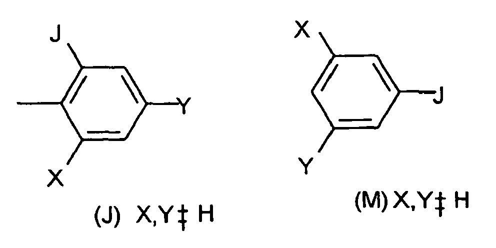
- A: steht hervorgehoben für C₁-C₄-Alkyl oder Cyclopropyl,
- B: steht hervorgehoben für Wasserstoff oder Methyl,
- A, B: und das Kohlenstoffatom an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methoxymethyl, Methoxy, Ethoxy, Propoxy, oder Butoxy substituiert ist,
oder für 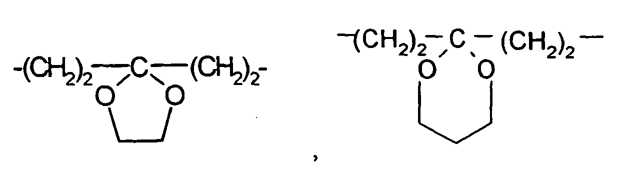
- D: steht hervorgehoben für Wasserstoff oder Cyclopropyl,
oder
- A und D: stehen gemeinsam hervorgehoben für C₃-C₅-Alkandiyl,
- G: steht hervorgehoben für Wasserstoff (a) oder für eine der Gruppen

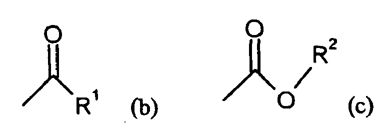

- R¹: steht hervorgehoben für C₁₋C₆-Alkyl,
- R²: steht hervorgehoben für C₁-C₈-Alkyl oder Benzyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt. Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

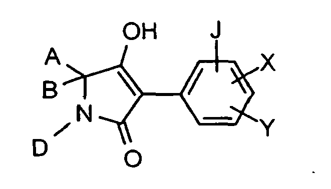

**Tabelle 1: 2-OCF₃; X = H; Y = H.**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 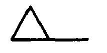 | CH₃ | H |
| 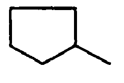 | CH₃ | H |
| 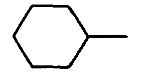 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -CH₂-CHOCH₃-(CH₂)₃- | | H |
| -CH₂-CHOC₂H₅-(CH₂)₃- | | H |
| CH₂-CHOC₃H₇-(CH₂)₃- | | H |
| -CH₂-CHOC₄H₉-(CH₂)₃- | | H |
| -CH₂-CHO-(CH₂)₂-OCH₃-(CH₂)₃- | | H |
| -CH₂-CH(O-CH₂)-(CH₂)₃- | | H |
| 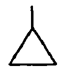 | | |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-i-C₃H₇-(CH₂)₂- | | H |
| -CH₂-CH(CH₂-OCH₃)-(CH₂)₃- | | H |
| -CH₂-CH(CH₂-CH₂-OCH₃)-(CH₂)₃- | | H |
| -(CH₂)₂-CH(CH₂-OCH₃)-(CH₂)₂- | | H |
| -(CH₂)₂-CH(CH₂-CH₂-OCH₃)-(CH₂)₂- | | H |
| -(CH₂)₂-C(CH₃)₂-(CH₂)₂- | | H |
| -CH₂-(CHCH₃)₂-(CH₂)₂- | | H |
| 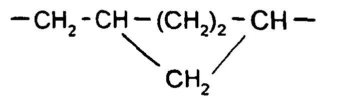 | | H |
| 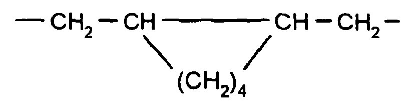 | | H |
| 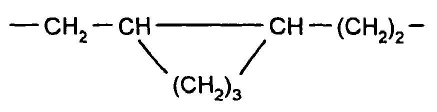 | | H |
| 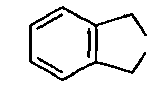 | | H |
| 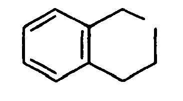 | | H |

| A | D | B |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 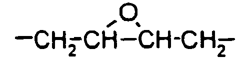 | | H |
| -CH₂-S-CH₂- | | H |

| A | B | D |
|---|---|---|
| -CH₂-S-(CH₂)₂- | | H |
| -(CH₂)₂-S-CH₂- | | H |
| 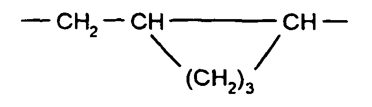 | | H |
| H | CH₃ | H |
| H | C₂H₅ | H |
| H | C₃H₇ | H |
| H | i-C₃H₇ | H |
| H | 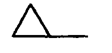 | H |
| H | 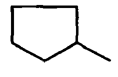 | H |
| H | 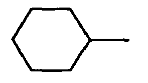 | H |
| CH₃ | CH₃ | H |
| CH₃ | C₂H₅ | H |
| CH₃ | C₃H₇ | H |
| CH₃ | i-C₃H₇ | H |
| CH₃ | 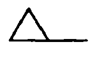 | H |
| CH₃ | 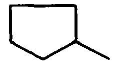 | H |
| CH₃ | 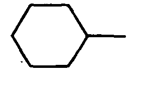 | H |
| 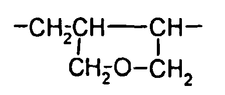 | | H |
| C₂H₅ | CH₃ | H |
| C₂H₅ | C₂H₅ | H |

**Tabelle 2:** A, B und D wie in Tabelle 1 angegeben
   J = 2-OCF₃; X = 4-CH₃; Y = H
**Tabelle 3:** A, B und D wie in Tabelle 1 angegeben
   J = 2-OCF₃; X = 6-CH₃; Y = H.
**Tabelle 4:** A, B und D wie in Tabelle 1 angegeben
   J = 2-OCF₃; X = 6-C₂H₅; Y = H.
**Tabelle 5:** A, B und D wie in Tabelle 1 angegeben
   X = 2-CH₃; Y = H; J = 5-OCF₃.
**Tabelle 6:** A, B und D wie in Tabelle 1 angegeben
   X = 2-CH₃; Y = 4-CH₃; J = 5-OCF₃.
**Tabelle 7:** A, B und D wie in Tabelle 1 angegeben
   J = 2-OCF₃; X = 4-CH₃; Y = 6-CH₃.
**Tabelle 8:** A, B und D wie in Tabelle 1 angegeben
   J = 2-OCF₃; X = 6-C₂H₅; Y = 4-CH₃.
**Tabelle 9:** A, B und D wie in Tabelle 1 angegeben
   J = 2-OCF₃; X = 6-CH₃; Y = 4-Cl.
**Tabelle 10:** A, B und D wie in Tabelle 1 angegeben
   J = 2-OCF₃; X = 6-C₂H₅; Y = 4-Cl.
**Tabelle 11:** A, B und D wie in Tabelle 1 angegeben
   J = 2-OCF₃; X = 6-Cl; Y = 4-CH₃.
**Tabelle 12:** A, B und D wie in Tabelle 1 angegeben
   J = 2-OCF₃; X = 5-CH₃; Y = 4-CH₃.
**Tabelle 13:** A, B und D wie in Tabelle 1 angegeben
   X = 2-CH₃; J = 4-OCF₃; Y = 6-CH₃.
**Tabelle 14:** A, B und D wie in Tabelle 1 angegeben
   X = 2-C₂H₅; J = 4-OCF₃; Y = 6-CH₃.
**Tabelle 15:** A, B und D wie in Tabelle 1 angegeben
   X = 2-C₂H₅; J = 4-OCF₃; Y = 6-C₂H₅.
**Tabelle 16:** A, B und D wie in Tabelle 1 angegeben
   X = 2-Cl; J = 4-OCF₃; Y = 6-CH₃.
**Tabelle 17:** A, B und D wie in Tabelle 1 angegeben
   X = 2-Cl; J = 4-OCF₃; Y = 6-C₂H₅.
**Tabelle 18:** A, B und D wie in Tabelle 1 angegeben
   X = 2-Cl; J = 4-OCF₃; Y = H.
**Tabelle 19:** A, B und D wie in Tabelle 1 angegeben
   X = 2-Br; J = 4-OCF₃; Y = H.
**Tabelle 20:** A, B und D wie in Tabelle 1 angegeben
   X = 2-OCH₃; J = 4-OCF₃; Y = 6-Cl.
**Tabelle 21:** A, B und D wie in Tabelle 1 angegeben
   X = 2-OCH₃; J = 6-OCF₃; Y = 4-Cl.
**Tabelle 22:** A, B und D wie in Tabelle 1 angegeben
   J = 2-OCF₃; X = 6-Cl; Y = 4-Cl.
**Tabelle 23:** A, B und D wie in Tabelle 1 angegeben
   J = 2-OCF₃; X = 6-Cl; Y = 4-Br.
**Tabelle 24:** A, B und D wie in Tabelle 1 angegeben
   J = 2 - OCF₃; X = 6-Br; Y = 4-Br.
**Tabelle 25:** A, B und D wie in Tabelle 1 angegeben
   J = 4 - OCF₃; X = 2-Cl; Y = 6-Cl.
**Tabelle 26:** A, B und D wie in Tabelle 1 angegeben
   J = 4 - OCF₃; X = 2-Br; Y = 6-Cl.
**Tabelle 27:** A, B und D wie in Tabelle 1 angegeben
   X = 2-Br; J = 4 - OCF₃; Y = 6-Br.
**Tabelle 28:** A, B und D wie in Tabelle 1 angegeben
   X=2-C1; J = 5-OCF₃;Y = H.
**Tabelle 29:** A, B und D wie in Tabelle 1 angegeben
   X = 2-Br; J = 5-OCF₃;Y = H.
**Tabelle 30:** A, B und D wie in Tabelle 1 angegeben
   X = H; J = 2 - OCF₃; Y = 5-CH₃.

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen

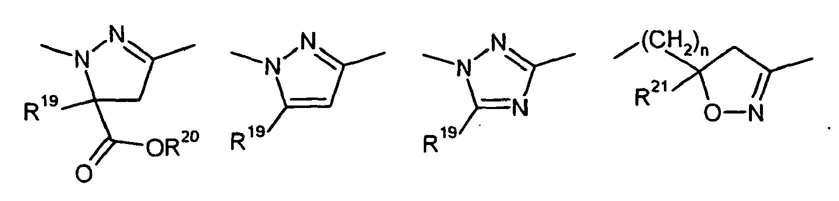

- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxy- carbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methyl- amino, Ethylamino, n- oder i-Propylamino, n-, i-, s- oder t-Butylamino, Dimethylamino oder Di- ethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexaloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substitu- iertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substitu- iertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t- Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluor- methyl, Fluordichlormethyl, Methoxy, Ethoxy, n- oder i-Propoxy, Difluormethoxy oder Tri- fluormethoxy.
- X²: steht bevorzugt für Wasserstoff, Nitro, Cyano, Fluor, Chlor, Brom, Methyl, Ethyl, n- oder i- Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i- Propyl, n-, i-, s- oder t-Butyl, Difluonnethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2 oder 3.
- R²²: steht bevorzugt für Wasserstoff, Methyl, Ethyl, n- oder i-Propyl.
- R²³: steht bevorzugt für Wasserstoff, Methyl, EthyL n- oder i-Propyl.

- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i- Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclo- butyl, Cyclopentyl, Cyclohexyl, Cyclopropyloxy, Cyclobutyloxy, Cyclopentyloxy, Cyclohexyl- oxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s- Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclo- hexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s- Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclo- hexyl, oder gegebenenfalls durch Nitro, Cyano, Fluor, Chlor, Brom, Methyl, Ethyl, n- oder i- Propyl, n-, i-, s- oder t-Butyl, Trifluormethyl, Methoxy, Ethoxy, n- oder i-Propoxy, Difluor- methoxy oder Trifluormethoxy substituiertes Phenyl, oder zusammen mit R²⁵ für jeweils gege- benenfalls durch Methyl oder Ethyl substituiertes Butan-1,4-diyl (Trimethylen), Pentan-1,5-diyl, 1-Oxa-butan-1,4-(hyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle Beispiele für die Verbindungen der Formel (IIa)**

| | | | |
|---|---|---|---|
| 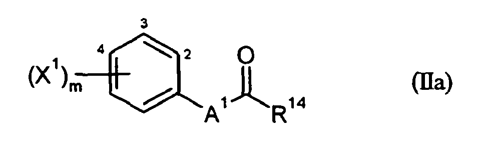 | | | |

| **Beispiel- Nr.** | **(Positionen) (X¹)ₘ** | **A¹** | **R¹⁴** |
|---|---|---|---|
| IIa-1 | (2) Cl, (4) Cl | 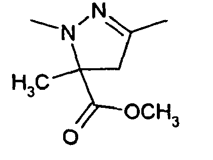 | OCH₃ |
| IIa-2 | (2) Cl, (4) Cl | 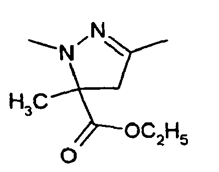 | OCH₃ |
| IIa-3 | (2) Cl, (4) Cl | 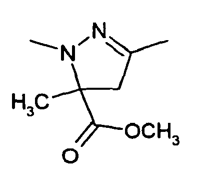 | OC₂H₅ |
| IIa-4 | (2) Cl, (4) Cl | 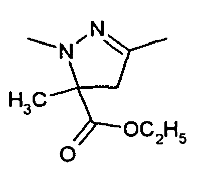 | OC₂H₅ |
| IIa-5 | (2) Cl | 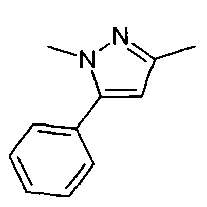 | OCH₃ |
| IIa-6 | (2) Cl, (4) Cl | 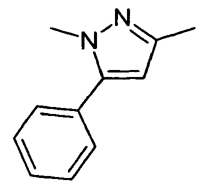 | OCH₃ |
| IIa-7 | (2) F | 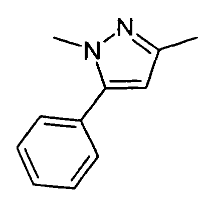 | OCH₃ |
| IIa-8 | (2) F | 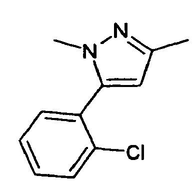 | OCH₃ |
| IIa-9 | (2) Cl, (4) Cl | 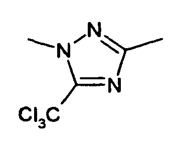 | OC₂H₅ |
| IIa-10 | (2) Cl, (4) CF₃ | 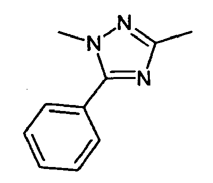 | OCH₃ |
| IIa-11 | (2) Cl | 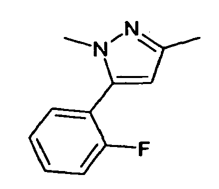 | OCH₃ |
| IIa-12 | - | 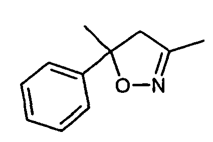 | OC₂H₅ |
| IIa-13 | (2) Cl, (4) Cl | 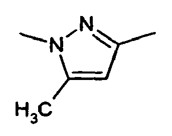 | OC₂H₅ |
| IIa-14 | (2) Cl, (4) Cl | 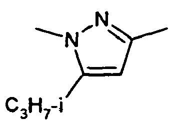 | OC₂H₅ |
| IIa-15 | (2) Cl, (4) Cl | 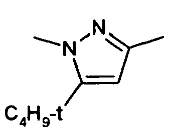 | OC₂H₅ |
| IIa-16 | (2) Cl, (4) Cl | 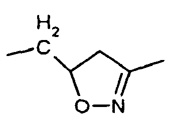 | OC₂H₅ |
| IIa-17 | (2) Cl, (4) Cl | 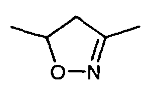 | OC₂H₅ |
| IIa-18 | - | 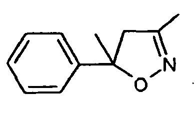 | OH |

Beispiele für die als erfindungsgemäße Herbizid-Safener ganz besonders bevorzugten Verbindungen der Formel (IIb) sind in der nachstehenden Tabelle aufgeführt.

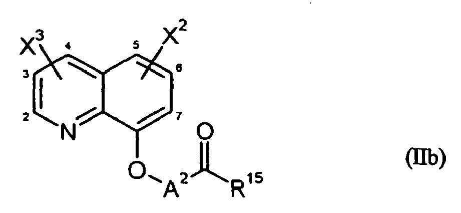

**Tabelle Beispiele für die Verbindungen der Formel (IIb)**

| **Beispiel- Nr.** | **(Position) X²** | **(Position) X³** | **A²** | **R¹⁵** |
|---|---|---|---|---|
| IIb-1 | (5) | - | CH₂ | OH |
| | Cl | | | |
| IIb-2 | (5) | - | CH₂ | OCH₃ |
| | Cl | | | |
| IIb-3 | (5) | - | CH₂ | OC₂H₅ |
| | Cl | | | |
| IIb-4 | (5) | - | CH₂ | OC₃H₇-n |
| | Cl | | | |
| IIb-5 | (5) | - | CH₂ | OC₃H₇-i |
| | Cl | | | |
| IIb-6 | (5) | - | CH₂ | OC₄H₉-n |
| | Cl | | | |
| IIb-7 | (5) | - | CH₂ | OCH(CH₃)C₅H₁₁-n |
| | Cl | | | |
| IIb-8 | (5) | (2) | CH₂ | OH |
| | Cl | F | | |
| IIb-9 | (5) | (2) | CH₂ | OH |
| | Cl | Cl | | |
| IIb-10 | (5) | - | CH₂ | OCH₂CH=CH₂ |
| | Cl | | | |
| IIb-11 | (5) | - | CH₂ | OC₄H₉-i |
| | Cl | | | |
| IIb-12 | (5) | - | CH₂ | 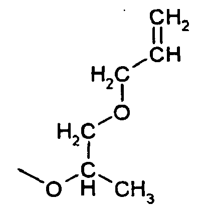 |
| | Cl | | | |
| IIb-13 | (5) | - | 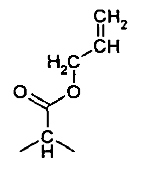 | OCH₂CH=CH₂ |
| | Cl | | | |
| IIb-14 | (5) | - | 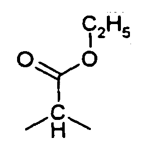 | OC₂H₅ |
| | Cl | | | |
| IIb-15 | (5) | - | 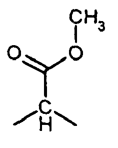 | OCH₃ |
| | Cl | | | |

Beispiele für die als erfindungsgemäße Herbizid-Safener ganz besonders bevorzugten Verbindungen der Formel (IIc) sind in der nachstehenden Tabelle aufgeführt.

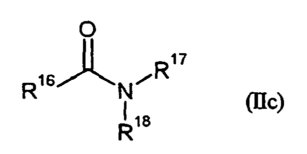

**Tabelle Beispiele für die Verbindungen der Formel (IIc)**

| **Beispiel- Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 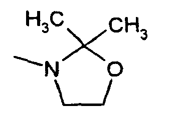 |
| IIc-3 | CHCl₂ | 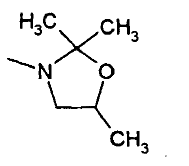 |
| IIc-4 | CHCl₂ | 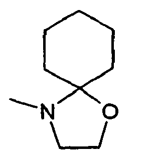 |
| IIc-5 | CHCl₂ | 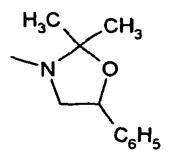 |
| IIc-6 | CHCl₂ | 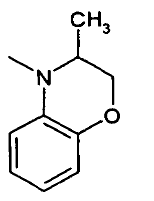 |
| IIc-7 | CHCl₂ | 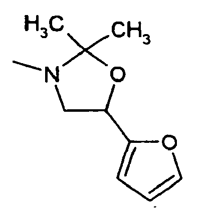 |

Beispiele für die als erfindungsgemäße Herbizid-Safener ganz besonders bevorzugten Verbindungen der Formel (IId) sind in der nachstehenden Tabelle aufgeführt.

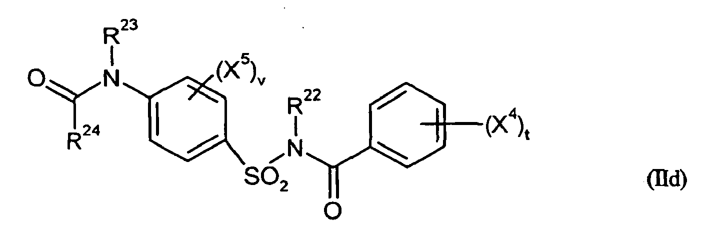

**Tabelle Beispiele für die Verbindungen der Formel (IId)**

| **Beispiel- Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 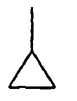 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-1 | (2) OCH₃ | - |
| | | | | (5) CH₃ | |
| IId-10 | H | H | 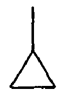 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 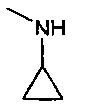 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId.25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

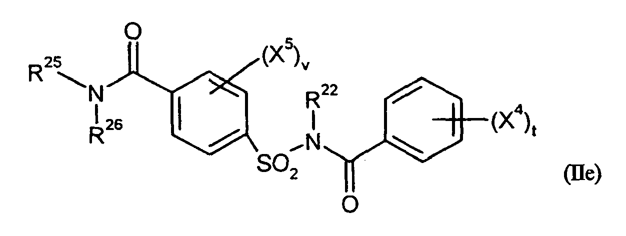

**Tabelle Beispiele für die Verbindungen der Formel (IIe)**

| **Beispiel- Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 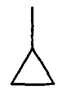 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 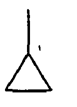 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyrdiethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| I-a | Cloquintocet-mexyl |
| I-a | Fenchlorazole-ethyl |
| I-a | Isoxadifen-ethyl |
| I-a | Mefenpyr-diethyl |
| I-a | Furilazole |
| I-a | Fenclorim |
| I-a | Cumyluron |
| I-a | Daimuron /Dymron |
| I-a | Dimepiperate |
| I-a | IIe-11 |
| I-a | IIe-5 |
| 1-b | Cloquintocet-mexyl |
| I-b | Fenchlorazole-ethyl |
| I-b | Isoxadifen-ethyl |
| I-b | Mefenpyr-diethyl |
| I-b | Furilazole |
| I-b | Fenclorim |
| I-b | Cumyluron |
| I-b | Daimuron /Dymron |
| I-b | Dimepiperate |
| I-b | IIe-11 |
| I-b | IIe-5 |
| I-c | Cloquintocet-mexyl |
| I-c | Fenchlorazole-ethyl |
| I-c | Isoxadifen-ethyl |
| I-c | Mefenpyr-diethyl |
| I-c | Furilazole |
| I-c | Fenclorim |
| I-c | Cumyluron |
| I-c | Daimuron /Dymron |
| I-c | Dimepiperate |
| I-c | IIe-5 |
| I-c | IIe-11 |
| I-d | Cloquintocet-mexyl |
| I-d | Fenchlorazole-ethyl |
| I-d | Isoxadifen-ethyl |
| I-d | Mefenpyr-diethyl |
| I-d | Furilazole |
| I-d | Fenclorim |
| I-d | Cumyluron |
| I-d | Daimuron /Dymron |
| I-d | Dimepiperate |
| I-d | IIe-11 |
| I-d | IIe-5 |
| I-e | Cloquintocet-mexyl |
| I-e | Fenchlorazole-ethyl |
| I-e | Isoxadifen-ethyl |
| I-e | Mefenpyr-diethyl |
| I-e | Furilazole |
| I-e | Fenclorim |
| I-e | Cumyluron |
| I-e | Daimuron /Dymron |
| I-e | Dimepiperate |
| I-e | IIe-5 |
| I-e | IIe-11 |
| I-f | Cloquiniocet-mexyl |
| I-f | Fenchlorazole-ethyl |
| I-f | Isoxadifen-ethyl |
| I-f | Mefenpyr-diethyl |
| I-f | Furilazole |
| I-f | Fenclorim |
| I-f | Cumyluron |
| I-f | Daimuron /Dymron |
| I-f | Dimepiperate |
| I-f | IIe-5 |
| I-f | IIe-11 |
| I-g | Cloquintocet-mexyl |
| I-g | Fenchlorazole-ethyl |
| I-g | Isoxadifen-ethyl |
| I-g | Mefenpyr-diethyl |
| I-g | Furilazole |
| I-g | Fenclorim |
| I-g | Cumyluron |
| I-g | Daimuron/Dymron |
| I-g | Dimepiperate |
| I-g | IIe-5 |
| I-g | IIe-11 |
| I-h | Cloquintocet-mexyl |
| I-h | Fenchlorazole-ethyl |
| I-h | Isoxadifen-ethyl |
| I-h | Mefenpyr-diethyl |
| I-h | Furilazole |
| I-h | Fenclorim |
| I-h | Cumyluron |
| I-h | Daimuron /Dymron |
| I-h | Dimepiperate |
| I-h | IIe-5 |
| I-h | IIe-11 |

Es wurde nun überraschend gefunden, dass die oben definierten Wirkstoffkombinationen aus Trifluormethoxy-phenyl substituierten Tetramsäure-Derivaten der allgemeinen Formel (I) und Safenern (Antidots) aus der oben aufgeführten Gruppe (b') bei sehr guter Nutzpflanzen-Vertraglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und bestimmte cyclische Ketoenole beschrieben (US 6 645 914, EP-A2 0 036 106, WO 07/068427). Eine entsprechende Wirkung bei Insektiziden wird für bestimmte cyclische Ketoenole durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun überraschend gefunden, dass sich auch die Wirkung von Insektiziden und/oder Akariziden und/oder Herbiziden aus der Klasse der Trifluormethoxy-phenyl substituierten Tetramsäure-Derivate der Formel (I) durch den Zusatz von Ammonium- oder Phosphoniumsalzen zur Anwendungslösung oder durch den Einbau dieser Salze in eine Formulierung enthaltend Trifluormethoxy-phenyl substituierte Tetramsäure-Derivate der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und / oder akarizid wirksame Trifluormethoxy-phenyl substituierte Tetramsäure-Derivate der Formel (I) als Wirkstoff enthalten. Gegenstand der Erfindung sind ebenfalls Mittel, die herbizid und/oder akarizid und/oder insektizid wirksame Trifluormethoxy-phenyl substituierte Tetramsäure-Derivate der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig. Diese Eigenschaften können jedoch durch den Zusatz von Ammonium- oder Phosphoniumsalzen insgesamt oder partiell verbessert werden.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Wirkstoff aus der Klasse der Trifluormethoxy-phenyl substituierten Tetramsäure-Derivate der Formel (I) steigern, werden durch Formel (III') definiert 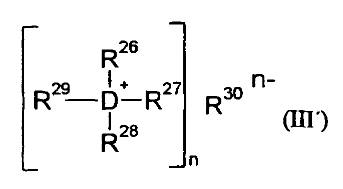 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶ R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R²⁶, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht.
- R³⁰: ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend Trifluormethoxy-phenyl sustituierte Tetramsäure-Derivate der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als sehr überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid wirksame, Trifluormethoxy-phenyl substituierte Tetramsäure-Derivate der Formel (I) als Wirkstoff enthalten. Gegenstand der Erfindung sind ebenfalls Mittel, die herbizid und/oder akarizid und/oder insektizid wirksame Trifluormethoxy-phenyl substituierte Tetramsäure-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,

- PO: für 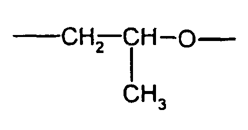 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,

- PO: für 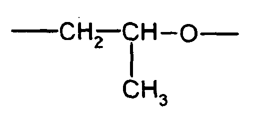 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R: die oben angegebenen Bedeutungen haben,
- EO: für CH₂-CH₂-O- steht,

- BO: für 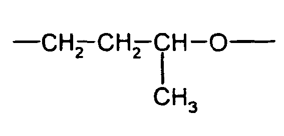 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R': die oben angegebenen Bedeutungen haben,
- BO: für 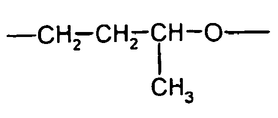 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderem sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV'-c) sei 2-Ethyl-hexyl-alkoxylat der Formel 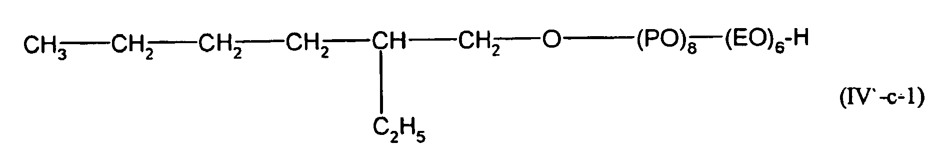 in welcher
- EO: für -CH₂-CH₂-O- steht,
- PO: für 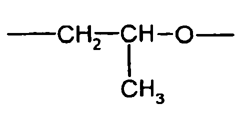 steht und
die Zahlen 8 und 6 Durchschnittswerte darstellen, genannt.

Als Beispiel für ein Alkanol-Alkoxylat der Formel (IV'-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: für 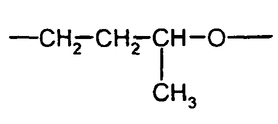 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskemöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.%, bevorzugt bei 1 bis 55 Gew.%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-(2,6-Dichlor-4-trifluormethoxy-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

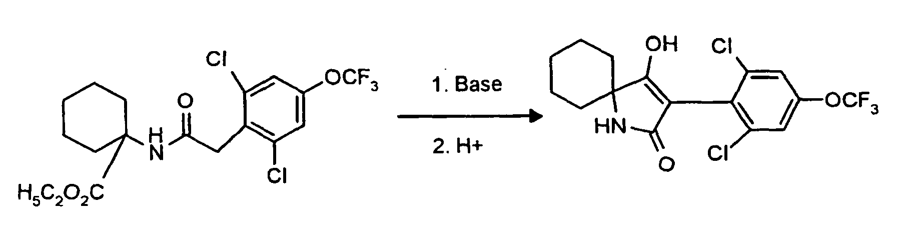

Verwendet man beispielsweise gemäß Verfahren (Bα) 3-(2-Chlor-4-trifluormethoxy-6-methoxyphenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

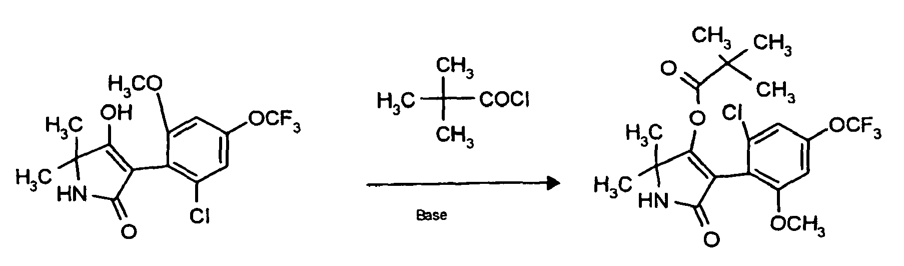

Verwendet man beispielsweise gemäß Verfahren (Bβ) 3-(2,6-Dibrom-4-trifluormethoxy-phenyl)-5,5-dimethylpyrrolidin-2,4-dion und Acetanhydrid als Ausgangsverbindungen, so kann der Verlauf des erfindungsgemäßen Verfahrens durch folgendes Reaktionsschema wiedergegeben werden: 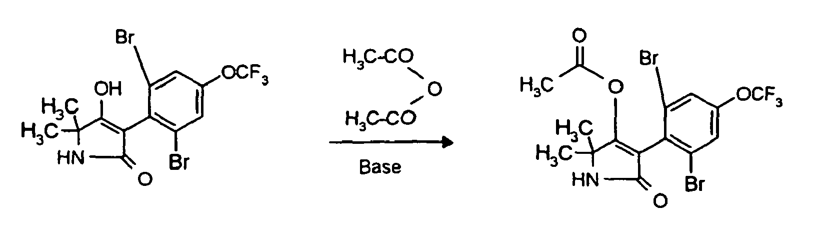 Verwendet man beispielsweise gemäß Verfahren (C) 8-[(2,6-Dichlor-4-trifluormethoxy)-phenyl]-1-aza-bicyclo-(4,3,0^{1,6})-nonan-7,9-dion und Chlorameisensäureethylester als Ausgangsverbindungen, so kann der Verlauf des erfindungsgemäßen Verfahrens durch folgendes Reaktionsschema wiedergegeben werden:

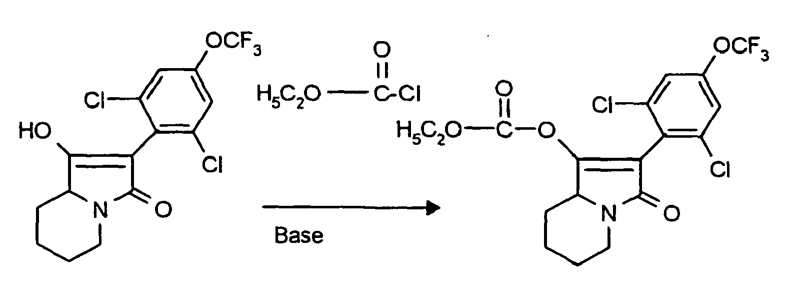

Verwendet man beispielsweise gemäß Verfahren (D), 3-(2,6-Dibrom-4-trifluormethoxy-phenyl)-5,5-dimethyl-6-pyrrolidin-2,4-dion und Chlormonothioameisensäuremethylester als Ausgangsprodukte, so kann der Reaktionsverlauf folgendermaßen wiedergegeben werden:

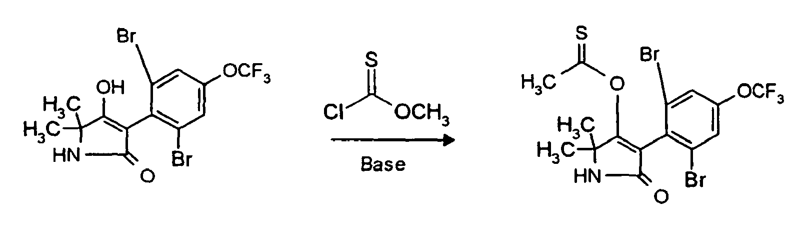

Verwendet man beispielsweise gemäß Verfahren (E) 3-(2,6-Dichlor-4-trifluormethoxy-phenyl)-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

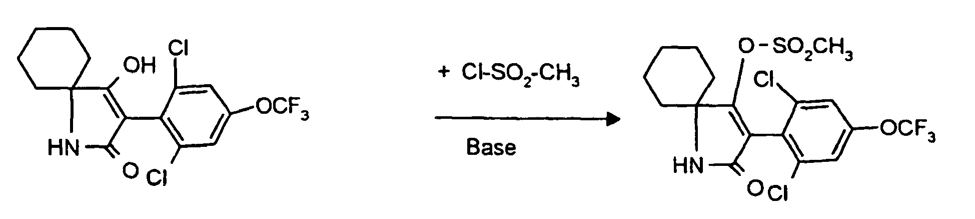

Verwendet man beispielsweise gemäß Verfahren (F) 3-(2-Trifluormethoxy-phenyl)-5,5-dimethyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

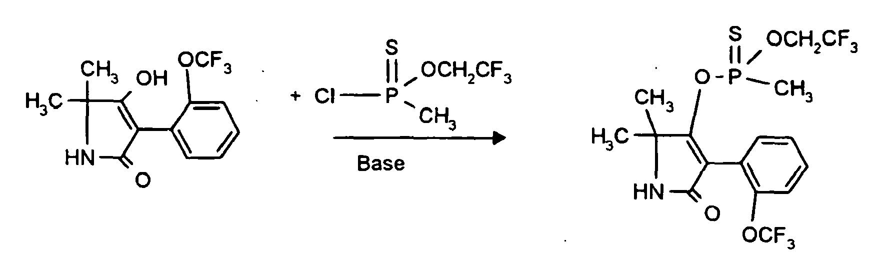

Verwendet man beispielsweise gemäß Verfahren (G) 3-(2-Chlor-4-trifluormethoxy-6-methoxyphenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

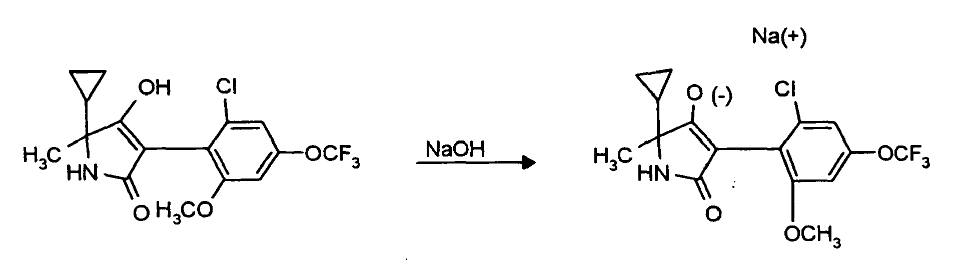

Verwendet man beispielsweise gemäß Verfahren (H) Variante α 3-(2,6-Dibrom-4-thfluonnethoxyphenyl)-5,5-tetramethylen-pyrrolidin-2,4-dion und Ethylisocyanat als Ausgangsprodukte, so kann der Reaktionsverlauf durch folgendes Reaktionsschema wiedergegeben werden:

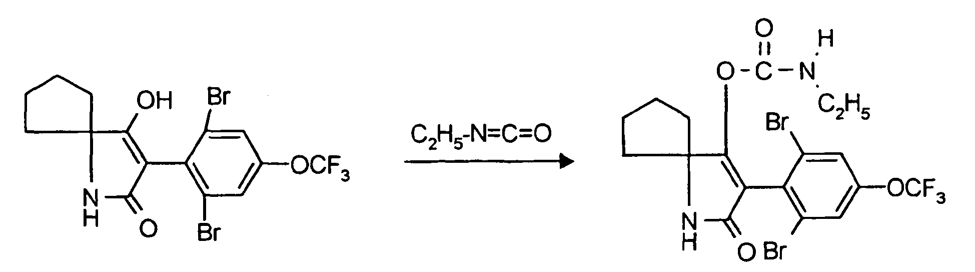

Verwendet man beispielsweise gemäß Verfahren (H) Variante β 3-(2,6-Dichlor-4-trifluormethoxy-phenyl)-5,5-dimethyl-pyrrolidin-2,4-dion und Dimethylcarbamidsäurechlorid als Ausgangsprodukte, so kann der Reaktionsverlauf durch folgendes Schema wiedergegeben werden:

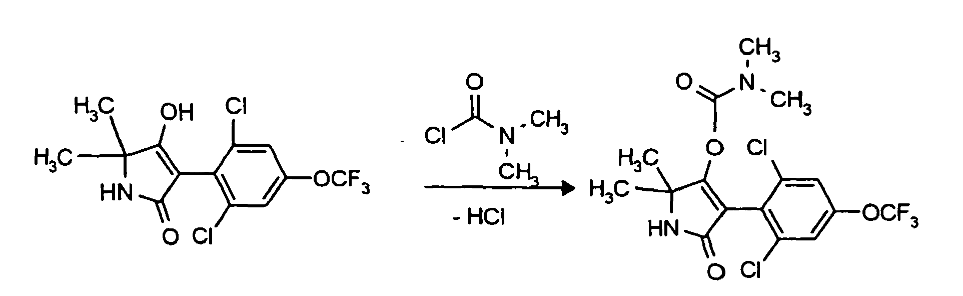

Die beim erfindungsgemäßen Verfahren (a) als Ausgangsstoffe benötigten Verbindungen der Formel (II) 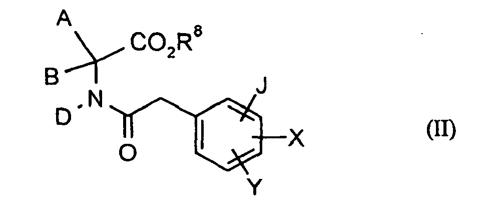 in welcher
A, B, D, J, X, Y und R⁸ die oben angegebenen Bedeutungen haben,
sind neu.
Man erhält die Acylaminosäureester der Formel (II) beispielsweise, wenn man Aminosäurederivate der Formel (XIII) 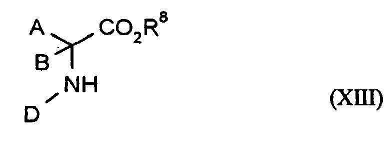 in welcher
A, B, R⁸ und D die oben angegebenen Bedeutungen haben,
mit substituierten Phenylessigsäurederivaten Formel (XIV) 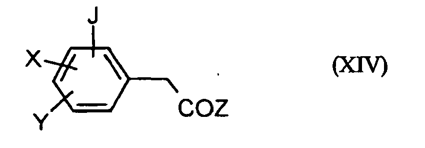 in welcher
- J, X und Y: die oben angegebenen Bedeutungen haben und
- Z: für eine durch Carbonsäureaktivierungsreagenzien wie Carbonyldiimidazol, Carbonyl- diimide (wie z.B. Dicyclohexylcarbondiimid), Phosphorylierungsreagenzien (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52,237-416 (1953); Bhattacharya, Indian J. Chem. 6, 341-5, 1968) oder wenn man Acylaminosäuren der Formel (XV) 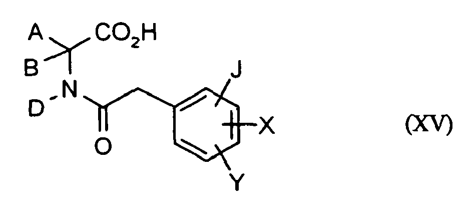 in welcher
- A, B, D, J, X und Y: die oben angegebenen Bedeutungen haben,
verestert (Chem. Ind. (London) 1568 (1968)).

Die Verbindungen der Formel (XV) 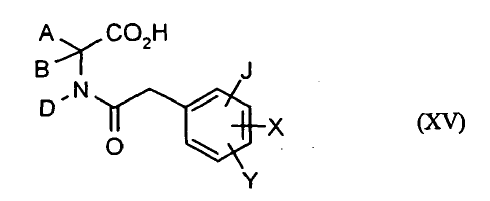 in welcher
- A, B, D, J, X und Y: die oben angegebenen Bedeutungen haben,
sind neu.

Man erhält die Verbindungen der Formel (XV), wenn man Aminosäuren der Formel (XVI) 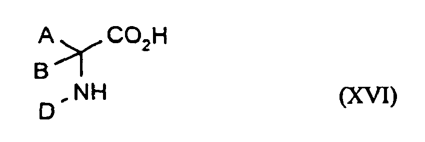 in welcher
- A, B und D: die oben angegebenen Bedeutungen haben,
mit substituierten Phenylessigsäurederivaten der Formel (XIV) 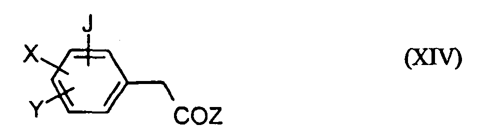 in welcher
- J, X und Y: die oben angegebenen Bedeutungen haben und
- Z: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XIV) sind neu. Sie lassen sich nach im Prinzip bekannten Verfahren und wie aus den Beispielen ersichtlich darstellen (s. z.B. H. Henecka, Houben-Weyl, Methoden der Organischen Chemie, Bd. 8, S. 467-469 (1952)).

Man erhält die Verbindungen der Formel (XIV) beispielsweise, indem man substituierte Phenylessigsäuren der Formel (XVII) 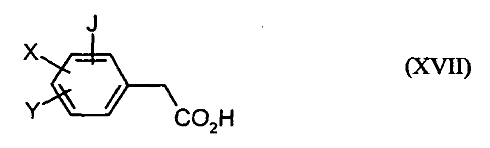 in welcher
- J, X und Y: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XVII) sind teilweise käuflich. Neue Verbindungen der Formel (XVII) werden im experimentellen Teil beschrieben oder lassen sich nach bekannten Verfahren aus den eingangs zitierten Anmeldungen herstellen.

Die Verbindungen der Formel (XIII) und (XVI) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XVI), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 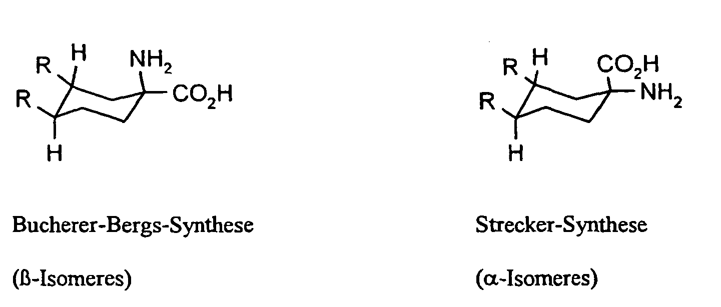 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 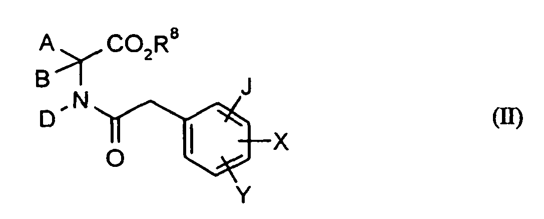 in welcher
A, B, D, J, X, Y und R⁸ die oben angegebenen Bedeutungen haben,
herstellen, wenn man Aminonitrile der Formel (XVIII) 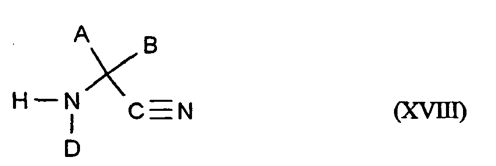 in welcher
- A, B und D: die oben angegebenen Bedeutungen haben,
mit substituierten Phenylessigsäurederivaten der Formel (XIV) 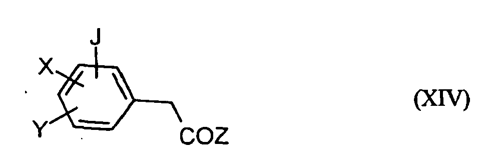 in welcher
- J, X, Y und Z: die oben angegebenen Bedeutungen haben,
zu Verbindungen der Formel (XIX) 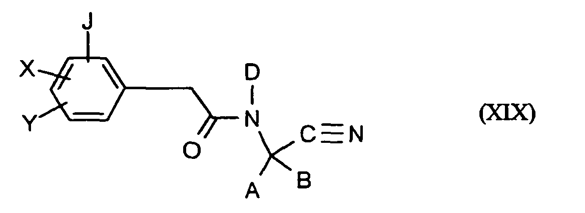 in welcher
- A, B, D, J, X und Y: die oben angegebenen Bedeutungen haben,
umsetzt,
und diese anschließend einer sauren Alkoholyse unterwirft.

Die Verbindungen der Formel (XIX) sind ebenfalls neu.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) sind allgemein bekannte Verbindungen der Organischen bzw. Anorganischen Chemie.

Die Verbindungen der Formeln (XIII), (XVI) und (XVIII) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, J, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B-α) ist dadurch gekennzeichnet, dass man Verbindungen der Formel (I-a) mit Carbonsäurehalogeruden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (B-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium-und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (B-α) innerhalb eines größeren Bereiches variiert werden. Im Allgemeinen arbeitet man bei Temperaturen zwischen - 20°C und +150°C, vorzugsweise zwischen 0°C und 100°C.

Bei der Durchführung des erfindungsgemäßen Verfahrens (B-α) werden die Ausgangsstoffe der Formeln (I-a) und das Carbonsäurehalogenid der Formel (III) im Allgemeinen in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (B-β) ist dadurch gekennzeichnet, dass man Verbindungen der Formel (I-α) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (B-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (B-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (B-β) werden die Ausgangsstoffe der Formel (I-a) und das Carbonsäureanhydrid der Formel (IV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, dass man Verbindungen der Formel (I-a) mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (C) wird im Allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (V) im Allgemeinen in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (D) ist dadurch gekennzeichnet, dass man Verbindungen der Formel (I-a) mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsförm durch Zusatz von starken Deprotonierungsmitteln wie z.B. Natriumhydrid oder Kaliumtertiärbutylat das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, dass man Verbindungen der Formel (I-a) mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formel (I-a) mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, dass man Verbindungen der Formel (I-a) mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (G) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (H) ist dadurch gekennzeichnet, dass man Verbindungen der Formel (I-a) mit (H-α) Verbindungen der Formel (XI) gegebenenfalls in Gegenwart eines Verdünnungsmittels und gegebenenfalls in Gegenwart eines Katalysators oder (H-ß) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (H-α) setzt man pro Mol Ausgangsverbindung der Formel (I-a) ca. 1 Mol Isocyanat der Formel (XI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H-ß) setzt man pro Mol Ausgangsverbindung der Formel (I-a) ca. 1 Mol Carbamidsäurechlorid der Formel (XII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden. Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helininthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitennes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura funiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofinannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:
Fungizide:
   Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanatmethyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefmazoat, Prochloraz, Triflumizol, Viniconazol,
   Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfemaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[1,5-a]pyrimidin-7-amine, N-[1-(5-Brom-3-chloropyridin-2-yl)ethyl]-2,4-dichloronicotinamid, N-(5-Brom-3-chlorpyridin-2-yl)methyl-2,4-dichlomicotinamid, 2-Butoxy-6-iod-3-propyl-benzopyranon-4-on, N-{(Z)-[(cyclopropylmethoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-N-methylacetamid
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Acetylcholinesterase (AChE) Inhibitoren
   Carbamate, zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate, zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/- ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
   Oxadiazine,
   zum Beispiel Indoxacarb
   Semicarbazon,
   zum Beispiel Metaflumizon (BAS3201)
Acetylcholin-Rezeptor-Agonisten/-Antagonisten
   Chloronicotinyle,
   zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
   zum Beispiel Spinosad
GABA-gesteuerte Chlorid-Kanal-Antagonisten
   Organochlorine,
   zum Beispiel Camphechlor, Chlordane, Endosulfan, Gamma-HCH, HCH, Heptachlor, Lindane, Methoxychlor
   Fiprole,
   zum Beispiel Acetoprole, Ethiprole, Fipronil, Pyrafluprole, Pyriprole, Vaniliprole
Chlorid-Kanal-Aktivatoren
   Mectine,
   zum Beispiel Abamectin, Emamectin, Emamectin-benzoate, Ivermectin, Lepimectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
   zum Beispiel Chromafenozide, Halofenozide, Methoxyfenozide, Tebufenozide
Inhibitoren der Chitinbiosynthese
   Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
   Pyrrole,
   zum Beispiel Chlorfenapyr
   Dinitrophenole,
   zum Beispiel Binapacyrl, Dinobuton, Dinocap, DNOC, Meptyldinocap
Seite-I-Elektronentransportinhibitoren
   METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen,
   Tetramsäuren, zum Beispiel Spirotetramat, cis-3-(2,5-dimethylphenyl)-4-hydroxy-8-methoxy-1-azaspiro[4.5]dec-3-en-2-on
   Carboxamide,
   zum Beispiel Flonicamid
   Oktopaminerge Agonisten,
   zum Beispiel Amitraz
Inhibitoren der Magnesium-stimulierten ATPase,
   Propargite
   Nereistoxin-Analoge,
   zum Beispiel Thiocyclam hydrogen oxalate, Thiosultap-sodium
Agonisten des Ryanodin-Rezeptors,
   Benzoesäuredicarboxamide,
   zum Beispiel Flubendiamid
   Anthranilamide,
   zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
   zum Beispiel Aluminium phosphide, Methyl bromide, Sulfuryl fluoride
   Fraßhemmer,
   zum Beispiel Cryolite, Flonicamid, Pymetrozine
   Milbenwachstumsinhibitoren,
   zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.% Wirkstoff, vorzugsweise zwischen 0,00001 und 1 Gew.% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur; Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestem, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise 2-Chloro-5-[2,6-dioxo-4-(trifluormethyl)-3,6-dihydropyrimidin-1(2H)-yl]-4-fluor-N-[methyl(1-methylethyl)sulfamoyl]benzamid, Acetochlor, Acifluorfen (-sodium), Aclonifen, Alachlor, Alloxydim (-sodium), Ametryne, Amicarbazone, Amidochlor, Amidosulfuron, Aminöpyralid, Anilofos, Asulam, Atrazine, Azafenidin, Azimsulfuron, Beflubutamid, Benazolin (-ethyl), Benfuresate, Bensulfuron (-methyl), Bentazon, Bencarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (- ethyl), Chlomitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, - sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz -methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (- ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (- sodium), Pyroxasulfone, Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thifensulfuron (- methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron, und 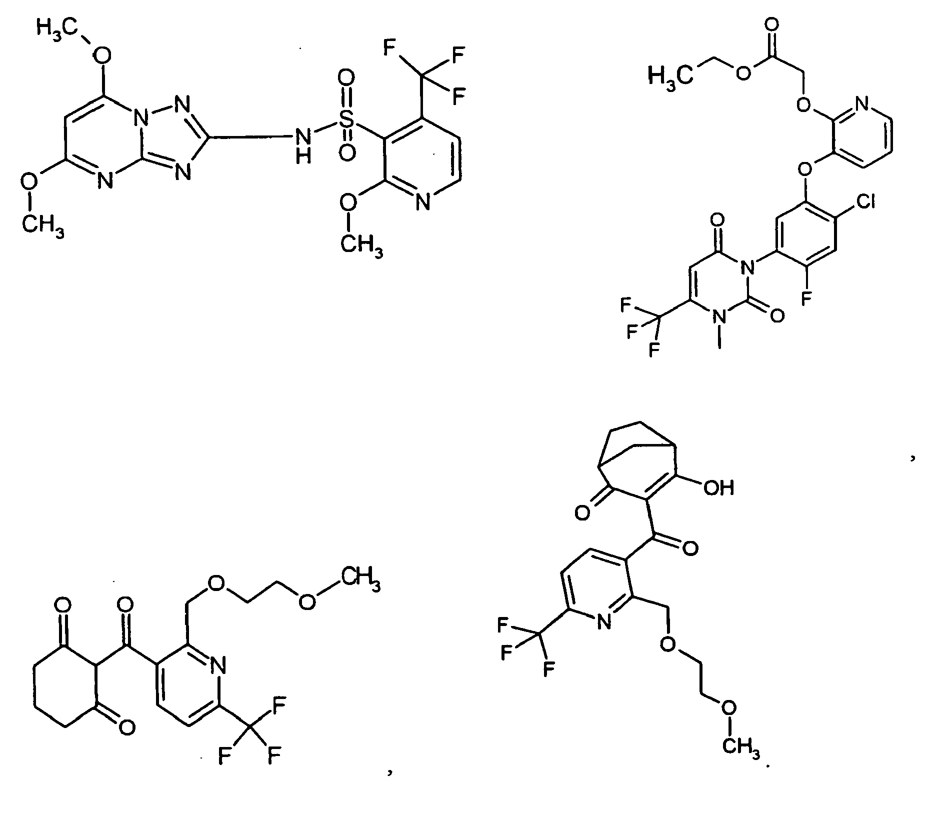 Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Die Bezeichnung "Wirkstoffe" schließt immer auch die hier genannten Wirkstoffkombinationen mit ein.

Die Herstellung und die Verwendung der erfindungsgemäßen Wirkstoffe geht aus den nachfolgenden Beispielen hervor.

### Anwendungsbeispiele

### Beispiel I-a-1

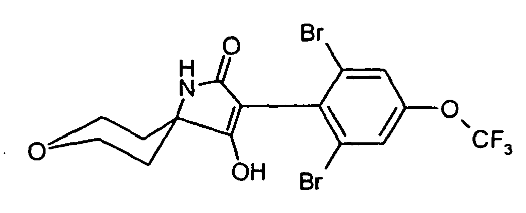

Es werden unter Argon 4,26 g Kalium-tert-butylat (36 mmol) in 10 ml Dimethylacetamid vorgelegt. Bei -20 bis -30°C tropft man 8,5 g (16,4 mMol) der Verbindung gemäß Beispiel II-1 in 15 ml Dimethylacetamid zu. Man rührt 1 Stunde bei 20°C (dünnschichtchromatographische Kontrolle). Nach Reaktionsende wird die Reaktionslösung in 100 ml Eiswasser eingerührt, mit konzentrierter Salzsäure auf pH2 gestellt und der Niederschlag abgesaugt. Der Niederschlag wird nochmals in 40 ml Dichlormethan gelöst und 20 ml 0,5 N NaOH-Lösung zugetropft. Man rührt 1 Stunde bei 20°C. Die Wasserphase wird auf pH 2 gestellt und der Niederschlag abgesaugt.

Es erfolgt Reinigung über Säulenchromatographie an Kieselgel (Dichlormethan:Essigsäureethylester = 5:3).

Ausbeute: 4,2 g (51 % d. Theorie), Fp. 259°C.

In Analogie zu Beispiel (I-a-1) und gemäß den allgemeinen Angaben zur Herstellung erhält man folgende Verbindungen der Formel (I-a)

| | | | | | | | | |
|---|---|---|---|---|---|---|---|---|
| 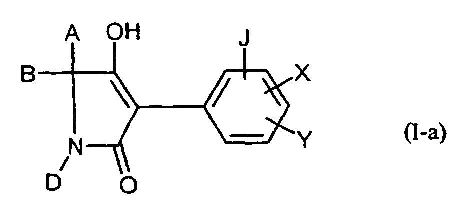 | | | | | | | | |

| Bsp.- Nr. | J | X | Y | D | A | B | Fp °C | Isomer |
|---|---|---|---|---|---|---|---|---|
| I-a-2 | 2-OCF₃ | H | H | H | -(CH₂)₂-CHOCH₃-(CH₂)₂- | | 228 | β |
| I-a-3 | 5-OCF₃ | 2-Br | H | H | -(CH₂)₂-CHOCH₃-(CH₂)₂- | | 258 | β |
| I-a-4 | 5-OCF₃ | 2-Br | H | H | -(CH₂)₂-O-(CH₂)₂- | | 138 | - |
| I-a-5 | 5-OCF₃ | 2-Br | H | H | 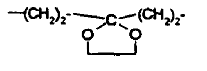 | | 230 | - |
| I-a-6 | 5-OCF₃ | 2-Br | H | H | -(CH₂)₂-CHOC₂H₅-(CH₂)₂- | | 96 | β |
| I-a-7 | 4-OCF₃ | 2-Br | 6-Br | H | -CH₂-CHOCH₃-(CH₂)₃- | | 372 | α |
| I-a-8 | 4-OCF₃ | 2-Br | 6-CH₃ | H | -(CH₂)₂-O-(CH₂)₂- | | *1.46-1.57(m,2H,CH₂); 2.20(s,3H,Ar-CH₃); 3.73-3.79(m,2H,OCH₂); 3.99-4.03(m,2H,OCH₂); 7.42-7.43 (d,1H,ArH) | |
| I-a-9 | 4-OCH₃ | 2-Br | 6-Br | H | -(CH₂)₂-CHOC₂H₅-(CH₂)₂- | | 170 | β |
| I-a-10 | 4-OCF₃ | 2-Br | 6-Cl | H | CH₃ | CH₃ | 108-111 | - |
| I-a-11 | 4-OCF₃ | 2-Br | 6-Cl | H | -CH₂-CHOC₄H₉-(CH₂)₃- | | 110-112 | β |
| I-a-12 | 4-OCF₃ | 2-Br | 6-Cl | -(CH₂)₃- | | | 220-221 | - |
| I-a-13 | 4-OCF₃ | 2-Br | 6-Cl | 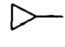 | CH₃ | | 100-102 | - |
| I-a-14 | 4-OCF₃ | 2-Br | 6-Br | H | -CH₂-CHOC₄H₉-(CH₂)₃- | | 106-110 | β |
| I-a-15 | 4-OCF₃ | 2-Br | 6-Br | -(CH₂)₃- | | H | 229-231 | - |
| I-a-16 | 4-OCF₃ | 2-Br | 6-Br | 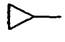 | CH₃ | H | 108-112 | - |
| I-a-17 | 4-OCF₃ | 2-Br | 6-Br | H | 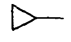 | CH₃ | 98-100 | - |
| I-a-18 | 4-OCF₃ | 2-Br | 6-Br | H | C₂H₅ | CH₃ | 85-87 | - |
| I-a-19 | 4-OCF₃ | 2-Br | 6-Cl | H | 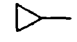 | CH₃ | 221-225 | - |
| I-a-20 | 4-OCF₃ | 2-Br | 6-Cl | H | C₂H₅ | CH₃ | 209-212 | - |
| I-a-21 | 4-OCF₃ | 2-OCH₃ | 6-Br | H | CH₃ | CH₃ | | - |
| I-a-22 | 4-OCF₃ | 2-OCH₃ | 6-Br | H | -(CH₂)₂-CHOCH₃-(CH₂)₂- | | 104-106 | β |
| I-a-23 | 4-OCF₃ | 2-OCH₃ | 6-Cl | H | CH₃ | CH₃ | 133-136 | - |
| I-a-24 | 4-OCF₃ | 2-OCH₃ | 6-Cl | H | -CH₂-CHOC₄H₉-(CH₂)₃- | | 93-96 | β |
| I-a-25 | 4-OCF₃ | 2-OCH₃ | 6-Cl | -(CH₂)₃- | | H | 170-172 | - |
| I-a-26 | 4-OCF₃ | 2-OCH₃ | 6-Cl, | H | -(CH₂)₂-CHOCH₃-(CH₂)₂- | | 170-173 | β |
| I-a-27 | 4-OCF₃ | 2-OCH₃ | 6-Cl | H | 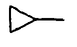 | CH₃ | 79-82 | - |
| I-a-28 | 4-OCF₃ | 2-OCH₃ | 6-Cl | H | -(CH₂)₂-O-(CH₂₎₂- | | 121 | - |
| I-a-29 | 4-OCF₃ | 2-Cl | 6-Cl | H | -(CH₂)₂-CHOCH₃-(CH₂)₂- | | 282 | β |
| I-a-30 | 4-OCF₃ | 2-Cl | 6-Cl | H | -(CH₂)₂-O-(CH₂)₂- | | 251 | - |
| I-a-31 | 4-OCF₃ | 2-Br | 6-Br | H | CH₃ | CH₃ | 216 | - |
| I-a-32 | 4-OCF₃ | 2-Br | 6-Br | H | -(CH₂)₂-CHOCH₃-(CH₂)₂- | | 280 | β |
| I-a-33 | 4-OCF₃ | 2-Br | 6-Br | H | -CH₂-CHOCH₃-(CH₂)₃- | | Wachs | β |
| I-a-34 | 5-OCF₃ | 2-Br | H | H | 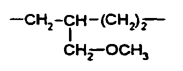 | | 125 | β |
| I-a-35 | 5-OCF₃ | 2-Br | H | H | 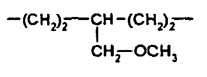 | | 189 | β |
| I-a-36 | 4-OCF₃ | 2-OCH₃ | 6-OCH₃ | H | -(CH₂)₂-CHOCH₃-(CH₂)₂- | | *** 3.32(m,1H,CHOCH₃) ; 6.40(s,2H,ArH) | β |
| I-a-37 | 4-OCF₃ | 2-CH₃ | 6-CH₃ | H | -CH₂-CHOC₂H₅-(CH₂)₃- | | 109-112 | β |
| I-a-38 | 4-OCF₃ | 2-CH₃ | 6-CH₃ | H | -(CH₂)₂-CHOCH₃-(CH₂)₂- | | 263-265 | β |
| I-a-39 | 4-OCF₃ | 2-Cl | 6-CH₃ | H | -(CH₂)₂-CHOCH₃-(CH₂)₂- | | 270 | β |
| I-a-40 | 4-OCF₃ | 2-Cl | 6-CH₃ | H | -CH₂-CHOCH₃-(CH₂)₂- | | 202-206 | cis |
| I-a-41 | 4-OCF₃ | 2-Cl | 6-CH₃ | H | -CH₂-CHOCH₃-(CH₂)₂- | | 75-95 | trans |
| I-a-42 | 4-OCF₃ | 2-OCH₃ | 6-Cl | H | 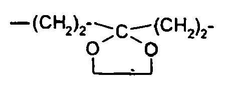 | | Zersetzung | - |
| I-a-43 | 4-OCF₃ | 2-Cl | 6-Cl | H | 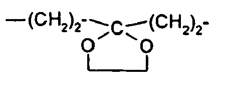 | | 272 | - |
| I-a-44 | 4-OCF₃ | 2-Br | 6-Br | H | 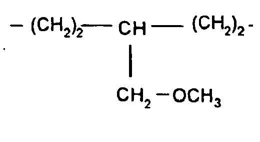 | | 240 | β |
| I-a-45 | 4-OCF₃ | 2-Br | 6-Br | H | 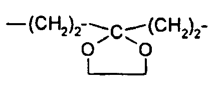 | | 291 | - |
| I-a-46 | 2-OCF₃ | 4-Br | H | H | -(CH₂)₂-CHOCH₃-(CH₂)₂- | | 253 | β |
| I-a-47 | 2-OCF₃ | 4-Cl | H | H | -(CH₂)₂-O-(CH₂)₂- | | 245 | - |
| I-a-48 | 2-OCF₃ | 6-Cl | 4-Br | H | -(CH₂)₂-O-(CH₂)₂- | | 258 | - |
| I-a-49 | 2-OCF₃ | 6-CH₃ | 4-CH₃ | H | -(CH₂)₂-CHOCH₃-(CH₂)₂- | | 216-219 | β |
| I-a-50 | 2-OCF₃ | 6-CH₃ | 4-CH₃ | H | -CH₂-CHOCH₃-(CH₂)₃- | | 189-192 | β |
| I-a-51 | 2-OCF₃ | 6-OCH₃ | 4-Br | H | -(CH₂)₂-O-(CH₂)₂- | | 208 | - |
| I-a-52 | 2-OCF₃ | 4-Cl | H | H | -(CH₂)₂-CHOCH₃-(CH₂)₂- | | 239 | β |
| I-a-53 | 2-OCF₃ | 6-Br | 4-Cl | H | -(CH₂)₂-O-(CH₂)₂- | | 275 | - |
| I-a-54 | 2-OCF₃ | 6-OCH₃ | 4-Cl | H | -(CH₂)₂-O-(CH₂)₂- | | 224 | - |
| I-a-55 | 2-OCF₃ | 6-Cl | 4-CH₃ | H | -(CH₂)₂-O-(CH₂)₂- | | ** 1.2-1.34 (m,2H,CH₂) - 2.37 (s,3H-ArCH₃ 3.83-3.88 (m,2H,OCH₂), 7.14,7.34 (2s,2H,ArH) | - |
| I-a-56 | 2-OCF₃ | 4-Br | H | H | -(CH₂)₂-O-(CH₂)₂- | | 246 | - |
| I-a-57 | 2-OCF₃ | 6-Cl | H | H | -(CH₂)₂CHOCH₃-(CH₂)₂- | | 254 | β |
| I-a-58 | 2-OCF₃ | 6-Br | 4-Cl | H | -(CH₂)₂-CHOCH₃-(CH₂)₂- | | 280 | β |
| I-a-59 | 2-OCF₃ | 6-Br | 4-Br | H | -(CH₂)₂-CHOCH₃-(CH₂)₂- | | 280 | β |
| I-a-60 | 2-OCF₃ | 6-Cl | 4-Br | H | -CH₂-CHOC₂H₅-(CH₂)₃- | | 73 | β |
| I-a-61 | 2-OCF₃ | 6-OCH₃ | 4-Cl | H | -(CH₂)₂-CHOCH)-(CH₂)₂- | | 196-198 | β |
| I-a-62 | 2-OCF₃ | 6-Cl | 4-CH₃ | H | -(CH₂)₂-CHOCH)-(CH₂)₂- | | 209-210 | β |
| I-a-63 | 2-OCF₃ | 6-Cl | 4-CH₃ | H | -CH₂-CHOC₂H₅-(CH₂)₃- | | 189-192 | β |
| I-a-64 | 2-OCF₃ | 6-Cl | 4-CH₃ | H | -CH₂-CHOC₃H₇-(CH₂)₃- | | 105-108 | β |
| I-a-65 | 2-OCF₃ | 6-OCH₃ | 4-Cl | H | -CH₂-CHOC₂H₅-(CH₂)₃- | | 89-91 | β |
| I-a-66 | 2-OCF₃ | 6-C₂H₅ | 4-Cl | H | -(CH₂)₂-CHOCH₃-(CH₂)₂ | | 237-240 | β |
| I-a-67 | 2-OCF₃ | 6-C₂H₅ | 4-Cl | H | 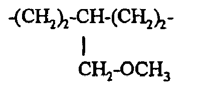 | | 201-204 | β |
| I-a-68 | 2-OCF₃ | 6-CH₃ | 4-Cl | H | -CH₂-CHOC₂H₅-(CH₂)₃- | | 222-223 | β |
| I-a-69 | 2-OCF₃ | 6-CH₃ | 4-Cl | H | -(CH₂)₂-CHOCH₃-(CH₂)₂- | | 280-282 | β |
| I-a-70 | 2-OCF₃ | 6-Cl | 4-Br | H | 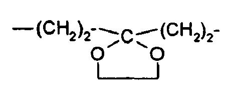 | | 209 | - |
| I-a-71 | 2-OCF₃ | 6-Cl | 4-Br | H | CH₃ | CH₃ | 181 | - |
| I-a-72 | 2-OCF₃ | 6-Cl | 4-Br | H | 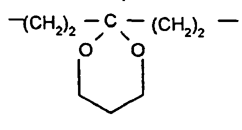 | | 257 | - |
| I-a-73 | 2-OCF₃ | 6-C₂H₅ | 4-Cl | gCH₂)₃--(CH₂)₃- | | H | 217-220 | |

| | | | | | | | | |
|---|---|---|---|---|---|---|---|---|
| *¹H-NMR (400 MHz, d₄-Methanol): Verschiebungen δ in ppm ** ¹H-NMR (400 MHz, d₆-DMSO): Verschiebungen δ in ppm *** ¹H-NMR (400 MHz, CDCl₃): Verschiebungen δ in ppm | | | | | | | | |

### Beispiel I-b-1

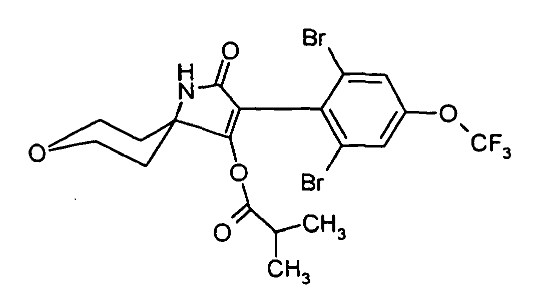

0,5 g (1 mmol) der Verbindung gemäß Bsp. I-a-1 in Essigsäureethylester unter Argon vorlegen, 0,14 ml Triethylamin plus 10 mg Steglich-Base zugeben und bei Rückfluss 0,1 ml 2-Methylpropionylchlorid in 5 ml Essigsäureethylester gelöst zutropfen, dann unter Rückfluss nachrühren.

Nach Reaktionsende (dünnschichtchromatographische Kontrolle) erfolgt eine RP-Säulentrennung (Wasser/Acetonitril:50/50 → 10/90).

Ausbeute: 0,23 g (40 % d.Theorie), Fp 219°C.

In Analogie zu Beispiel (I-b-1) und gemäß den allgemeinen Angaben zur Herstellung erhält man folgende Verbindungen der Formel (1-b):

| | | | | | | | | | |
|---|---|---|---|---|---|---|---|---|---|
| 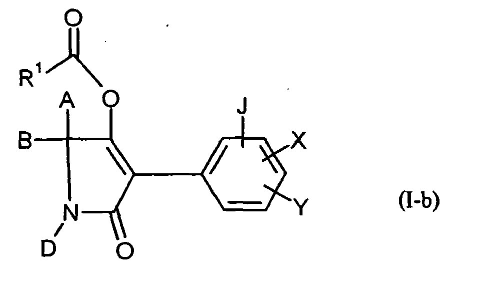 | | | | | | | | | |

| Bsp.- Nr. | J | X | Y | D | A | B | R¹ | Fp °C | Isomer |
|---|---|---|---|---|---|---|---|---|---|
| I-b-2 | 5-OCF₃ | 2-Br | H | H | -(CH₂)₂-CHOCH₃-(CH₂)₂- | | i-C₃H₇ | 165 | β |
| I-b-3 | 5-OCF₃ | 2-Br | H | H | 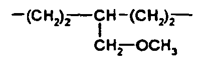 | | i-C₃H₇ | 156 | β |
| I-b-4 | 4-OCF₃ | 2-Br | 6-Cl | 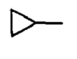 | CH₃ | H | i-C₃H₇ | Öl, * 1) | - |
| I-b-5 | 4-OCF₃ | 2-Br | 6-Br | H | -CH₂-CHOC₄H₉-(CH₂)₃- | | i-C₃H₇ | 182-185 | β |
| I-b-6 | 4-OCF₃ | 2-Br | 6-Cl | H | 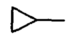 | CH₃ | i-C₃H₇ | 156-159 | - |
| I-b-7 | 4-OCF₃ | 2-Br | 6-Br | H | 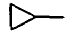 | CH₃ | i-C₃H₇ | 150-156 | - |
| I-b-8 | 4-OCF₃ | 2-Br | 6-Br | H | C₂H₅ | CH₃ | i-C₃H₇ | 129-132 | - |
| I-b-9 | 4-OCF₃ | 2-Br | 6-Cl | H | -CH₂-CHOC₄H₉-(CH₂)₃- | | i-C₃H₇ | 183-186 | β |
| I-b-10 | 4-OCF₃ | 2-Br | 6-Br | -(CH₂)₃- | | H | i-C₃H₇ | ** 2) | - |
| I-b-11 | 4-OCH₃ | 2-Br | 6-Cl | 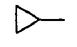 | CH₃ | H | i-C₃H₇ | ** 3) | - |
| I-b-12 | 4-OCF₃ | 2-Br | 6-Cl | H | C₂H₅ | CH₃ | i-C₃H₇ | 135-141 | - |
| I-b-13 | 4-OCF₃ | 2-OCH₃ | 6-Br | H | -(CH₂)₂-CHOCH₃-(CH₂)₂- | | i-C₃H₇ | 204 | β |
| I-b-14 | 4-OCF₃ | 2-Br | 6-Cl | -(CH₂)₃- | | H | i-C₃H₇ | ** 4) | - |
| I-b-15 | 4-OCF₃ | 2-OCH₃ | 6-Cl | H | -CH₂-CHOC₄H₉-(CH₂)₃- | | i-C₃H₇ | 178-186 | β |
| I-b-16 | 4-OCF₃ | 2-OCH₃ | 6-Cl | -(CH₂)₃- | | H | i-C₃H₇ | ** 5) | - |
| I-b-17 | 4-OCF₃ | 2-OCH₃ | 6-Cl | H | -(CH₂)₂-CHOCH₃-(CH₂)₂- | | i-C₃H₇ | 198 | β |
| I-b-18 | 4-OCF₃ | 2-OCH₃ | 6-Cl | H | 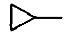 | CH₃ | i-C₃H₇ | 153-174 | - |
| I-b-19 | 4-OCF₃ | 2-OCH₃ | 6-Cl | H | CH₃ | CH₃ | i-C₃H₇ | 110-118 | - |
| I-b-20 | 4-OCF₃ | 2-Cl | 6-Cl | H | -(CH₂)₂-CHOCH₃-(CH₂)₂- | | i-C₃H₇ | 190 | β |
| I-b-21 | 4-OCH₃ | 2-Cl | 6-CH₃ | H | -CH₂)-CHOCH₃-(CH₂)₂- | | CH₃ | 212 | β |
| I-b-22 | 4-OCH₃ | 2-Cl | 6-CH₃ | H | -CH₂)₂-CHOCH₃-(CH₂)₂- | | i-C₃H₇ | 159-161 | β |
| I-b-23 | 4-OCF₃ | 2-Br | 6-Br | H | 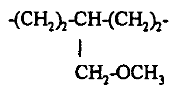 | | i-C₃H₇ | 204 | β |
| I-b-24 | 2-OCF₃ | 6-CH₃ | 4-CH₃ | H | -CH₂-CHOC₂H₅-(CH₂)₃- | | CH₃ | 228-230 | β |
| I-b-25 | 2-OCF₃ | 6-CH₃ | 4-CH₃ | H | -(CH₂)₂-CHOCH₃-(CH₂)₂- | | CH₃ | 202-205 | β |
| I-b-26 | 2-OCF₃ | 6-CH₃ | 4-CH₃ | H | gCH₂)₂-CHOCH₃-(CH₂)- | | i-C₃H₇ | 207 | β |
| I-b-27 | 2-OCF₃ | 6-C₂H₅ | 4-Cl | H | 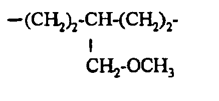 | | i-C₃H₇ | * 6) | β |
| I-b-28 | 2-OCF₃ | 6-CH₃ | 4-Cl | H | -(CH₂)₂-CHOCH₃-(CH₂)₂- | | CH₃ | * 7) | β |

| | | | | | | | | | |
|---|---|---|---|---|---|---|---|---|---|
| 1) 2.61 (m, 1H, CH(C5)); 2.70 (m,1H (CH(CH₃)₂); 7.31, 7.44 (je dd, 1H, ArH) 2) 2.70 (m, 1H, CH (CH₃)₂); 4.79 (m, 1H, CH(C5); 7.47 (dd, 2-H, ArH) 3) 2.62 (m, 1H CH(C5); 2.70 (m, 1H, CH(CH₃)₂; 7.47 (dd, 1H ArH) 4) 2.67 (m, 1H CH(CH₃)₂; 4.75 (m, 1H, CH(C5); 7,31, 7.43 (je dd, 1H, ArH) 5) 2.66 (m, 1H CH(CH₃)₂; 4.70 (m, 1H, CH(C5); 6.68, 6.96 (je dd, 1H, ArH) 6) 2.62 (m, 1H, CH(CH₃)₂); 2.71 (m, 2H, Ar CH₂(CH₃); 3.36 (s,3H, NOCH₃) 7) 2.1 (s, 3H, COCH₃); 2.33 (s, 3H, ArCH₃); 3.40 (s, 3H, OCH₃) *** ¹H-NMR (400 MHz, CDCl₃): Verschiebung δ in ppm** **** ¹H-NMR (300 MHz, CDCl₃): Verschiebung δ in ppm** | | | | | | | | | |

### Beispiel I-c-1

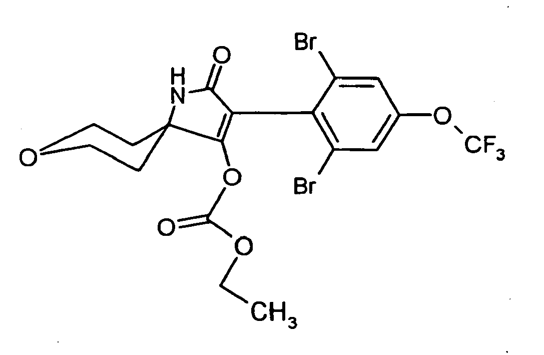

Es werden 0,5 g (1 mmol) der Verbindung gemäß Bsp. I-a-1 unter Argon in Methylenchlorid vorgelegt, 0,14 ml Triethylamin zugegeben und bei ca. 20°C den Chlorameisensäureester (0,1 ml) gelöst in 5 ml Dichlormethan zugetropft und bei 20 bis 30°C nachgerührt.

Nach Reaktionsende (dünnschichtchromatographische Kontrolle) erfolgt eine RP-Säulentrennung (Wasser/Acetonitril:50/50 → 10/90).

Ausbeute: 0,415 g (74 % d.Theorie), Fp 194°C.

In Analogie zu Beispiel (I-c-1) und gemäß den allgemeinen Angaben zur Herstellung erhält man folgende Verbindungen der Formel (1-c):

| | | | | | | | | | | | |
|---|---|---|---|---|---|---|---|---|---|---|---|
| 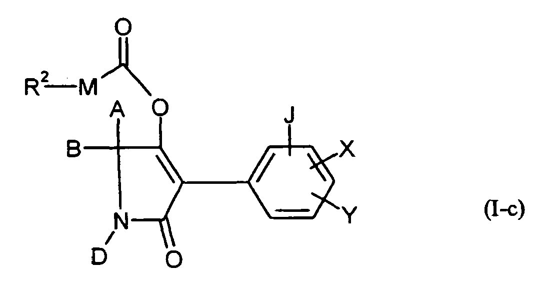 | | | | | | | | | | | |

| Bsp.- Nr. | J | X | Y | D | A | B | | M | R² | Fp°C | Isomer |
|---|---|---|---|---|---|---|---|---|---|---|---|
| I-c-2 | 5-OCF₃ | 2-Br | H | H | -(CH₂)₂-CHOCH₃-(CH₂)₂- | | | O | C₂H₅ | 161 | β |
| I-c-3 | 5-OCF₃ | 2-Br | H | H | 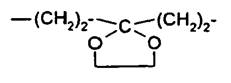 | | | O | C₂H₅ | 201 | - |
| I-c-4 | 5-OCF₃ | 2-Br | H | H | 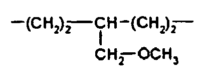 | | | O | C₂H₅ | 182 | β |
| I-c-5 | 4-OCF₃ | 2-Br | 6-Br | H | -(CH₂)₂-CHOCH₃-(CH₂)₂- | | | O | C₂H₅ | 166 | β |
| I-c-6 | 4-OCF₃ | 2-Br | 6-Cl | H | -CH₂-CHOC₄H₉-(CH₂)₃- | | | O | C₂H₅ | 184 | β |
| I-c-7 | 4-OCF₃ | 2-Br | 6-Cl | -(CH₂)₃- | | | H | O | C₂H₅ | * 1) | - |
| I-c-8 | 4-OCF₃ | 2-Br | 6-Cl | 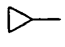 | CH₃ | | H | O | C₂H₅ | zähes Öl-* 2) | - |
| I-c-9 | 4-OCF₃ | 2-Br | 6-Br | H | -CH₂-CHOC₄H₉-(CH₂)₃- | | | O | C₂H₅ | 201 | β |
| I-c-10 | 4-0CF₃ | 2-Br | 6-Br | -(CH₂)₃- | | | H | O | C₂H₅ | zähes Öl-* 3) | - |
| I-c-I11 | 4-OCF₃ | 2-Br | 6-Br | 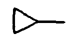 | CH₃ | | H | O | C₂H₅ | zähes Öl-*4) | - |
| I-c-12 | 4-OCF₃ | 2-Br | 6-Cl | H | 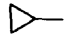 | | CH₃ | O | C₂H₅ | 143 | - |
| I-c-13 | 4-OCF₃ | 2-Br | 6-Br | H | 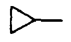 | | CH₃ | O | C₂H₅ | 126-130 | - |
| I-c-14 | 4-OCF₃ | 2-Br | 6-Br | H | C₂H₅ | | CH₃ | O | C₂H₅ | 110-114 | - |
| I-c-15 | 4-OCF₃ | 2-Br | 6-Cl | H | C₂H₅ | | CH₃ | O | C₂H₅ | 130-131 | - |
| I-c-16 | 4-OCF₃ | 2-OCH₃ | 6-Br | H | -(CH₂)₂-CHOCH₃-(CH₂)₂- | | | O | C₂H₅ | 177-179 | β |
| I-c-17 | 4-OCF₃ | 2-OCH₃ | 6-Cl | H | -CH₂-CHOC₄H₉-(CH₂)₃- | | | O | C₂H₅ | 188-190 | β |
| I-c-18 | 4-OCF₃ | 2-OCH₃ | 6-Cl | H | -(CH₂)₂-CHOCH₃-(CH₂)₂- | | | O | C₂H₅ | 147-150 | β |
| I-c-19 | 4-OCF₃ | 2-OCH₃ | 6-Cl | -(CH₂)₃- | | | H | O | C₂H₅ | 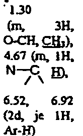 | - |
| I-c-20 | 4-OCF₃ | 2-Cl | 6-Cl | H | | -(CH₂)₂-CHOCH₃-(CH₂)₂- | | O | C₂H₅ | 164 | β |
| I-c-21 | 4-OCF₃ | 2-Cl | 6-Cl | H | | -(CH₂)₂-O-(CH₂)₂- | | O | C₂H₅ | 212 | - |
| I-c-22 | 4-OCF₃ | 2-CH₃ | 6-CH₃ | H | | -(CH₂)₂-CHOCH₃-(CH₂)₂- | | O | C₂H₅ | 182-183 | β |
| I-c-23 | 4-OCF₃ | 2-CH₃ | 6-CH₃ | H | | -CH₂-CHOC₂H₅-(CH₂)₃- | | O | C₂H₅ | 205-206 | β |
| I-c-24 | 4-OCH₃ | 2-Cl | 6-CH₃ | H | | -CH₂-CHOCH₃-(CH₂)₂- | | O | C₂H₅ | 86-89 | cis |
| I-c-25 | 4-OCF₃ | 2-Cl | 6-CH₃ | H | | -CH₂-CHOCH₃-(CH₂)₂- | | O | C₂H₅ | 146-149 | trans |
| I-c-26 | 4-OCF₃ | 2-Cl | 6-CH₃ | H | | -(CH₂)₂-CHOCH₃-(CH₂)₂- | | O | CH₅ | 163-165 | β |
| I-c-27 | 4-OCF₃ | 2-Br | 6-Br | H | | 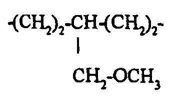 | | O | C₂H₅ | 201 | β |
| I-c-28 | 2-OCF₃ | 6-2Hs | 4-Cl | -(CH₂)₃₆- | | H | O | CH₂₋ C₆H₅ | * 5) | - | |
| I-c-29 | 2-OCF₃ | 6-CH₃ | 4-CH₃ | H | -(CH₂)₂-CHOCH₃-(CH₂)₂- | | O | C₂H₅ | 210-212 | β | |
| I-c-30 | 2-OCF₃ 3 | 6-CH₃ | 4-CH₃ | H | -(CH₂-CHOC₂H₅-(CH₂)₂- | | O | C₂H₅ | 203 | β | |
| In-31 | 2-OCF₃ | 6-OCH₃ | 4-Cl | H | -(CH₂)₂-CHOCH₃-(CH₂)₂- | | O | C₂H₅ | 168-170 | β | |
| I-c-32 | 2-OCF₃ | 6-C₂H₅ | 4-Cl | H | -(CH₂)₂-CHOCH₃-(CH₂)₂- | | O | C₂H₅ | 139-141 | β | |
| I-c-33 | 2-OCF₃ | 6-C₂H₅ | 4-Cl | H | 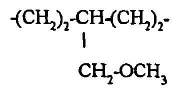 | | O | C₂H₅ | 160-162 | β | |
| I-c-34 | 2-OCF₃ | 6-CH₃ | 4-Cl | H | -CH₂-CHOC₂H₅-(CH₂)₃- | | O | C₂H₅ | 186-188 | β | |
| I-c-35 | 2-OCF₃ | 6-OCH₃ | 4-Cl | H | -CH₂-CHOC₂H₅-(CH₂)₃- | | O | C₂H₅ | 186-187 | β | |
| I-c-36 | 2-OCF₃ | 6-Cl | 4-CH₃ | H | -(CH₂)₂-CHOCH₃-(CH₂)₂- | | O | C₂H₅ | 227-228 | β | |
| I-c-37 | 2-OCF₃ | 6-Cl | 4-CH₃ | H | -CH₂-CHOC₂H₅-(CH₂)₃- | | O | C₂H₅ | 144-145 | β | |
| I-c-38 | 2-0CF₃ | 6-CH₃ | 4-Cl | H | -(CH₂)₂-CHOCH₃-(CH₂)₂- | | O | C₂H₅ | * 6 | β | |
| I-c-39 | 2-OCF₃ | 6-C₂H₅ | 4-Cl | H | -(CH₂)₂-CHOCH₃-(CH₂)₂- | | O | CH₃ | * 7 | β | |

| | | | | | | | | | | | |
|---|---|---|---|---|---|---|---|---|---|---|---|
| 1) 4.29 (m, 2H, OCH₂-CH₃); 4.81 (m, 1H, CH (C5)); 7.33, 7.47 (je dd, 1H, ArH) 2) 2.62 (m, 1H, CH (C5)); 4.26 (m, 2H, OCH₂CH₃); 7.32, 7,45 (je dd, 1H, ArH) 3) 4.27 (q, 2H, OCH₂); 4.80 (m, 1H, CH (C5); 7.47 (dd, 2H, ArH) 4) 2.62 (m, 1H, CH (C5); 4.26 (m, 2H, OCH₂); 7.47 (d, 2H, ArH) 5) 1.08 (m, 3H, Ar-CH₂-CH₃); 4.76 (m, 1H, OCH(C5); 5.2 (d, 2H, O-CH₂) 6) 2.33 (s, 3H, Ar CH₃); 3.38 (s, 3H, OCH₃); 4.08 (q, 2 H, OCH₂) 7) 2.67 (m, 2H, Ar-CH₂CH₃); 3.37 (s, 3H, OCH₃); 3.69 (s, 3H CO₂CH₃) *¹H-NMR (400 MHz, CDCl₃): Verschiebung δ in ppm **¹H-NMR (300 MHz, CDCl₃): Verschiebung δ in ppm: | | | | | | | | | | | |

### Beispiel I-d-1

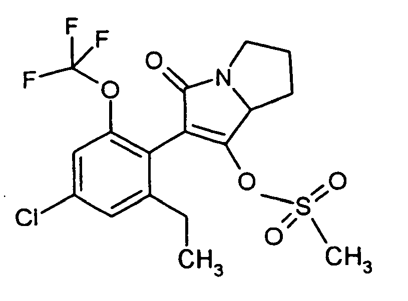

0.15 g (0.41 mmol) der Verbindung gemäß Beispiel (I-a-73), 0.05 g Trithylamin und 5 mg 4-N,N'-Dimethylamino-pyridin werden in 5 ml Chloroform vorgelegt. Nach 10 Min. Rühren werden 0.05 g (0.45 mmol) Methansulfonsäurechlorid zugegeben und über Nacht bei Raumtemperatur weitergerührt. Der Ansatz wird auf 5 ml 5%ige Natriumhydrogencarbonat-Lösung gegeben, 10 Min. bei Raumtemperatur gerührt, die organische Phase abgetrennt, über Natriumsulfat getrochnet und einrotiert. Es erfolgt eine chromatographische Reinigung an Kieselgel auf einer Biotage-Trennanlage mit einem Gradienten (Essigsäureetylester:n-Heptan 1:4 nach 4:1). Ausbeute: 0.052 g (28 % d. Theorie)

¹H-NMR (300 MHz, CDCl₃): δ = 3.04 (s, 3H, SOCH₃), 4.64 (m, 1H, CH (C5), 7.16, 7.22 (je dd, 1H, Ar-H) ppm.

### Beispiel I-f-1

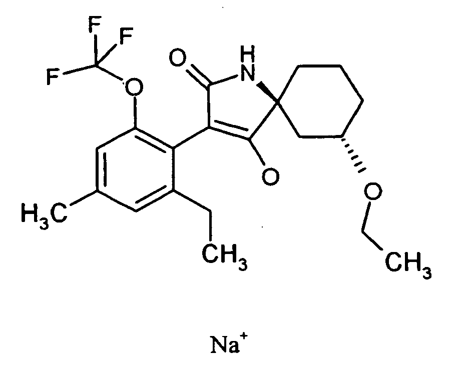

0.15 g (0.38 mmol) der Verbindung gemäß Beispiel (I-a-50) werden in 6 ml Methanol vorgegeben und 0.07 ml einer 30%igen Natriummethylatlösung hinzugegeben. Nach 2 h Rühren bei Raumtemperatur wird eingeengt und der Rückstand im Hochvakuum getrocknet. Man erhält 0.155 g (= 97 % der Theorie) eines Feststoffs.

¹H-NMR (400 MHz, D₂O): δ = 3.67 (m, 3H, O-CH und O-CH₂), 7.05, 7.13 (je d, 1H, ArH) ppm.

In Analogie zu Beispiel (I-f-1) und gemäß den allgemeine Angaben zur Herstellung erhält man folgende Verbindungen der Formel (1-f)

| | | | | | | | | | |
|---|---|---|---|---|---|---|---|---|---|
| 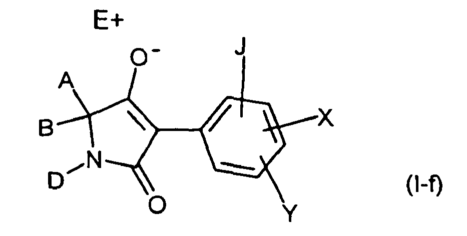 | | | | | | | | | |

| Bsp.- Nr. | J | X | Y | D | A | B | E | NMR | Isomer |
|---|---|---|---|---|---|---|---|---|---|
| I-f-2 | 2-OCF₃ | 6-C₂H₅ | 4-Cl | H | -(CH₂)₂-CHOCH₃-(CH₂)₂- | | Na⁺ | * 1) | β |
| I-f-3 | 2-OCF₃ | 4-CH₃ | 6-CH₃ | H | -(CH₂)₂-CHOCH₃-(CH₂)₂- | | Na⁺ | * 2) | β |

| | | | | | | | | | |
|---|---|---|---|---|---|---|---|---|---|
| 1) 3.23 (s, 3H, OCH₃); 6.98, 7.11 (je d, 1H, ArH) 2) 3.16 (s, 3H, OCH₃), 6.72, 7.85 (je d, 1H, Ar-H) * ¹H-NMR (400 MHz, d₆-DMSO): Verschiebung δ in ppm | | | | | | | | | |

### Beispiel II-1

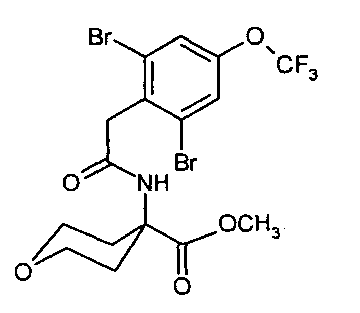

Es werden unter Argon 4,3 g (22 mmol) 1-Amino-tetrahydropyranyl-carbonsäuremethylester x HCl in 40 ml wasserfreien Tetrahydrofuran vorgelegt und 6,2 ml (44 mmol) Triethylamin zugegeben. Man rührt 5 min und gibt 7,6 g 2,6-Dibrom-4-trifluormethoxy-phenylessigsäure zu, rührt nochmals 15 min bei Raumtemperatur, gibt 4,4 ml Triethylamin zu und tropft sofort 1,2 ml Phosphoroxychlorid so zu, dass die Lösung mäßig siedet. Man rührt 30 min unter Rückfluss. Die Reaktionsmischung wird i. Vak. eingedampft und der Rückstand säulenchromatographisch an Kieselgel gereinigt (Dichlormethan/Essigsäureethylester = 3:1)

Ausbeute: 8,5 g (79 % d.Theorie), Fp.: 212°C

### Beispiel II-19

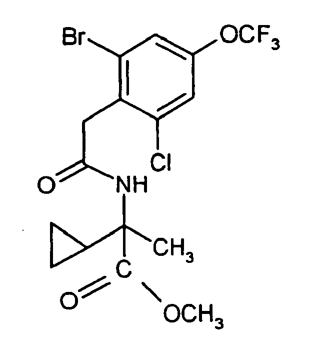

Zu 2,513 ml Schwefelsäure gibt man 3,46 g (8,13 mmol) der Verbindung gemäß Beispiel XIX-1 in 10 ml Dichlormethan. Man rührt 3 h bei 35°C und gibt 20 ml Methanol hinzu. Man rührt 4 h bei 60° und rührt dann bei Raumtemperatur über Nacht. Man gibt weitere 2 ml Schwefelsäure hinzu, da das Edukt noch nicht abreagiert ist. Man rührt 4 h bei 60°C. Nach dünnschichtchromatographischer Kontrolle gibt man die Reaktionslösung auf 100 ml Wasser trennt die organische Phase ab und trocknet über Natriumsulfat. Dann wird das Lösungsmittel abrotiert.

Ausbeute: 3,31 g (88 % d.Theorie)

In Analogie zu den Beispielen (II-1) und (II-19) und gemäß den allgemeinen Angaben zur Herstellung erhält man folgende Verbindungen der Formel (II):

| | | | | | | | | | |
|---|---|---|---|---|---|---|---|---|---|
| 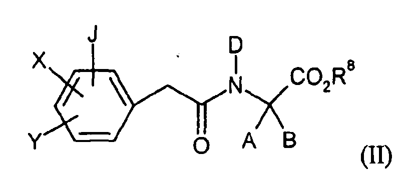 | | | | | | | | | |

| Bsp.-Nr. | J | X | Y | D | A | B | R⁸ | Fp.°C | Isomer |
|---|---|---|---|---|---|---|---|---|---|
| II-2 | 2-OCF₃ | H | H | H | -(CH₂)₂-CHOCH₃-(CH₂)₂- | | CH₃ | 105 | β |
| II-3 | 5-OCF₃ | 2-Br | H | H | -(CH₂)₂-CHOCH₃-(CH₂)₂- | | CH₃ | Öl | β |
| II-4 | 5-OCF₃ | 2-Br | H | H | -(CH₂)₂-O-(CH₂)₂- | | CH₃ | 125 | - |
| II-5 | 5-OCF₃ | 2-Br | H | H | 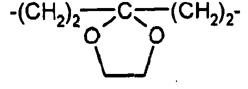 | | CH₃ | 138 | - |
| II-6 | 5-OCF₃ | 2-Br | H | H | -(CH₂)₂-CHOC₂H₅-(CH₂)₂- | | CH₃ | 100 | β |
| II-7 | 4-OCF₃ | 2-Br | 6-Br | H | -CH₂-CHOCH₃-(CH₂)₃- | | CH₃ | 102 | β |
| II-8 | 5-OCF₃ | 2-Br | H | H | 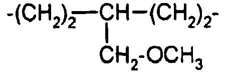 | | CH₃ | Wachs | β |
| II-9 | 4-OCF₃ | 2-Br | 6-Br | H | -(CH₂)₂-CHOC₂H₅-(CH₂)₂- | | CH₃ | 186 | β |
| II-10 | 4-OCF₃ | 2-Br | 6-Cl | H | CH₃ | CH₃ | CH₃ | 127-129 | - |
| II-11 | 4-OCF₃ | 2-Br | 6-Cl | H | -CH₂-CHOC₄H₉-(CH₂)₃- | | | CH₃ 3.97 (d, 2H, Ar-CH₂), 7.32 u. 7.44 (je s, 1H, Ar-H) | trans |
| II-12 | 4-OCF₃ | 2-Br | 6-Cl | -(CH₂)₃- | | H | CH₃ | *4.02 (d, 2H, Ar-CH₂)7.26 u. 7.38 (je 9, 1H, Ar-H), 4.53 (m, 1H, C(2)-H) | - |
| II-13 | 4-OCF₃ | 2-Br | 6-Cl | 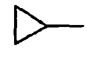 | CH₃ | H | CH₃ | *1.51 (d, 3H, CH₃) 4.24 (s, 2H, Ar-CH₂) 7.26 u. 7.38 (je s, 1H, Ar-H) | - |
| II-14 | 4-OCF₃ | 2-Br | 6-Br | H | -CH₂-CHOC₄H₉-CH₂)₃- | | CH₃ | *0.91 (t, 3H, CH₃) 4.03 (s, 2H, Ar-CH₂) 7.51 (s, 2H, Ar-H) | ß |
| II-15 | 4-OCF₃ | 2-Br | 6-Br | -(CH₂)₃- | | H | CH₃ | *4.53 (m, 1H, C(2)-H, 4.09 (d, 2H, Ar-CH₂) 7.44 (s, 2H, Ar-H) | - |
| II-16 | 4-OCF₃ | 2-Br | 6-Br | 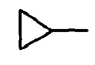 | CH₃ | H | CH₃ | *1.51 (d 3H, CH₃) 4.31 (s, 2H, Ar-CH₂) 7.44 (s, 2H, Ar-H) | - |
| II-17 | 4-OCF₃ | 2-Br | 6-Br | H | 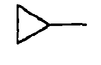 | CH₃ | CH₃ | **1.29 (m, 1H, CH-Cyclopropyl), 3.9R (d, 2H, Ar-CH₂) 7.47 (s, 2H, Ar-H) | - |
| II-18 | 4-OCF₃ | 2-Br | 6-Br | H | C₂H₅ | CH₃ | CH₃ | **1.60 (s, 3H, CH₃-) 4.01 (d, 2H, Ar-CH₂) 7.47 (s, 2H, Ar-H) | - |
| II-19 | 4-OCF₃ | 2-Br | 6-Cl | H | 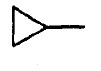 | CH₃ | CH₃ | **1.46 (s, 3H, CH₃)-, 3.94 (d, 2H, Ar-CH₂) 7.30 u. 7.43 (je s, 1H, Ar-H) | *- |
| II-20 | 4-OCF₃ | 2-Br | 6-Cl | H | C₂H₅ | CH₃ | CH₃ | **1.59(s,3H, CH₃) 3.97(d, 2H. Ar·CH₂) 7.30 u. 7.43 (je s,1H, Ar-H) | - |
| II-21 | 4-OCF₃ | 2-OCH₃ | 6-Br | H | CH₃ | CH₃ | CH₃ | 110°C | - |
| II-22 | 4-OCF₃ | 2-OCH₃ | 6-Br | H | -(CH₂)₂-CHOCH₃-(CH₂)₂- | | CH₃ | **3.33 (s, 3H, CH-OCH₃) 6.72 u. 7.11 (je s, 1H, Ar-H) | β |
| II-23 | 5-OCF₃ | 2-Br | H | H | 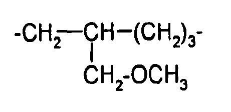 | | CH₃ | 117 | β |
| II-24 | 4-OCF₃ | 2-OCH₃ | 6-Cl | H | -CH₂-CHOC₄H₉-(CH₂)₃- | | CH₃ | **0.93 (1, 3H, CH₃) 3.91 (d, 2H, Ar-CH₂) 6.71 u. 6.98 (je s, 1H, Ar-H) | β |
| II-25 | 4-OCF₃ | 2-OCH₃ | 6-Cl | -(CH₂)₃- | | H | CH₃ | **3.68 (s, 3H, Ar-OCH₃) 4.53 (m, 1H, C(2)-H) 6.63 u. 6.91 (je s, 1H, Ar-H) | - |
| II-26 | 4-OCF₃ | 2-OCH₃ | 6-Cl | H | -(CH₂)₂-CHOCH₃-(CH₂)₂- | | CH₃ | **3.33 (s, 3H, CH-OCH₃) 6.84 u. 6.96 (s, 1H, Ar-H) | β |
| II-27 | 4-OCF₃ | 2-OCH₃ | 6-Cl | H | -(CH₂)₂-O-(CH₂)₂- | | CH₃ | 170 | - |
| II-28 | 4-OCF₃ | 2-Cl | 6-Cl | H | -(CH₂)₂-CHOCH₃-(CH₂)₂- | | CH₃ | 168 | β |
| II-29 | 4-OCF₃ | 2-Cl | 6-Cl | H | -(CH₂)₂-O-(CH₂)₂- | | CH₃ | 182 | - |
| II-30 | 4-OCF₃ | 2-Br | 6-Br | H | CH₃ | CH₃ | CH₃ | 170 | - |
| II-31 | 4-OCF₃ | 2-Br | 6-Br | H | -(CH₂)₂-CHOCH₃-(CH₂)₂- | | CH₃ | 180 | β |
| II-32 | 4-OCF₃ | 2-Br | 6-Br | H | -CH₂-CHOCH₃-(CH₂)₃- | | CH₃ | Öl | α |
| II-34 | 4-OCF₃ | 2-OCH₃ | 6-OCH₃ | H | -(CH₂)₂-CHOCH₃-(CH₂)₂- | | CH₃ | 137-139 | β |
| II-35 | 4-OCF₃ | 2-CH₃ | 6-CH₃ | H | -(CH₂)₂-CHOCH₃-(CH₂)₂- | | CH₃ | 164-168 | β |
| II-36 | 4-OCF₃ | 2-CH₃ | 6-CH₃ | H | -CH₂-CHOC₂H₅-(CH₂)₃- | | CH₃ | 141-144 | β |
| II-37 | 4-OCF₃ | 2-Cl | 6-CH₃ | H | -CH₂-CHOCH₃-(CH₂)₂- | | C₂H₅ | ** 1) | Gemisch ca. 1:1 |
| II-38 | 4-OCF3 | 2-Cl | 6-CH3 | H | -(CH₂)₂-CHOCH₃-(CH₂)₂- | | CH₃ | 164 | β |
| II-39 | 4-OCF₃ | 2-Br | 6-Br | H | 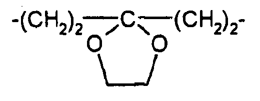 | | CH₃ | 180 | - |
| II-40 | 4-OCF₃ | 2-OCH₃ | 6-Cl | H | 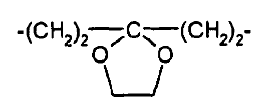 | | CH₃ | 143 | - |
| 11-41 | 4-OCF₃ | 2-Cl | 6-Cl | H | 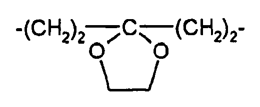 | | CH₃ | 160 | - |
| II-42 | 4-OCF₃ | 2-Br | 6-Br | H | 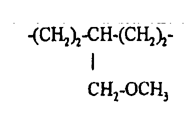 | | CH₃ | 173 | β |
| II-43 | 2-OCF₃ | 4-Br | H | H | -(CH₂)₂-CHOCH₃-(CH₂)₂- | | CH₃ | 134 | β |
| II-44 | 2-OCF₃ | 4-Cl | H | H | -(CH₂)₂-O-(CH₂)₂- | | CH₃ | 132 | - |
| II-45 | 2-OCF₃ | 6-Cl | 4-Br | H | -(CH₂)₂-O-(CH₂)₂- | | CH₃ | 144 | - |
| II-46 | 2-OCF₃ | 4-Cl | H | H | -(CH₂)₂-CHOCH₃-(CH₂)₂- | | CH₃ | 133 | β |
| II-47 | 2-OCF₃ | 6-CH₃ | 4-CH₃ | H | -(CH₂)2-CHOCH₃-(CH₂)₂- | | CH₃ | 138 | β |
| II-48 | 2-OCF₃ | 6-CH₃ | 4-CH₃ | H | -CH₂-CHOC₂H5-(CH₂)₃- | | CH₃ | **2) | β |
| II-49 | 2-OCF₃ | 4-Br | H | H | -(CH₂)₂-O-(CH₂)₂- | | CH₃ | 142 | - |
| II-50 | 2-OCF₃ | 6-OCH₃ | 4-Br | H | -(CH₂)₂-O-(CH₂)₂- | | CH₃ | 182 | - |
| II-51 | 2-OCF₃ | 6-Br | 4-Cl | H | -(CH₂)₂-O-(CH₂)₂- | | CH₃ | 160 | - |
| II-52 | 2-OCF₃ | 6-OCH₃ | 4-Cl | H | -(CH₂)₂-O-(CH₂)₂- | | CH₃ | 181 | - |
| II-53 | 2-OCF₃ | 6-Cl | 4-Br | H | -(CH₂)₂-CHOCH₃-(CH₂)₂- | | CH₃ | 131 | β |
| II-54 | 2-OCF₃ | 6-Cl | 4-Br | H | -CH₂-CHOC₂H₅-(CH₂)₃- | | CH₃ | 128 | β |
| II-55 | 2-OCF₃ | 6-Br | 4-Cl | H | -(CH₂)₂-CHOCH₃-(CH₂)₂- | | CH₃ | 144 | β |
| II-56 | 2-OCF₃ | 6-Br | 4-Br | H | -(CH₂)₂-CHOCH₃-(CH₂)₂- | | CH₃ | 138 | β |
| II-57 | 2-OCF₃ | 6-Cl | 4-Br | H | 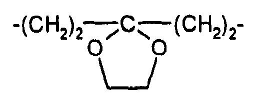 | | CH₃ | 155 | - |
| II-58 | 2-OCF₃ | 6-Cl | 4-Br | H | CH₃ | CH₃ | CH₃ | 129 | - |
| II-59 | 2-OCF₃ | 6-OCH₃ | 4-Cl | H | -(CH₂)₂-CHOCH₃-(CH₂)₂- | | CH₃ | ** 3 | β |
| II-60 | 2-OCF₃ | 6-Cl | 4-CH₃ | H | -(CH₂)₂-CHOCH₃-(CH₂)₂- | | CH₃ | 150-151 | β |
| II-61 | 2-OCF₃ | 6-Cl | 4-CH₃ | H | -CH₂-CHOC₂H₅-(CH₂)₃- | | CH₃ | 120-123 | β |
| II-62 | 2-OCF₃ | 6-Cl | 4-CH₃ | H | -CH₂-CHOC₃H₇-(CH₂)₃- | | CH₃ | *** 4 | β |
| II-63 | 2-OCF₃ | 6-CH₃ | 4-Cl | H | -CH₂-CHOC₂H₅-(CH₂)₃- | | CH₃ | *** 5 | β |
| II-64 | 2-OCF₃ | 6-OCH₃ | 4-Cl | H | -CH₂-CHOC₂H₅-(CH₂)₃- | | CH₃ | ** 6 | β |
| II-65 | 2-OCF₃ | 6-C₂H₅ | 4-Cl | H | -(CH₂)₂-CHOCH₃-(CH₂)₂- | | CH₃ | ** 7 | β |
| II-66 | 2-OCF₃ | 6-C₂H₃ | 4-Cl | H | 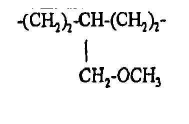 | | CH₃ | ** 8 | β |

| | | | | | | | | | |
|---|---|---|---|---|---|---|---|---|---|
| 1) 3.11,3.29 (je s, zusammen 3H, OCH₃); 4.16 (m, 2H, OCH₂CH₃) 2) 3.08 (m, 1H, OCH); 3.63 (s, 2H, CH₂); 3.68 (s, 3H, OCH₃) 3) 3.19 (m, 1H, OCH); 3.68 (s, 3H, OCH₃); 3.90 (s, 3H, Ar OCH₃) 4) 3.21 (m, 1H, OCH); 3.68 (s, 3H, OCH₃); 3.76 (s, 2H, CH₂) 5) 3.11 (m, 1H, OCH); 3.62 (s, 3H, OCH);3.76 (s, 2H, CH₂) 6) 3.15(m, 1H, OCH); 3.60 (s, 2H, CH₂); 3.67 (s, 3H, OCH₃) 7) 3.15 (m, 1H, OCH); 3.63 (s, 2H, CH₂); 3.66 (s, 3H, OCH₃) 8) 3.31 (s, 3H, CH₂-OCH₃- 3.63 (s, 2H, CH₂); 3.66 (s, 3H, OCH₃) ^{*1}H-NMR (500 MHz, CDCl₃): Verschiebungen δ in ppm ^{**1}H-NMR (400 MHz, CDCl₃): Verschiebungen δ in ppm ^{***1}H-NMR (300 MHz, CDCl₃): Verschiebungen δ in ppm | | | | | | | | | |

### Beispiel XIX-1

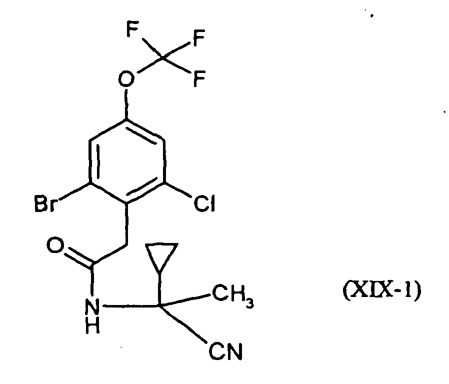

3 g (9 mmol) der Verbindung gemäß Bsp. XVII-2 vorlegen, 1 Tropfen DMF zugeben, 3,21 g (27 mmol) Thionylchlorid zugeben. Bis Ende Gasentwicklung unter Rückfluss rühren, SOCl₂ abrotieren, Rückstand in 3 ml Dichlormethan aufnehmen (= Lösung 1).

2,62 ml Triethylamin in 10 ml Dichlormethan vorlegen, bei 0°C Lösung 1 langsam zutropfen. Über Nacht bei Raumtemperatur rühren bis Edukt abreagiert ist, 10 ml Wasser zugeben, 10 min bei Raumtemperatur rühren, extrahieren, organische Phase über Natriumsulfat trocknen und einrotieren.

Ohne Aufreinigung weiter umsetzen.

Ausbeute: 3,46 g (90 % d. Theorie)

1.79 (s 3H, CH₃)

3.98 (s, 2H, Ar-CH₂)

7.32 u. 7.44 (je s, 1H, Ar-H)

*¹-NMR (500 MHz, CDCl₃) Verschiebung δ in ppm.

In Analogie zu Beispiel (XLX-1) und gemäß den allgemeinen Angaben zur Herstellung erhält man folgende Verbindungen der Formel (XIX):

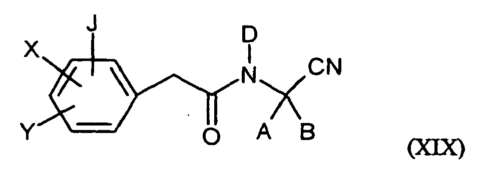

| Bsp.- Nr. | J | X | Y | A | B | Fp°C |
|---|---|---|---|---|---|---|
| XIX-2 | 4-OCF₃ | 2-Br | 6-Cl | C₂H₅ | CH₃ | *1.67 (s 3H, CH₃) 3.97 (s, 2H, Ar-CH₂) 7.31 u. 7.43 (je s, 1H, Ar-H) |
| XEX-3 | 4-OCF₃ | 2-Br | 6-Br | 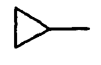 | CH₃ | *1.80 (s 3H, CH₃) 4.02 (s, 2H, Ar-CH₂) 7.49 (s, 2H, Ar-H) |
| XIX-4 | 4-OCF₃ | 2-Br | 6-Br | C₂H₅ | CH₃ | 130-132 |

| | | | | | | |
|---|---|---|---|---|---|---|
| mitD=H ^{*1}H-NMR (500 MHz, CDCl₃) Verschiebung δ in ppm. | | | | | | |

### Beispiel XVII-1

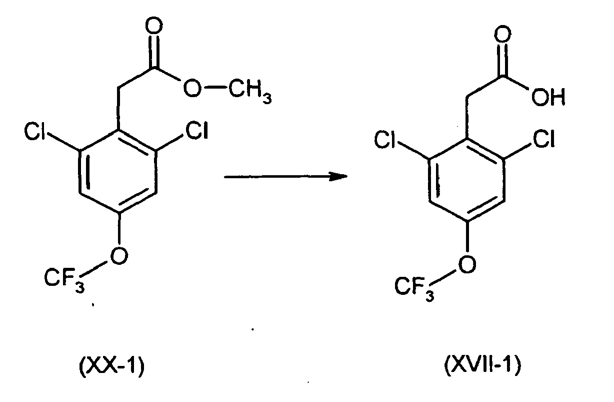

Es werden 465 g (1,53 mol) der Verbindung gemäß Bsp.-Nr. XX-1 in 10 %iger NaOH (1795,87 g = 4,49 mmol) bei Raumtemperatur vorgelegt und auf 40°C erhitzt. Es wird bei 40°C gerührt (dünnschichtchromatographische Kontrolle). Nach Reaktionsende gibt man 250 ml Dichlormethan zu, die organische Phase wird abgetrennt, die wässrige Phase mit konz. HCl sauer gestellt und der Niederschlag wird abgesaugt.

Ausbeute: 278 g (59 % d. Theorie)

In Analogie zu Beispiel (XVII-1) und gemäß den allgemeinen Angaben zur Herstellung erhält man folgende Verbindungen der Formel (XVII):

| | | | | |
|---|---|---|---|---|
| 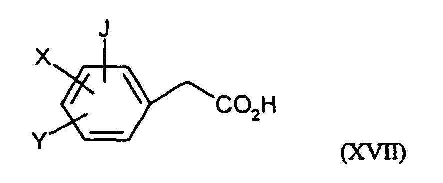 | | | | |

| Bsp.-Nr. | J | X | Y | Fp°C |
|---|---|---|---|---|
| XVII-2 | 4-OCF₃ | 2-Br | 6-Cl | 144-145 |
| XVII-3 | 4-OCF₃ | 2-Br | 6-Br | 165-166 |
| XVII-4 | 4-OCF₃ | 2-OCH₃ | 6-Br | 149-150 |
| XVII-5 | 4-OCF₃ | 2-OCH₃ | 6-Cl | 135-137 |
| XVII-6 | 4-OCF₃ | 2-Cl | 6-Cl | 124 |
| XVII-7 | 4-OCF₃ | 2-OCH₃ | 6-Cl | 136-137 |
| XVII-8 | 4-OCF₃ | 2-OCH₃ | 6-Br | 148-149 |
| XVII-9 | 4-OCF₃ | 2-CH₃ | 6-CH₃ | 144-147 |
| XVII-10 | 4-OCF₃ | 2-Cl | 6-CH₃ | 116-120 |
| XVII-11 | 2-OCF₃ | 2-Cl | 4-Br | 130 |
| XVII-12 | 2-OCF₃ | 6-CH₃ | 4-CH₃ | 140 |
| XVII-13 | 2-OCF₃ | 6-OCH₃ | 4-Cl | 138-140 |
| XVII-14 | 2-OCF₃ | 6-Cl | 4-CH₃ | 121-124 |
| XVII-15 | 2-OCF₃ | 6-C₂H₅ | 4-Cl | 107-108 |

### Beispiel XX-1

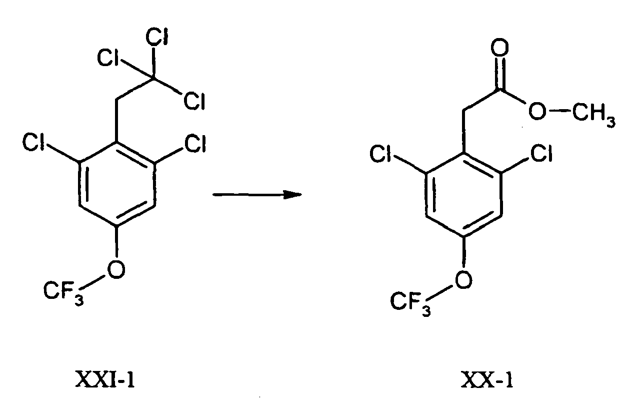

423,98 g Kaliumhydroxid in 2000 ml Methanol vorlegen, auf 50°C erhitzen, Verbindung gemäß Bsp. XXI-1 in 1474 ml Methanol lösen, zutropfen. Über Nacht bei ca. 55°C rühren, dann abkühlen und mit konz. Schwefelsäure auf pH 3 stellen, 1 Stunde bei Rückfluss rühren.

Lösungsmittel abdestillieren und Niederschlag in 500 ml Dichlormethan und 500 ml Wasser aufnehmen. Die organische Phase wird abgetrennt und das Lösungsmittel abdestilliert.

Ausbeute: 465 g (52 % d. Theorie)

Die Verbindung XX-1 wird ohne weitere Aufreinigung zur Herstellung von Verbindung XVII-1 weiter eingesetzt.

### Beispiel XX-5

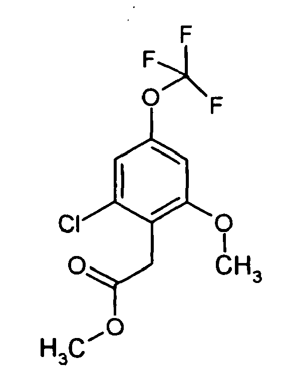

Ein Reaktionsgemisch bestehend aus 1 g (2.88 mmol) der Verbindung gemäß Beispiel (XX-2), 1.56 g (8.6 mmol) 30%ige Natriummethyladösung, 0.083 g (0.57 mmol) Kupfer-I-bromid und 0.78 g (10.5 mmol) Essigsäuremethylester werden bei 120°C Badtemperatur ca. 7 h gerührt. Man nimmt in Wasser auf, filtriert und stellt das Filtrat mit 1N HCl auf pH1 ein, extrahiert mit Essigsäureethylester (EE), trocknet über Natriumsulfat und rotiert ein. Die Reinigung erfolgt durch Säulenchromatographie am Kieselgel mit EE/n-Heptan 1:1

Ausbeute: 0.6 g (= 69 % der Theorie)

Spektroskopische Daten siehe Beispieltabelle der Formel (XX).

### Beispiel XX-13

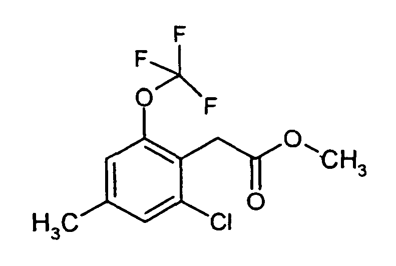

2 g (5.7 mmol) der Verbindung gemäß Beispiel (XX-14) werden in 40 ml Dioxan und 4 ml Wasser vorgelegt und 1.11 g (8 mmol) Kaliumcarbonat, 1.98 g (1.7 mmol Tetrakistriphenylphosphinpalladium und 1.08 g (8.6 mmol) Trimethylboroxin werden zugegeben. Man rührt 4 Stunden unter Rückfluss, engt das Reaktionsgemisch ein, nimmt mit 50 ml 1N HCl auf, extrahiert zweimal mit 20 ml Essigsäureethylester (EE), trocknet die organische Phase über Natriumsulfat und rotiert ein. Der Rückstand wird an Kieselgel mit EE/n-Heptan 1:9 gereinigt.

Ausbeute: 1 g (= 61 % der Theorie)

Spektroskopische Daten siehe Beispieltabelle der Formel (XX)

In Analogie zu den Beispielen (XX-1), (XX-5) und (XX-13) lassen sich folgende Verbindungen der Formel (XX) herstellen:

| | | | | |
|---|---|---|---|---|
| 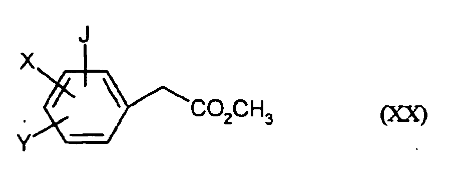 | | | | |

| Bsp.-Nr. | J | X | Y | ¹H-NMR Daten |
|---|---|---|---|---|
| XX-2 | 4-OCF₃ | 2-Br | 6-Cl | direkte Umsetzung zu XVII-2 |
| XX-3 | 4-OCF₃ | 2-Br | 6-Br | direkte Umsetzung zu XVII-3 |
| XX-4 | 4-OCF₃ | 2-OCH₃ | 6-Br | *3.71 (s, 3H, OCH₃) 3.81 (s 3H, CO₂CH₃) 3.84 (s, 2H, Ar-CH₂) 6.68, 7.09 (2s, 2H-Ar-H) |
| XX-5 | 4-OCF₃ | 2-OCH₃ | 6-Cl | *3.68 (s, 3H, OCH₃) 6.63, 6.91 (2s 2H, Ar-H) |
| XX-6 | 4-OCF₃ | 2-CH₃ | 6-CH₃ | Fp.47°C |
| XX-7 | 4-OCF₃ | 2-Br | 6-Cl | **3.74 (s, 3H, OCH₃) 4.05 (s, 2H, CH₂) 7.28, 7.41 (je s, 1H, ArH) |
| XX-8 | 4-OCF₃ | 2-Cl | 6-CH₃ | **3.71 (s, 3H, OCH₃) 3.83 (s, 2H, CH₂) 6.99, 7.14 (je s, 1H, ArH) |
| XX-9 | 2-OCF₃ | 6-Br | 4-Br | **3.72 (s, 3H, OCH₃) 3.87 (s, 2H, CH₂) 7.41, 7.71 (je s, 1H, ArH) |
| XX-10 | 2-OCF₃ | 6-CH₃ | 4-CH₃ | **3.68 (s, 3H, OCH₃) 3.70 (s, 2H, CH₂) 6.92, 6.96 (je s, 1H, ArH) |
| XX-11 | 2-OCF₃ | 6-OCH₃ | 4-Cl | ** 3.67 (s, 2H, CH₃) 3.70 (s, 3H, OCH₃) 3.85 (s, 3H, ArOCH₃) 6.84, 6.95 (je s, 1H, ArH) |
| XX-12 | 2-OCF₃ | 6-CH₃ | 4-Cl | *** 3.71 (pseudo s, 5H, CH₂ und OCH₃) 7.16 (s, 2H, ArH) |
| XX-13 | 2-OCF₃ | 6-Cl | 4-CH₃ | *** 3.71 (s, 3H, OCH₃) 3.84 (s, 2H, CH₂) |
| | | | | 7.03, 7.21 (je s, 1H, ArH) |
| XX-14 | 2-OCF₃ | 6-Cl | 4-Br | *3.71 (s, 3H, OCH₃) 3.83 (s, 3H, CH₂) 7.37, 7.54 (je 2d, 1H, ArH) |

| | | | | |
|---|---|---|---|---|
| *¹H-NMR (500 MHz, CDCl₃): Verschiebungen δ in ppm ** ¹H-NMR (300 MHz, CDCl₃): Verschiebungen δ in ppm *** ¹H-NMR (400 MHz, CDCl₃): Verschiebungen δ in ppm | | | | |

### Beispiel XXI-1

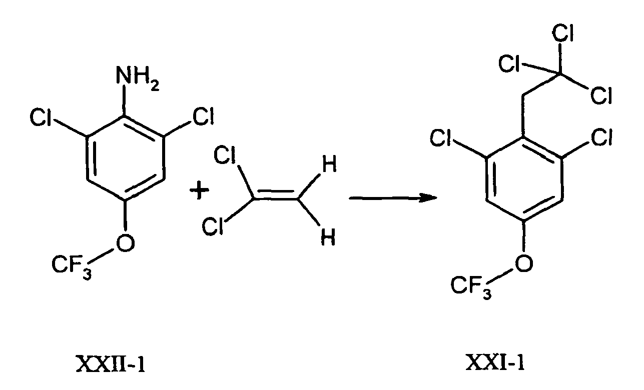

tert.-Butylnitrit (229,9 g = 2,23 mol) und Kupfer(II)chlorid (233,9 g = 1,74 mol) bei Raumtemperatur in 1500 ml Acetonitril vorlegen. Vinylidenchlorid (733,6 g = 7,57 mmol) in 40 min zulaufen lassen, dann 2,6-Dichlor-4-trifluormethoxy-anilin (400 g = 1,3 mmol) in 1350 ml Acetonitril in 5 h bei ca.40°C zutropfen (leicht exotherm, sofortige Gasentwicklung) nach Ende der Gasentwicklung Temperatur auf 60°C steigern und ca. 18 Stunden rühren.

Das Reaktionsgemisch gibt man auf 4 Liter 1N HCl-Lösung, trennt die organische Phase ab, extrahiert die wässrige Phase mit 300 ml Methyl-tert.-butylether und wäscht die organischen Phasen mit 2 Liter 1N HCI-Lösung und trennt die organische Phase ab. Das Lösungsmittel wird abdestilliert.

Ausbeute: 573 g (53 % d. Theorie)

In Analogie zu Beispiel (XXI-1) lassen sich die folgenden Verbindungen der Formel (XXI) herstellen:

| | | | |
|---|---|---|---|
| 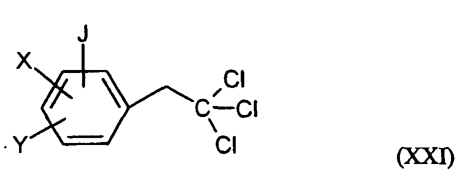 | | | |

| Bsp.-Nr. | J | X | Y |
|---|---|---|---|
| XXI-2 | 4-OCF₃ | 2-Br | 6-Cl |
| XXI-3 | 4-OCF₃ | 2-Br | 6-Br |

Die Verbindungen der Formel XXI werden ohne weitere Aufreinigung zur Herstellung der Verbindungen der Formel XX verwendet.

### Beispiel Nr. 1

**Myzus-Test (MYZUPE Spritzbehandlung)**
- Lösungsmittel:: 78 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele eine Wirkung von ≥ 80 % bei einer Aufwandmenge von 100 g/ha:

Bsp. Nr. I-c-5

Bei diesem Test zeigen z.B. die folgenden Verbindungen der Herstellungsbeispiele eine Wirkung von ≥ 80 % bei einer Aufwandmenge von 500 g/ha:

Bsp. Nr. I-a-1, I-a-2, I-a-4, I-a-5, I-a-6, I-a-7, I-a-8, I-a-9, I-a-11- I-a-14, I-a-22, I-a-26, I-a-28, I-a-29, I-a-30, I-a-33, I-a-34, I-a-35, I-a-37- I-a-38, I-a-39, I-a-40, I-a-42, I-a-43, I-a-44, 1-a-45, I-a-46, I-a-48, I-a-49- I-a-50, I-a-52, I-a-53, I-a-54, I-a-55, I-a-57, I-a-58, I-a-59, I-a-60, I-a-62- I-a-63, 1-a-66, I-a-67- I-a-70, I-b-2, I-b-3, 1-b-10, I-b-13, I-b-17, I-b-20, I-b-21, I-b-22, I-b-23, I-b-24, I-b-25, I-b-26, I-c-1, I-c-2, 1-c-3, I-c-4, I-c-16, I-c-18, I-c-20, I-c-22, I-c-24, I-c-25, I-c-26, I-c-27, I-c-30, I-c-31, I-c-33, I-c-36

### Beispiel Nr. 2

**Tetranychus-Test OP-resistent (TETRUR Spritzbehandlung)**
- Lösungsmittel:: 78 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnnülbe (*Tetranychus utricae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 20 g/ha:

Bsp. Nr. I-a-38, I-b-22, I-c-26

Bei diesem Test zeigen z. B. die folgenden Verbindungen der Herstellungsbeispiele eine Wirkung von ≥ 80 % bei einer Aufwandmenge von 100 g/ha:

Bsp. Nr. I-a-1; I-a-7, I-a-8, I-a-9, I-a-10, I-a-18, I-a-19, I-a-22 I-a-26- I-a-28, I-a-29, I-a-31, I-a-33, I-a-35, I-a-37, I-a-39, I-a-40, I-a-41, I-a-43- I-a-44, I-a-47, I-a-48, I-a-49, I-a-51, I-a-52, I-a-53, I-a-54, I-a-55, I-a-56- I-a-57, I-a-60, I-a-61, I-a-62, I-a-63, I-a-66, I-a-67, I-a-70, I-b-2, I-b-3, I-b-6, I-b-7, I-b-8, I-b-12, I-b-13, I-b-17, I-b-20, I-b-21, I-b-23, I-b-24, I-b-25, I-b-26, I-c-10, I-c-11, I-c-12, I-c-13, I-c-14, I-c-15, I-c-16, I-c-17, I-c-20, I-c-24, I-c-27, I-c-31, I-c-36

### Beispiel Nr. 3

**Phaedon -Test (PHAECO Spritzbehandlung)**
- Lösungsmittel:: 78 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele eine Wirkung von ≥ 80 % bei einer Aufwandmenge von 500 g/ha:

Bsp. Nr. I-a-4, I-a-6, I-a-11, I-a-14, I-a-29, I-a-30, I-a-34, I-a-35, I-c-4, I-b-2, I-b-3, I-b-21, I-b-23, I-b-25, I-a-27, I-a-41, I-a-44, I-a-49, I-a-50, I-c-2, 1-c-13, I-c-25, I-c-27, I-c-29, I-c-33

### Beispiel Nr. 4

**Nilaparvata lugens -Test (NILALU hydroponische Behandlung)**
- Lösungsmittel:: 78 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Die Wirkstoffzubereitung wird in Wasser pipettiert. Die angegebene Konzentration bezieht sich auf die Wirkstoffmenge pro Volumeneinheit Wasser (mg/l = ppm), anschlie0end wird mit der Braunrückigen Reiszikade (*Nilaparvata lugens*) infiziert.

Nach der gewünschten Zeit wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z.B. die folgenden Verbindungen der Herstellungsbeispiele mit einer Konzentration von 500 ppm nach 7 d eine Wirksamkeit von ≥ 80 %: I-a-4.

### Beispiel Nr. 5

**Spodoptera frugiperda-Test ( SPODFR Spritzbehandlung)**
- Lösungsmittel:: 78 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele mit einer Aufwandmenge von 500 g a.i./ha nach 7 d eine Wirksamkeit von ≥ 80 %: I-a-6, I-a-34, I-b-2, I-b-3, I-c-2, I-c-4.

### Beispiel Nr. 6

**Meloidogyne-Test (MELGIN Spritzbehandlung)**
- Lösungsmittel:: 80 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm:

Bsp. Nr. I-a-49, I-a-50, I-b-25, I-c-22

### Beispiel Nr. 7

**Nephotettix-Test (NEPHCI)**
- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge (*Oryza sativa*) werden durch Tauchen in die Wirkstoffzubereitung der gewünschten Konzentration behandelt und mit der Grünen Reiszikade (*Nephotettix cincticeps*) besetzt, solange die Blätter noch feucht sind.

Nach der gewünschten Zeit wird die Abtötung in % bestimmt. Dabei bedeutet 100%, daß alle Zikaden abgetötet wurden; 0% bedeutet, daß keine Zikaden abgetötet wurden.

Bei diesem Test zeigen z. B. die folgenden Verbindungen der Herstellungsbeispiele eine Wirkung von ≥ 80 % bei einer Aufwandmenge von 100 ppm:

Bsp. Nr. I-c-2

### Beispiel Nr. 8

**Boophilus microplus -Test (BOOPMI Injektion)**
- Lösungsmittel:: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen (*Boophilus microplus*) injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier:

Bsp. Nr. I-a-1, I-a-3, I-a-9, I-a-28, I-a-29, I-a-38, I-a-39, I-b-20, I-b-21, I-c-20

### Beispiel Nr. 9

**Lucilia cuprina-Test (LUCICU)**
- Lösungsmittel:: Dimethylsulfoxid

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:

Bsp. Nr. I-a-29, I-a-38, I-b-21

### Beispiel 10

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 1/ha unter Zusatz von 0,2 % Netzmittel auf die Oberfläche der Abdeckerde appliziert.

Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 l/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

Folgende Verbindungen zeigen im Vorauflauf mit 320 g/ha a.i. gegen Lolium multiflorum und Setaria viridis eine Wirkung von ≥ 80 %: I-a-2, I-a-22, I-a-25, I-a-26, I-a-27, I-a-29, I-a-38, I-a-39, I-a-46, I-a-49, I-a-50, I-a-51, I-a-52, I-a-54, I-a-57, I-a-59, I-a-60, I-a-61, I-a-62, I-a-63, I-a-64, I-a-65, I-a-66, I-a-67, I-a-68, I-a-70, I-b-13, I-b-15, I-b-17, I-b-21, I-b-22, I-b-24, I-b-25, I-b-26, I-c-16, I-c-18, I-c-22, I-c-26, I-c-29, I-c-30, I-c-31, I-c-32, I-c-33, I-c-34, I-c-35, I-c-36, I-c-37.

Folgende Verbindungen zeigen im Nachauflauf mit 80 g/ha a.i. gegen Echinochloa crus-galli, Lolium multiflorum und Setaria viridis und eine Wirkung von ≥ 70 %: I-a-24, I-a-26, I-a-37, I-a-46, I-a-49, I-a-50, I-a-51, I-a-54, I-a-58, I-a-59, I-a-60, I-a-61, I-a-63, I-a-64, I-a-65, I-a-66, I-a-67, I-a-68, I-b-25, I-c-32, I-c-37.

### Herbizide Wirkung im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien außerhalb des Gewächshauses, unter guten Wachstumsbedingungen angezogen. 2 bis 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden dann in verschiedene Dosierungen mit einer Wasseraufwandmenge von umgerechnet 300 l/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit eine bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche mit Getreide im Gewächshaus

**Mefenpyr 1 Tag vor Herbizidapplikation**

| | | 10 Tage nach Applikation |
|---|---|---|
| | Aufwandmenge | Sommerweizen |
| | g a.i./ha | beobachtet (%) |
| | | |
| (I-a-49) | 100 | 40 |
| | 50 | 20 |
| | 25 | 10 |
| | | |
| | | |
| (I-a-49) | 100 + 50 | 0 |
| + Mefenpyr | 50 + 50 | 0 |
| | 25+50 | 0 |
| | | |

| | | 28 Tage nach Applikation | |
|---|---|---|---|
| | Aufwandmenge | Sommergerste | Sommerweizen |
| | g a.i./ha | beobachtet (%) | beobachtet (%) |
| | | | |
| (I-a-50) | 100 | 60 | 70 |
| | 50 | 50 | 60 |
| | 25 | 30 | 40 |
| | 12,5 | 8 | 10 |
| | | | |
| (I-a-50) | 100 + 50 | 30 | 20 |
| + Mefenpyr | 50 + 50 | 10 | 5 |
| | 25 + 50 | 5 | 0 |
| | 12,5 + 50 | 0 | 0 |

| | | 10 Tage nach Applikation | |
|---|---|---|---|
| | Aufwandmenge | Sommergerste | Sommerweizen |
| | g a.i./ha | beobachtet (%) | beobachtet (%) |
| | | | |
| (I-b-24) | 100 | 30 | 40 |
| | 50 | 30 | 40 |
| | 25 | 20 | 40 |
| | 12,5 | 10 | 30 |
| | | | |
| (I-b-24) | 100 + 50 | 10 | 20 |
| + Mefenpyr | 50 + 50 | 8 | 10 |
| | 25 + 50 | 5 | 5 |
| | 12,5 + 50 | 5 | 0 |

| | | 28 Tage nach Applikation |
|---|---|---|
| | Aufwandmenge | Sommerweizen |
| | g a,i./ha | beobachtet (%) |
| | | |
| (I-b-24) | 100 | 20 |
| | 50 | 20 |
| | | |
| | | |
| (I-b-24) | 100 + 50 | 5 |
| + Mefenpyr | 50 + 50 | 5 |
| | | |

| | | 10 Tage nach Applikation |
|---|---|---|
| | Aufwandmenge | Sommerweizen |
| | g a.i./ha | beobachtet (%) |
| | | |
| (I-b-25) | 100 | 20 |
| | | |
| | | |
| (I-b-25) | 100 + 50 | 0 |
| + Mefenpyr | | |

### Beispiel 12

Heliothis virescens - Test - Behandlung transgener Pflanzen
- Lösungsmittel:: 7 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel 13

Grenzkonzentrations-Test / Bodeninsekten - Behandlung transgener Pflanzen
- Testinsekt:: Diabrotica balteata - Larven im Boden
- Lösungsmittel:: 7 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskömer der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel 14

**Steigerung der Penetration in die Pflanze durch Ammonium- oder Phosphoniumsalze und synergistische Steigerung der Penetration in die Pflanze durch Ammonium- / Phosphoniumsalze in Kombination mit Penetrationsförderern**

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 µl der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 5 bis 6 Messungen. Deutlich ist zu sehen, dass bereits Ammoniumsulfat alleine die Penetration deutlich verbessert und zusammen mit RME ein überadditiver (synergistischer) Effekt auftritt.

| Wirkstoff | Penetration nach 24h / % | | | |
|---|---|---|---|---|
| | EC | EC + AS | EC + | EC + RME (1 g/l) |
| | | (1 g/l) | RME | + AS (1 g/l) |
| | | | (1 g/l) | |
| Beispiel I-a-73 | 0.9 | 2.8 | 2.9 | 34.6 |
| 0.2 g/l in | | | | |
| Wasser/Aceton 6:4 | | | | |
| Beispiel I-a-49 | 0.24 | 0.6 | 1.4 | 26 |
| 0.2 g/l in | | | | |
| Wasser/Aceton 6:4 | | | | |

RME = Rapsölmethylester (Einsatz formuliert als 500 EW, Konzentrationsangabe in g Wirkstoff / 1)

AS = Ammoniumsulfat

EC = Emulgierbares Konzentrat

### Beispiel 15: Wirkungssteigerung durch Ammonium- / Phosphoniumsalze

*Myzus persicae*-Test
- Lösungsmittel:: 7 Gewichtsteile Dimethylformamid
- Emulgator:: 2 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen werden diese in einer Konzentration von 1000 ppm der Spritzbrühe hinzugegeben.

Paprikapflanzen (*Capsicum annuum*), die stark von der Gründen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Wirkstoff | Abtötungsgrad / % | |
|---|---|---|---|
| | ppm | nach 6 Tagen | |
| | | | + AS (1000 ppm) |
| I-a-32 | 20 | 0 | 85 |
| I-a-39 | 20 | 10 | 40 |

| | | | |
|---|---|---|---|
| AS = Ammoniumsulfat | | | |

### Beispiel 16

**Aphis gossypii -Test**

| | |
|---|---|
| Lösungsmittel: 7 | Gewichtsteile Dimethylformamid |
| Emulgator: 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium-oder Phosphoniumsalzen werden diese in einerKonzentration von 1000 ppm der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Wirkstoff | Abtötungsgrad / % | |
|---|---|---|---|
| | ppm | nach 6 Tagen | |
| | | | + AS (1000 ppm) |
| I-a-9 | 20 | 65 | 85 |
| I-a-29 | 20 | 70 | 85 |
| I-a-29 | 4 | 5 | 70 |

### Beispiel 17: Wirkungssteigerung durch Ammonium- / Phosphoniumsalze in Kombination mit Penetrationsförderern

***Myzus persicae* -Test**
- Lösungsmittel:: 7 Gewichtsteile Dimethylformamid
- Emulgator:: 2 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen und Penetrationsförderer (Rapsölmethylester 500 EW) werden diese jeweils in einerKonzen-tration von 1000 ppm der Spritzbrühe hinzugegeben.

Paprikapflanzen (*Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Wirkstoff / | Abtötungsgrad / % | | | |
|---|---|---|---|---|---|
| | ppm | nach 6 Tagen | | | |
| | | | + AS (1000 | +RME(1000 | + RME + AS (je 1000 |
| | | | ppm) | ppm) | ppm) |
| I-a-29 | 20 | 0 | 70 | 95 | 100 |

### Beispiel 18

**Aphis gossypii -Test**
- Lösungsmittel: 7: Gewichtsteile Dimethylformamid
- Emulgator: 2: Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium-oder Phosphoniumsalzen und Penetrationsförderern (Rapsölmethylestern 500 EW) werden diese jeweils in einer Konzentration von 1000 ppm a.i. der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Wirkstoff / | Abtötungsgrad / % | | | |
|---|---|---|---|---|---|
| | ppm | nach 6 Tagen | | | |
| | | | + AS (1000 | + RME (1000 | + RME + AS (je 1000 |
| | | | ppm) | ppm) | ppm) |
| I-a-32 | 4 | 5 | 30 | 70 | 80 |

## Patentansprüche

1. Verbindungen der Formel (I) 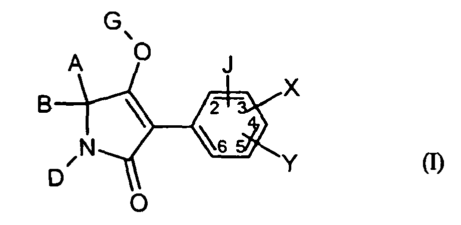 in welcher
J für Trifluormethoxy steht,
X für Wasserstoff, Alkyl, Halogen, Halogenalkyl, Alkoxy oder Halogenalkoxy steht,
Y für Wasserstoff, Alkyl, Alkoxy oder Halogen steht,
mit der Maßgabe, dass mindestens einer der Reste J, X oder Y in der 2-Position des Phenylrestes steht und dabei ungleich Wasserstoff ist,
A für Wasserstoff, jeweils gegebenenfalls durch Halogen substituiertes Alkyl, Alkenyl, Alkoxyalkyl, Alkylthioalkyl, gesättigtes oder ungesättigtes, ge- gebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogen- alkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
D für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen.
G für Wasserstoff (a) oder für eine der Gruppen 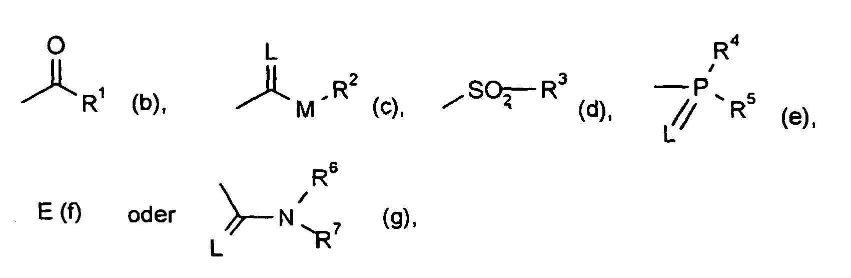
steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alke- nyl, Alkoxyalkyl, Alkylthioalkyl, Polyalkoxyalkyl oder gegebenen- falls durch Halogen, Alkyl oder Alkoxy substituiertes Cycloalkyl, das durch mindestens ein Heteroatom unterbrochen sein kann, je- weils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alke- nyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenen- falls substituiertes Phenyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebe- nenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

2. Verbindungen der Formel (I) gemäß Anspruch 1, in welcher
J für Trifluormethoxy steht,
X für Wasserstoff, Halogen, C₁-C₆-Alkyl, C₁-C₄-Halogenalkyl, C₁-C₆-Alkoxy oder C₁-C₄-Halogenalkoxy steht,
Y für Wasserstoff, C₁-C₆-Alkyl, C₁-C₆-Alkoxy oder Halogen steht,
mit der Maßgabe, dass mindestens einer der Reste J, X oder Y in der 2-Position des Phenylrestes steht und dabei ungleich Wasserstoff ist und wobei sich folgende Phenylsubstitutionsmuster ergeben 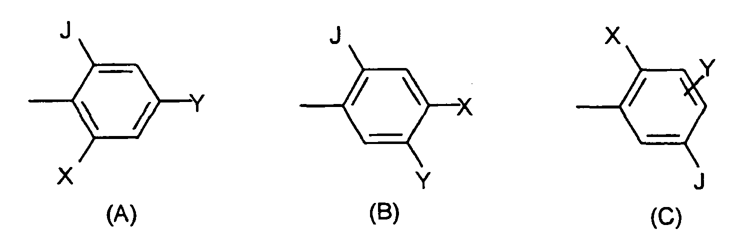 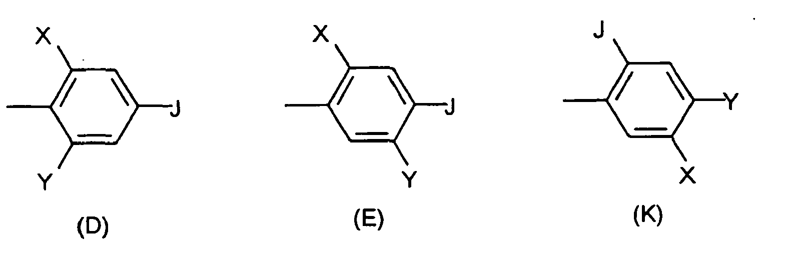 wobei in den Phenylsubstitutionsmustern (C), (D), (E) und (K) X und Y gleichzeitig ungleich Wasserstoff stehen,
A für Wasserstoff oder jeweils gegebenenfalls durch Halogen substituiertes C₁-C₁₂- Alkyl, C₃-C₈-Alkenyl, C₁-C₁₀-Alkoxy-C₁-C8-alkyl, C₁-C₁₀-Alkylthio-C₁-C₆- alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁ - C₆-Alkoxy, C₁-C₆-Halogenalkoxy, Cyano oder Nitro substituiertes Phenyl, Naphthyl, Hetaryl mit 5 bis 6 Ringatomen, Phenyl-C₁-C₆-alkyl oder Naphthyl-C₁- C₆-alkyl steht,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃-C₁₀₋ Cycloalkyl oder ungesättigtes C₅-C₁₀-Cycloalkyl, worin gegebenenfalls ein Ring- glied durch Sauerstoff oder Schwefel ersetzt ist und welche gegebenenfalls einfach oder zweifach durch C₁-C₈-Alkyl, C₁-C₆-Alkoxy-C₁-C₄-Alkyl, C₁-C₆-Alkoxy- C₁-C₄-alkoxy, C₃-C₆-Cycloalkyl-C₁-C₂-alkoxy, C₃-C₁₀-Cycloalkyl, C₁-C₈- Halogenalkyl, C₁-C₈-Alkoxy, C₁-C₈-Alkylthio, Halogen oder Phenyl substituiert sind oder
A, B und das Kohlenstoffatom, an das sie gebunden sind, für C₃-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl-, oder durch eine Alkylendioxyl- oder durch eine Alkylendithioyl- Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅- C₈-Cycloalkenyl stehen, in welchen zwei Substituenten gemeinsam mit den Kohlen- stoffatomen, an die sie gebunden sind, für jeweils gegebenenfalls durch C₁-C₆₋ Alkyl, C₁-C₆-Alkoxy oder Halogen substituiertes C₂-C₆-Alkandiyl, C₂-C₆- Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁- C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen Phenyl-C₁-C₆-alkyl oder Hetaryl-C₁-C₆-alkyl mit 5 oder 6 Ringatomen steht oder
A und D gemeinsam für jeweils gegebenenfalls substituiertes C₃-C₆-Alkandiyl oder C₃-C₆- Alkendiyl stehen, worin gegebenenfalls eine Methylengruppe durch eine Carbonyl- gruppe, Sauerstoff oder Schwefel ersetzt ist und wobei als Substituenten jeweils in Frage kommen: Halogen, Hydroxy, Mercapto oder jeweils gegebenenfalls durch Halogen sub- stituiertes C₁-C₁₀Alkyl, C₁-C₆-Alkoxy, C₁-C₆-Alkylthio, C₃-C₇-Cycloalkyl, Phenyl oder Benzyloxy, oder eine weitere C₃-C₆ Alkandiylgruppierung, C₃-C₆- Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden, der Sauerstoff oder Schwefel enthalten kann, oder worin gegebenenfalls eine der folgenden Gruppen 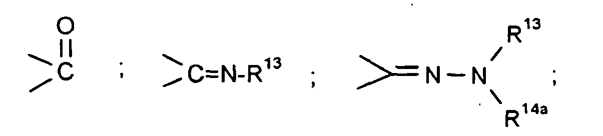 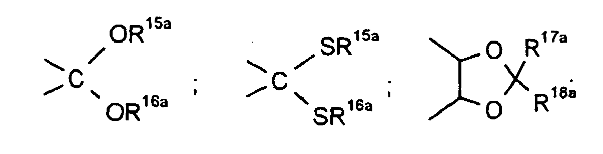 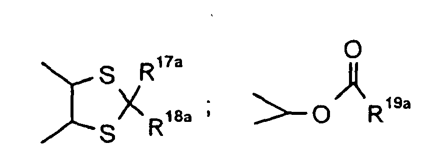
oder 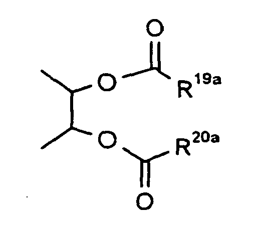 enthalten ist,
G für Wasserstoff (a) oder für eine der Gruppen 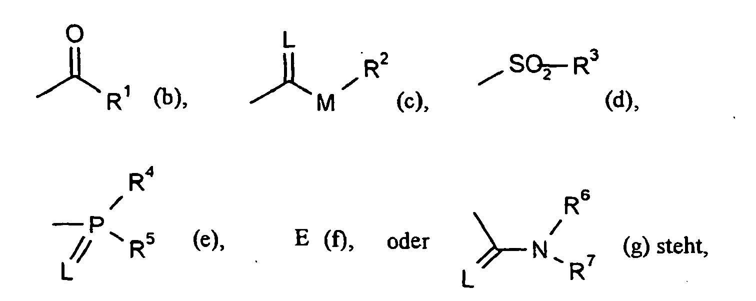
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀- Alkenyl, C₁-C₈-Alkoxy-C₁-C₈-alkyl, C₁-C₈-Alkylthio-C₁-C₈-alkyl, Poly-C₁-C₈- alkoxy-C₁-C₈-alkyl oder gegebenenfalls durch Halogen, C₁-C₆-Alkyl oder C₁-C₆- Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind, für gegebenenfalls durch Halogen, Cyano, Nitro, C₁-C₆-Alkyl, C₁-C₆-Alkoxy, C₁- C₆-Halogenalkyl, C₁-C₆-Halogenalkoxy, C₁-C₆-Alkylthio oder C₁-C₆-Alkylsulfo- nyl substituiertes Phenyl, für gegebenenfalls durch Halogen, Nitro, Cyano, C₁-C₆-Alkyl, C₁-C₆-Alkoxy, C₁- C₆-Halogenalkyl oder C₁-C₆-Halogenalkoxy substituiertes Phenyl-C₁-C₆-alkyl, für gegebenenfalls durch Halogen oder C₁-C₆-Alkyl substituiertes 5- oder 6- gliedriges Hetaryl, für gegebenenfalls durch Halogen oder C₁-C₆-_{A}lkyl substituiertes Phenoxy-C₁-C₆- alkyl oder für gegebenenfalls durch Halogen, Amino oder C₁-C₆-Alkyl substituiertes 5- oder 6-gliedriges Hetaryloxy-C₁-C₆₋alkyl steht,
R² für jeweils gegebenenfalls durch Halogen substituiertes C₁-C₂₀-Alkyl, C₂-C₂₀- Alkenyl, C₁-C₈-Alkoxy-C₂-C₈-alkyl, Poly-C₁-C₈-alkoxy-C₂-C₈-alkyl, für gegebenenfalls durch Halogen, C₁-C₆-Alkyl oder C₁-C₆-Alkoxy substituiertes C₃-C₈-Cycloalkyl oder für jeweils gegebenenfalls durch Halogen, Cyano, Nitro, C₁-C₆-Alkyl, C₁-C₆-Alk- oxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈- Alkylthio, C₂-C₈-Alkenylthio, C₃-C₇-Cycloalkylthio oder für jeweils gegebenen- falls durch Halogen, Nitro, Cyano, C₁-C₄-Alkoxy, C₁-C₄-Halogenalkoxy, C₁-C₄- Alkylthio, C₁-C₄ -Halogenalkylthio, C₁-C₄-Alkyl oder C₁-C₄-Halogenalkyl substi- tuiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈- Alkenyl, C₁-C₈-Alkoxy-C₁-C₈-alkyl, für gegebenenfalls durch Halogen, C₁-C₈- Halogenalkyl, C₁-C₈-Alkyl oder C₁-C₈-Alkoxy substituiertes Phenyl, gegebe- nenfalls durch Halogen, C₁-C₈-Alkyl, C₁-C₈-Halogenalkyl oder C1-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
R¹³ für Wasserstoff, für jeweils gegebenenfalls durch Halogen substituiertes C₁-C₈- Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy steht,
R^{14a} für Wasserstoff oder C₁-C₈-Alkyl steht oder
R¹³ und R^{14a} gemeinsam für C₄-C₆-Alkandiyl stehen,
R^{15a} und R^{16a} gleich oder verschieden sind und für C₁-C₆-Alkyl stehen oder
R^{15a} und R^{16a} gemeinsam für einen C₂-C₄-Alkandiylrest stehen, der gegebenenfalls durch C₁-C₆-Alkyl, C₁-C₆-Halogenalkyl oder durch gegebenenfalls durch Halogen, C₁- C₆-Alkyl, C₁-C₄-Halogenalkyl, C₁-C₆-Alkoxy, C₁-C₄-Halogenalkoxy, Nitro oder Cyano substituiertes Phenyl substituiert ist,
R^{17 a} und R^{18a} unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆₋Alkoxy, C₁-C₄-Halogenalkyl, C₁-C₄-Halogenalkoxy, Nitro oder Cyano substituiertes Phenyl stehen oder
R^{17a} und R^{18a} gemeinsam mit dem Kohlenstoffatom, an das sie gebunden sind, für eine Carbonylgruppe oder für gegebenenfalls durch Halogen, C₁-C₄-Alkyl oder C₁-C₄- Alkoxy substituiertes C₅-C₇-Cycloalkyl stehen, in dem gegebenenfalls eine Methylengruppe durch Sauerstoff oder Schwefel ersetzt ist,
R^{19a} und R^{20a} unabhängig voneinander für C₁-C₁₀-Alkyl, C₂-C₁₀-Alkenyl, C₁-C₁₀- Alkoxy, C₁-C₁₀-Alkyamino, C₃-C₁₀-Alkenylamino, Di-(C₁-C₁₀-alkyl)amino oder Di-(C₃-C₁₀-alkenyl)amino stehen.

3. Verbindungen der Formel (I) gemäß Anspruch 1, in welcher
J für Trifluormethoxy steht,
X für Wasserstoff, Fluor, Chlor, Brom, C₁-C₄-Alkyl, Trifluormethyl oder C₁-C₄- Alkoxy steht,
Y für Wasserstoff, Fluor, Chlor, Brom, C₁-C₄-Alkoxy oder C₁-C₄-Alkyl steht
mit der Maßgabe, dass mindestens einer der Reste J, X oder Y in der 2-Position des Phenylrestes steht und dabei ungleich Wasserstoff ist, wobei sich folgende Phenylsubstitutionsmuster ergeben 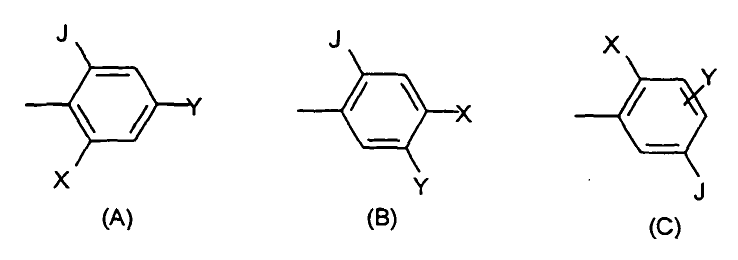 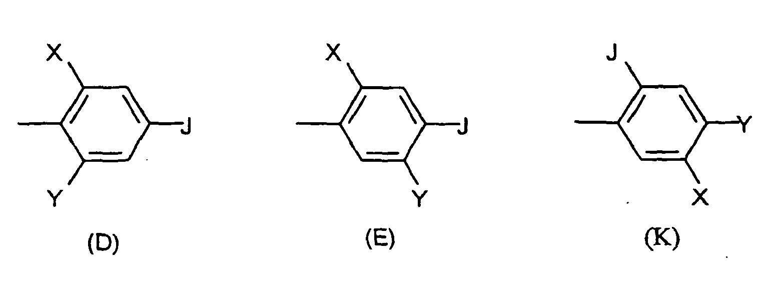 wobei in den Phenylsubstitutionsmustern (C), (D), (E) und (K) X und Y gleichzeitig ungleich Wasserstoff stehen,
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes C₃-C₆-Cycloalkyl, welches gegebenenfalls durch ein Sauerstoffatom unterbrochen sein kann, für jeweils einfach bis zweifach gegebenenfalls durch Fluor, Chlor, Brom, C₁-C₄-Alkyl, C₁- C₂-Halogenalkyl, C₁-C₄-Alkoxy, C₁-C₂-Halogenalkoxy, Cyano oder Nitro substi- tuiertes Phenyl oder Benzyl steht,
B für Wasserstoff, C₁-C₄-Alkyl oder C₁-C₂₋Alkoxyl-C₁-C₂-alkyl steht oder
A, B und das Kohlenstoffatom an das sie gebunden sind, für gesättigtes oder ungesättigtes C₅-C₇-Cycloalkyl, worin gegebenenfalls ein Ringglied durch Sauerstoff oder Schwefel ersetzt ist und welches gegebenenfalls einfach bis zweifach durch C₁-C₆- Alkyl, C₁-C₄-Alkoxy-C₁-C₂-alkyl, C₁-C₄-Alkoxy-C₁-C₂-alkoxy, C₃-C₆- Cycloalkyl-methoxy, Trifluormethyl oder C₁-C₆-Alkoxy, substituiert ist, oder
A, B und das Kohlenstoffatom, an das sie gebunden sind, für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf oder sechsgliedrigen Ring bildet, oder
A, B und das Kohlenstoffatom, an das sie gebunden sind, für C₃-C₆-Cycloalkyl oder C₅- C₆-Cycloalkenyl stehen, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
D für Wasserstoff, für jeweils gegebenenfalls einfach bis dreifach durch Fluor substituiertes C₁-C₆-Alkyl, C₃-C₆-Alkenyl, C₁-C₄-Alkoxy-C₂-C₃-alkyl, für gegebenenfalls einfach bis zweifach durch C₁-C₄-Alkyl, C₁-C₄-Alkoxy oder C₁- C₂-Halogenalkyl substituiertes C₃-C₆-Cycloalkyl steht, in welchem gegebenenfalls eine Methylengruppe durch Sauerstoff ersetzt ist, oder
A und D gemeinsam für gegebenenfalls einfach bis zweifach substituiertes C₃-C₅-Alkandiyl stehen, in welchem eine Methylengruppe durch eine Carbonylgruppe, Sauerstoff oder Schwefel ersetzt sein kann, wobei als Substituenten C₁-C₂-Alkyl oder C₁-C₂- Alkoxy in Frage kommen oder
A und D gemeinsam mit den Atomen, an die sie gebunden sind, für eine der Gruppen AD-1 bis AD-10 stehen: 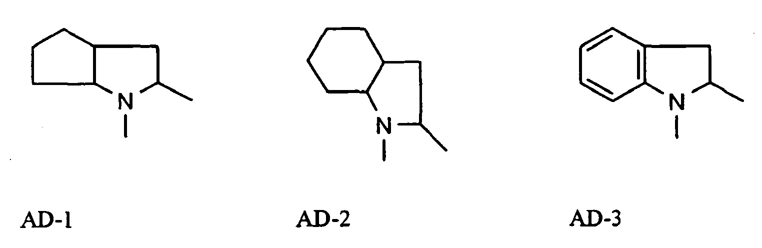 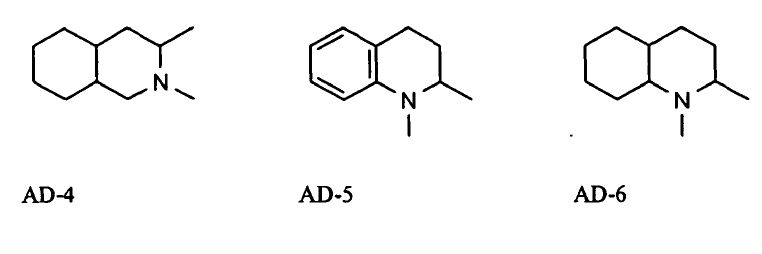 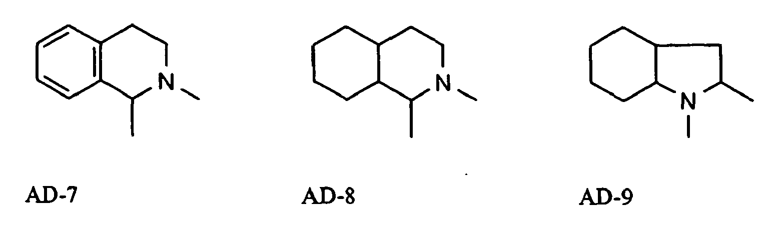 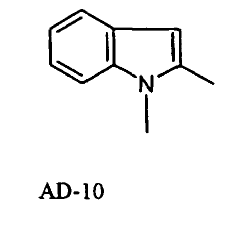
G für Wasserstoff (a) oder für eine der Gruppen 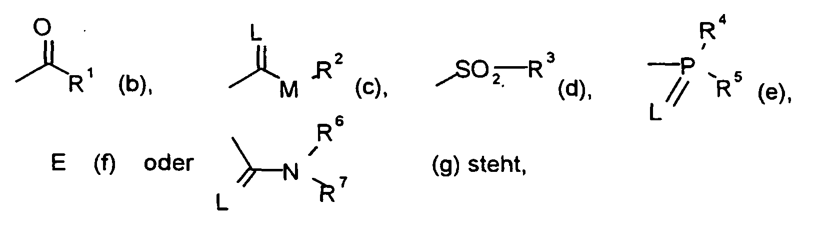
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₈-Alkyl, C₂-C₁₈-Alkenyl C₁-C₄-Alkoxy-C₁-C₂-alkyl, C₁-C₄-Alkylthio-C₁- C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind, für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl steht,
R² für jeweils gegebenenfalls einfach bis dreifach durch Fluor substituiertes C₁-C₈- Alkyl, C₂-C₈-Alkenyl oder C₁-C₄-Alkoxy-C₂-C₄-alkyl für gegebenenfalls einfach durch C₁-C₂-Alkyl oder C₁C₂-Alkoxy substituiertes C₃-C₆-Cycloalkyl oder für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substitu- iertes Phenyl oder Benzyl steht,
R³ für gegebenenfalls einfach bis dreifach durch Fluor substituiertes C₁-C₆-Alkyl oder für jeweils gegebenenfalls einfach durch Fluor, Chlor, Brom, C₁-C₄-Alkyl, C₁-C₄- Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁- C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁- C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio steht,
R⁵ für C₁-C₆-Alkoxy oder C₁-C₆-Alkylthio steht,
R⁶ für Wasserstoff, C₁-C₆-Alkyl_{,} C₃-C₆-Cycloalkyl, C₁-C₆-Alkoxy, C₃-C₆-Alkenyl, C ₁-C₆-Allcoxy-C ₁-C₄-alkyl; für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl steht,
R⁷ für C₁-C₆-Alkyl, C₃-C₆-Alkenyl oder C₁-C₆-Alkoxy-C₁-C₄-alkyl steht,
R⁶ und R⁷ zusammen für einen gegebenenfalls durch Methyl oder Ethyl substituierten C₄- C₅-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
J für Trifluormethoxy steht,
X für Wasserstoff, Fluor, Chlor, Brom, Methyl, Ethyl, Propyl, Methoxy oder Ethoxy steht,
Y für Wasserstoff, Chlor, Brom, Methyl, Ethyl oder Methoxy steht,
mit der Maßgabe, dass mindestens einer der Reste J, X oder Y in der 2-Position des Phenylrestes steht und ungleich Wasserstoff ist, wobei sich
folgende Phenylsubstitutionsmuster 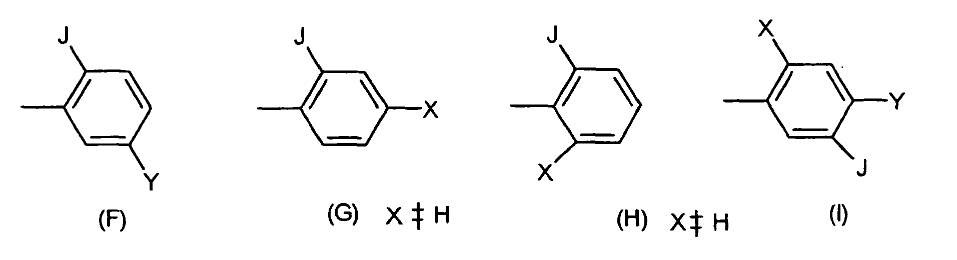 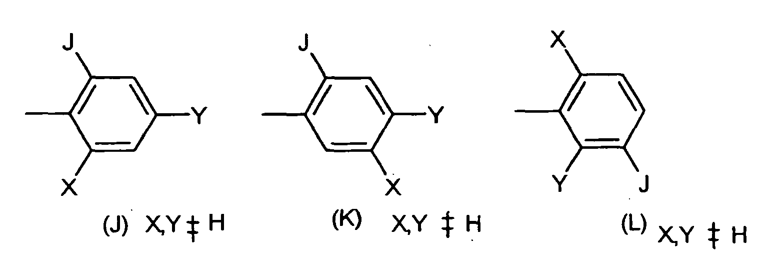 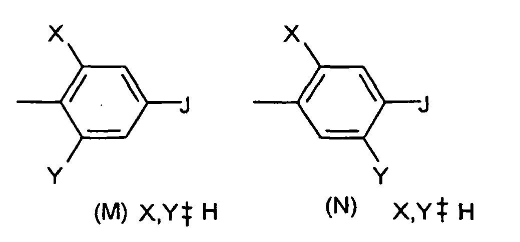 ergeben,
A für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
B für Wasserstoff, Methyl oder Ethyl steht oder
A, B und das Kohlenstoffatom an das sie gebunden sind, für gesättigtes C₅-C₆- Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Methoxymethyl, Methoxymethyl, Propoxymethyl, Trifluormethyl, Methoxy, Ethoxy, Propoxy, Butoxy, Methoxyethyl, Ethoxyethyl, Methoxyethoxy, Ethoxyethoxy, Cyclopropylmethoxy, Cyclopentylmethoxy oder Cyclohexylmethoxy substituiert ist, oder
A, B und das Kohlenstoffatom, an das sie gebunden sind, für C₆-Cycloalkyl, welches
gegebenenfalls durch mit zwei nicht direkt benachbarten Sauerstoffatomen enthaltende Alkylendioxyl-Gruppe substituiert ist, oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
D für Wasserstoff, für jeweils gegebenenfalls einfach bis dreifach durch Fluor
substituiertes C₁-C₄-Alkyl, C₃-C₄-Alkenyl, C₁-C₄-Alkoxy-C₂-C₃-alkyl, für Cyclopropyl, Cyclopentyl oder Cyclohexyl steht,
oder
A und D gemeinsam für gegebenenfalls einfach durch Methyl oder Methoxy substituiertes
C₃-C₅-Alkandiyl, worin gegebenenfalls ein Kohlenstoffatom durch Sauerstoff oder Schwefel ersetzt ist oder für die Gruppe AD-1 stehen,
G für Wasserstoff (a) oder für eine der Gruppen 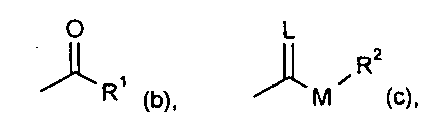 -SO₂-R³ (d) oder E (f) steht ,
in welchen
L für Sauerstoff oder Schwefel steht,
M für Sauerstoff oder Schwefel steht und
E für ein Ammoniumion steht,
R¹ für C₁-C₆-Alkyl, C₂-C₁₇-Alkenyl, C₁-C₂-Alkoxy-C₁-alkyl, C₁-C₂-Alkylthio-C₁- alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆- Alkenyl oder C₁-C₄-Alkoxy-C₂-C₃-alkyl, Phenyl oder Benzyl steht,
R³ für C₁-C₈-Alkyl steht.

5. Verbindungen der Formel (I) gemäß Anspruch 1, in welcher
J für Trifluormethoxy steht,
X für Wasserstoff, Chlor, Brom, Methyl, Ethyl, oder Methoxy steht,
Y für Wasserstoff, Chlor, Brom, Methyl oder Methoxy steht,
mit der Maßgabe, dass mindestens einer der Reste J, X oder Y in der 2-Position des Phenylrestes steht und ungleich Wasserstoff ist, wobei sich
folgende Phenylsubstitutionsmuster ergeben 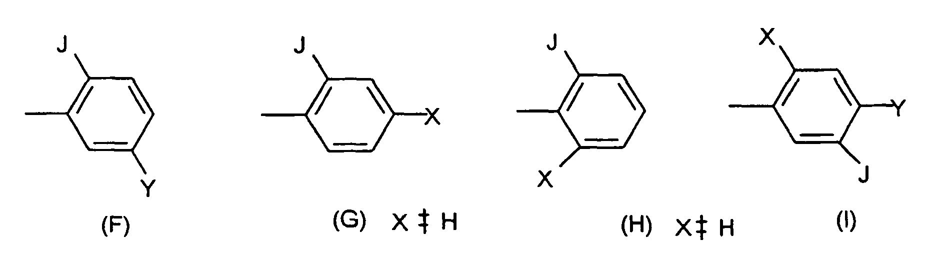 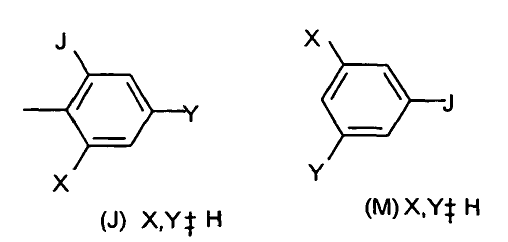
A für C₁-C₄-Alkyl oder Cyclopropyl steht,
B für Wasserstoff oder Methyl steht,
A, B und das Kohlenstoffatom an das sie gebunden sind, für gesättigtes C₅-C₆- Cycloalkyl stehen, in welchem gegebenenfalls ein Ringglied durch Sauerstoff ersetzt ist und welches gegebenenfalls einfach durch Methoxymethyl, Methoxy, Ethoxy, Propoxy, oder Butoxy substituiert ist, oder für 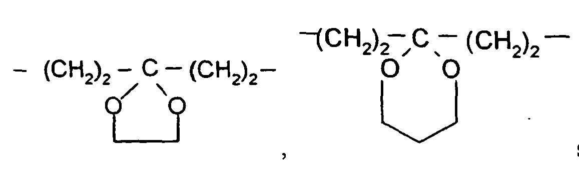
stehen,
D für Wasserstoff oder Cyclopropyl steht, oder
A und D gemeinsam für C₃-C₅-Alkandiyl stehen,
G für Wasserstoff (a) oder für eine der Gruppen 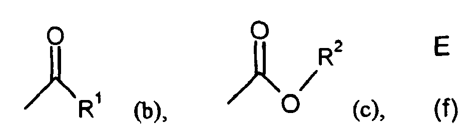
steht,
R¹ für C₁-C₆-Alkyl steht,
R² für C₁-C₈-Alkyl oder Benzyl steht.

6. Verfahren zur Herstellung von Verbindungen der Formel (I) gemäß Anspruch 1, **dadurch gekennzeichnet, dass** man zum Gehalt von
(A) Verbindungen der Formel (I-a) 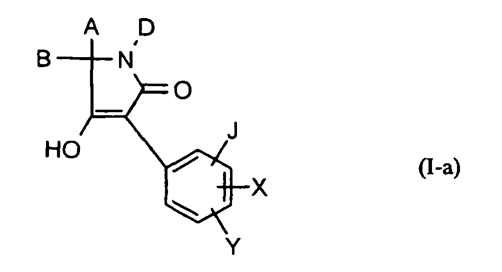 in welcher
A, B, D, J, X und Y die oben angegebenen Bedeutungen haben,
N-Acylaminosäureester der Formel (II) 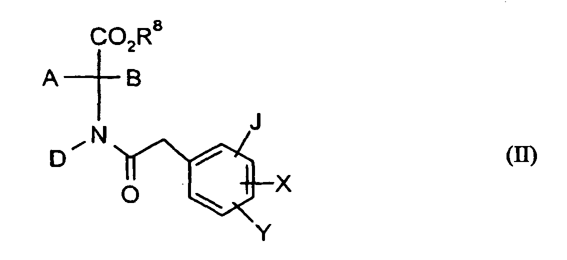 in welcher
A, B, D, J, X und Y die oben angegebenen Bedeutungen haben,
und
R⁸ für Alkyl steht,
in Gegenwart eines Verdünnungsmittels und in Gegenwart einer Base intramolekular kondensiert,
(B) Verbindungen der oben gezeigten Formel (I-b), in welcher A, B, D, J, R¹, X, und Y die oben angegebenen Bedeutungen haben, Verbindungen der oben gezeigten Formel (I-a), in welcher A, B, D, J, X und Y die oben angegebenen Bedeutungen haben, jeweils
(α) mit Säurehalogeniden der Formel (III) 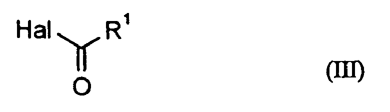 in welcher
R¹ die oben angegebene Bedeutung hat und
Hal für Halogen steht
oder
(β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(C) Verbindungen der oben gezeigten Formel (I-c), in welcher A, B, D, J, R², M, X und Y die oben angegebenen Bedeutungen haben und L für Sauerstoff steht, Verbindungen der oben gezeigten Formel (I-a), in welcher A, B, D, J, X und Y die oben angegebenen Bedeutungen haben, jeweils
mit Chlorameisensäureestern oder Chlorameisensäurethioestern der Formel (V)
R²-M-CO-Cl (V)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(D) Verbindungen der oben gezeigten Formel (I-c), in welcher A, B, D, J, R², M, X und Y die oben angegebenen Bedeutungen haben und L für Schwefel steht, Verbindungen der oben gezeigten Formel (I-a), in welcher A, B, D, J, X und Y die oben angegebenen Bedeutungen haben, jeweils
mit Chlormonothioameisensäureestern oder Chlordithioameisensäureestern der Formel (VI) 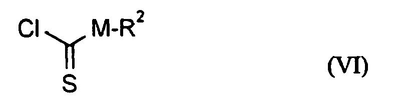 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(E) Verbindungen der oben gezeigten Formel (I-d), in welcher A, B, D, J, R³, X und Y die oben angegebenen Bedeutungen haben, Verbindungen der oben gezeigten Formel (I-a), in welcher A, B, D, J, X und Y die oben angegebenen Bedeutungen haben, jeweils
mit Sulfonsäurechloriden der Formel (VII)
R³-SO₂-Cl (VII)
in welcher
R³ die oben angegebene Bedeutung hat,
gegebenenfalls in Gegenwart eines Verdünnungsmittels und gegebenenfalls in Gegenwart eines Säurebindemittels umsetzt,
(F) Verbindungen der oben gezeigten Formel (I-e), in welcher A, B, D, J, L, R⁴, R⁵, X und Y die oben angegebenen Bedeutungen haben, Verbindungen der oben gezeigten Formel (I-a), in welcher A, B, D, J, X und Y die oben angegebenen Bedeutungen haben, jeweils
mit Phosphorverbindungen der Formel (VIII) 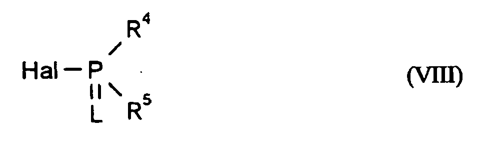 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(G) Verbindungen der oben gezeigten Formel (I-f), in welcher A, B, D, E, J, X und Y die oben angegebenen Bedeutungen haben, Verbindungen der Formel (I-a), in welcher A, B, D, J, X und Y die oben angegebenen Bedeutungen haben, jeweils
mit Metallverbindungen oder Aminen der Formeln (IX) oder (X) 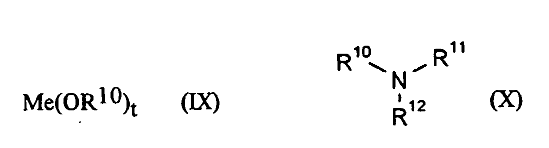 in welchen
Me für ein ein- oder zweiwertiges Metall oder für ein Ammoniumion 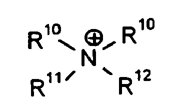
t für die Zahl 1 oder 2 und
R¹⁰, R¹¹, R¹² unabhängig voneinander für Wasserstoff oder Alkyl stehen, gegebenenfalls in Gegenwart eines Verdünnungsmittels umsetzt,
(H) Verbindungen der oben gezeigten Formel (I-g), in welcher A, B, D, J, L, R⁶, R⁷, X und Y die oben angegebenen Bedeutungen haben, Verbindungen der oben gezeigten Formel (I-a), in welcher A, B, D, J, X und Y die oben angegebenen Bedeutungen haben, jeweils
(α) mit Isocyanaten oder Isothiocyanaten der Formel (XI)
R⁶-N=C=L (XI)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XII) 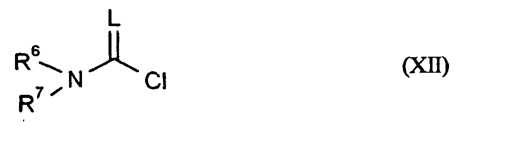 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

7. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

8. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

9. Verwendung von Verbindungen der Formel (I) gemäß Anspruch 1 zur Bekämpfung von tierischen Schädlingen und/oder unerwünschtem Pflanzenbewuchs.

10. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen und/oder unerwünschtem Pflanzenwuchs.

12. Mittel enthaltend einen wirksamen Gehalt an einer Wirkstoffkombination umfassend als Komponenten
(a') mindestens ein trifluormethoxy-phenylsubstituiertes Tetramsäure-Derivat der Formel (I),
in welcher A, B, D, G, J, X und Y die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxyessigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-hamstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl-vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-ylmethyl)-N-(2-propenyl)-acetamid (PPG-1292), 3-Dichloracetyl-2,2-dimethyl-oxazolidin (R-28725), 3-Dichloracetyl-2,2,5-trimethyhxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlorchinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylaminocarbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 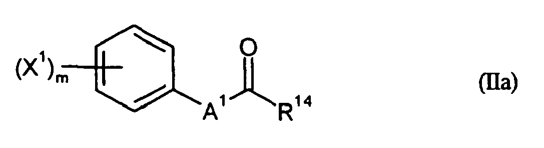 oder der allgemeinen Formel (IIb) 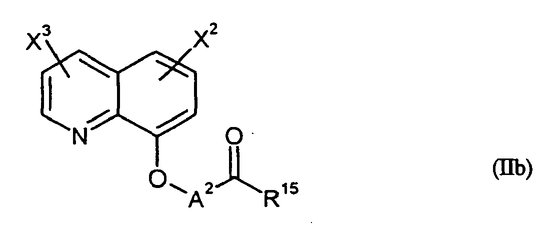 oder der Formel (IIc) 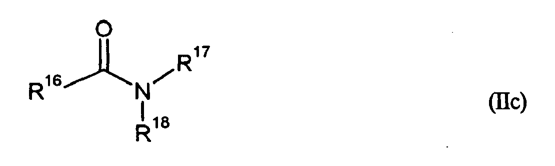 wobei
m für eine Zahl 0, 1, 2, 3, 4 oder 5 steht,
A¹ für eine der nachstehend skizzierten divalenten heterocyclischen Gruppierungen steht, 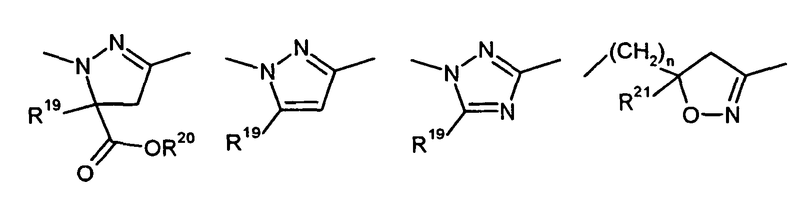
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁-C₄. Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Akylamino oder Di-(C₁-C₄-alkyl)-amino steht,
R¹⁵ für Hydroxy, Mercapto, Amino, C₁-C₇-Alkoxy, C₁-C₆Alkenyloxy, C₁-C₆-Alkenyloxy- C₁-C₆-alkoxy, C₁-C₆-Akylthio, C₁-C₆Alkylamino oder Di-(C₁-C₄-alkyl)-amino steht,
R¹⁶ für jeweils gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy, Dioxolanyl- C₁-C₄-alkyl, Furyl, Furyl-C₁-C₄-alkyl, Thienyl, Thiazolyl, Piperidinyl, oder gegebenen- falls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl- C₁-C₄-alkyl, Furyl, Furyl-C₁-C₄-alkyl, Thienyl, Thiazolyl, Piperidinyl, oder gegebenen- falls durch Fluor, Chlor und/oder Brom oder C₁-Cₐ-Alkyl substituiertes Phenyl,
R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄- Halogenalkoxy steht,
X² für Wasserstoff, Cyano, Nitro, Halogen, C₁-C₄-Alkyl, C₁-C₄-Halogenalkyl, C₁-C₄- Alkoxy oder C₁-C₄-Haloganalkoxy steht,
X³ für Wasserstoff, Cyano, Nitro, Halogen, C₁-C₄-Alkyl, C₁-C₄-Halogenalkyl, C-C₄- Alkoxy oder C₁-C₄-Halogenalkoxy steht,
und/oder die folgenden durch allgemeine Formeln definierten Verbindungen der allgemeinen Formel (IId) 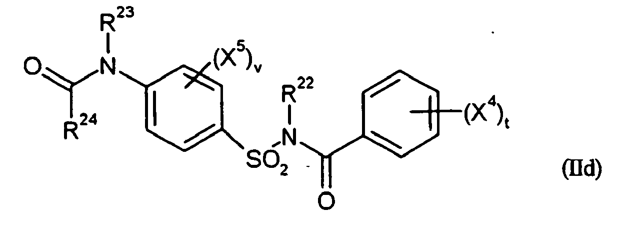
oder der allgemeinen Formel (IIe) 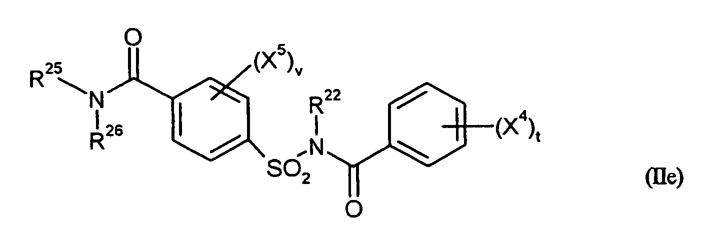 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy sub- stituiertes C₁-C₆-Allcyl, C₁-C₆-Alkoxy, C₁-C₆-Alkylthio, C₁-C₆-Alkylamino oder Di-(C₁- C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆Cycloalkylthio oder C₃-C₆- Cycloalkylamino steht,
R²⁵ für Wasserstoff gegebenenfalls durch Cyano, Hydroxy, Halogen oder C₁-C₄-Alkoxy substituiertes C₁-C₆-Alkyl, jeweils gegebenenfalls durch Cyano oder Halogen substitu- iertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl,jeweils gegebenenfalls durch Cyano oder Halogen substitu- iertes C₃-C₆-Alkenyl oder C₃-C₆-Alkinyl, gegebenenfalls durch Cyano, Halogen oder C₁- C₄-Alkyl substituiertes C₃-C₆-Cycloalkyl, oder gegebenenfalls durch Nitro, Cyano, Halogen, C₁-C₄ Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁- C₄-Alkyl substituiertes C₂-C₆-Alkandiyl oder C₂-C₅-Oxaalkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogen- alkoxy steht, und
x⁵ für Nitro, Cyano, Carboxy, Carbamoyl, Formyl, Sulfamoyl, Hydroxy, Amino, Halogen, C₁-C₄-Akyl, C₁-C₄-Halogenalkyl, C₁-C₄-Alkoxy oder C₁-C₄-Halogen- alkoxy steht.

13. Mittel nach Anspruch 12, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 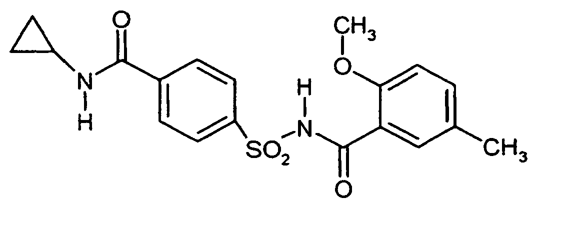 und

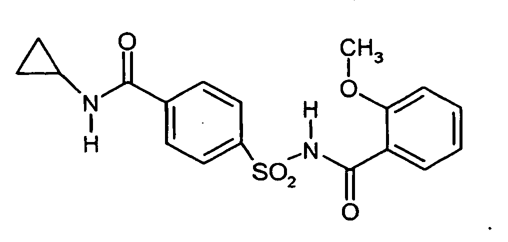

14. Mittel gemäß einem der Ansprüche 12 oder 13, bei denen die Kulturpflanzen-Verträglichkeit verbessernde Verbindung Cloquintocet-mexyl ist.

15. Mittel gemäß einem der Ansprüche 12 oder 13, bei denen die Kulturpflanzen-Verträglichkeit verbessernde Verbindung Mefenpyr-diethyl ist.

16. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

17. Verwendung eines Mittels gemäß Anspruch 12 zum Bekämpfen von unerwünschten Pflanzenwuchs.

18. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

19. Verbindungen der Formel (II) 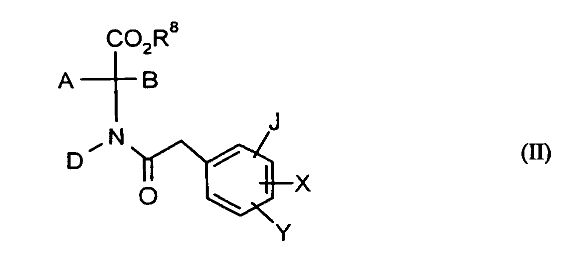 in welcher
A, B, D, J, X und Y und R⁸ die oben angegebenen Bedeutungen haben.

20. Verbindungen der Formel (XV) 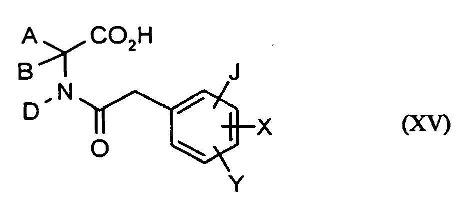 in welcher
A, B, D, J, X und Y die oben angegebenen Bedeutungen haben.

21. Verbindungen der Formel (XIV) 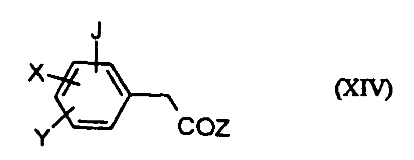 in welcher
Z. für eine durch Carbonsäureaktivierungsreagenzien wie Carbonyldiimidazol, Carbonyl- diimide (wie z.B. Dicyclohexylcarbondiimid), Phosphorylierungsreagenzien (wie z.B. POCl₃, BOP-Cl), Halogenierungsmittel z.B. Thionylchlorid, Oxalylchlorid, Phosgen oder Chlorameisensäsureester eingeführte Abgangsgruppe steht, J, X und Y die oben angegebenen Bedeutungen haben.

22. Verbindungen der Formel (XIX) 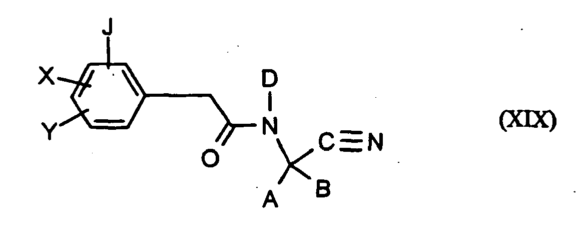 in welcher
A, B, D, J, X und Y die oben angegebenen Bedeutungen haben.

23. Verbindungen der Formel (XVII) 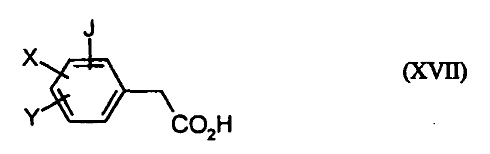 in welcher
J, X und Y die in der Tabelle angegebene Bedeutung haben
| **J** | **X** | **Y** |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-Cl | 6-Cl |
| 4-OCF₃ | 2-OCH₃ | 6-Cl |
| 4-OCF₃ | 2-OCH₃ | 6-Br |
| 4-OCF₃ | 2-CH₃ | 6-CH₃ |
| 4-OCF₃ | 2-Cl | 6-CH₃ |
| 2-OCF₃ | 6-Cl | 4-Br |
| 2-OCF₃ | 6-CH₃ | 4-CH₃ |
| 2-OCF₃ | 6-OCH₃ | 4-Cl |
| 2-OCF₃ | 6-Cl | 4-CH₃ |
| 2-OCF₃ | 6-C₂H₅ | 4-Cl |

24. Verbindungen der Formel (XX) 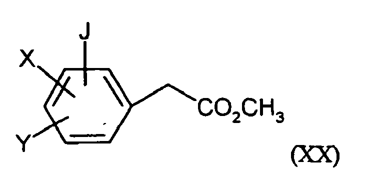 in welcher J, X un Y die in der Tabelle angegebenen Bedeutungen haben
| **J** | **X** | **Y** |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-CH₃ | 6-CH₃ |
| 4-OCF₃ | 2-Br | 6-Cl |
| 4-OCF₃ | 2-Cl | 6-CH₃ |
| 2-OCF₃ | 6-Br | 4-Br |
| 2-OCF₃ | 6-CH₃ | 4-CH₃ |
| 2-OCF₃ | 6-OCH₃ | 4-Cl |
| 2-OCF₃ | 6-CH₃ | 4-Cl |
| 2-OCF₃ | 6-Cl | 4-CH₃ |
| 2-OCF₃ | 6-Cl | 4-Br |

25. Zusammensetzung umfassend
- mindestens eine Verbindung der Formel (I) gemäß Anspruch 1 oder ein Mittel gemäß Anspruch 12 und
- mindestens ein Salz der Formel (III') 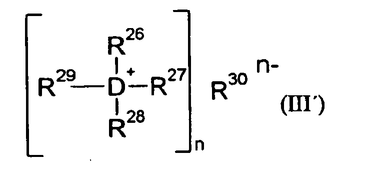 in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

26. Zusammensetzung gemäß Anspruch 25 **dadurch gekennzeichnet, dass** sie mindestens einen Penetrationsförderer enthält.

27. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 12, **dadurch gekennzeichnet, dass** das anwendungsfertige Mittel (Spritzbrühe) unter Einsatz eines Salzes der Formel (III') gemäß Anspruch 25zubereitet wird.

28. Verfahren gemäß Anspruch 27 **dadurch gekennzeichnet, dass** die Spritzbrühe unter Einsatz eines Penetrationsförderers zubereitet wird.

## Claims

1. Compounds of the formula (I) 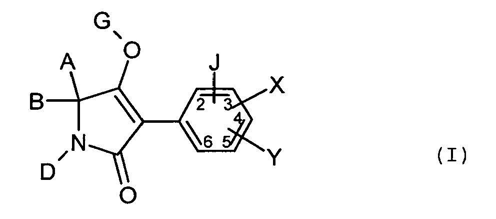 in which
J represents trifluoromethoxy,
X represents hydrogen, alkyl, halogen, haloalkyl, alkoxy or haloalkoxy,
Y represents hydrogen, alkyl, alkoxy or halogen,
with the proviso, that at least one of the radicals J, X and Y is located in the 2-position of the phenyl radical and is not hydrogen,
A represents hydrogen, in each case optionally halogen-substituted alkyl, alkenyl, alkoxyalkyl, alkylthioalkyl, saturated or unsaturated, optionally substituted cycloalkyl in which optionally at least one ring atom is replaced by a heteroatom, or in each case optionally halogen-, alkyl-, haloalkyl-, alkoxy-, haloalkoxy-, cyano- or nitro-substituted aryl, arylalkyl or hetaryl,
B represents hydrogen, alkyl or alkoxyalkyl, or
A and B together with the carbon atom to which they are attached represent a saturated or unsaturated, unsubstituted or substituted cycle which optionally contains at least one heteroatom,
D represents hydrogen or an optionally substituted radical from the group consisting of alkyl, alkenyl, alkynyl, alkoxyalkyl, saturated or unsaturated cycloalkyl in which optionally one or more ring members are replaced by heteroatoms, arylalkyl, aryl, hetarylalkyl or hetaryl or
A and D together with the atoms to which they are attached represent a saturated or unsaturated cycle which optionally contains at least one heteroatom and which is unsubstituted or substituted in the A,D moiety,
G represents hydrogen (a) or represents one of the groups 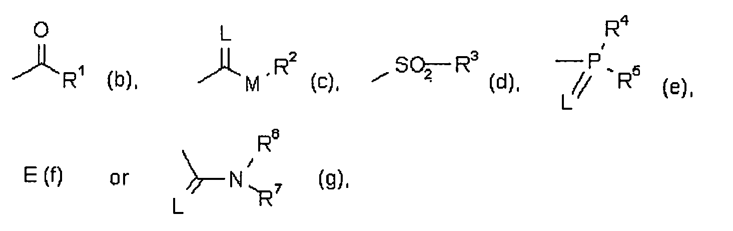
in which
E represents a metal ion equivalent or an ammonium ion,
L represents oxygen or sulphur,
M represents oxygen or sulphur,
R¹ represents in each case optionally halogen-substituted alkyl, alkenyl, alkoxyalkyl, alkylthioalkyl, polyalkoxyalkyl or optionally halogen-, alkyl- or alkoxy-substituted cycloalkyl which may be interrupted by at least one heteroatom, in each case optionally substituted phenyl, phenylalkyl, hetaryl, phenoxyalkyl or hetaryloxy- alkyl,
R² represents in each case optionally halogen-substituted alkyl, alkenyl, alkoxyalkyl, polyalkoxyalkyl or represents in each case optionally substituted cycloalkyl, phenyl or benzyl,
R³, R⁴ and R⁵ independently of one another represent in each case optionally halogen-substituted alkyl, alkoxy, alkylamino, dialkylamino, alkylthio, alkenylthio, cycloalkylthio or represent in each case optionally substituted phenyl, benzyl, phenoxy or phenylthio,
R⁶ and R⁷ independently of one another represent hydrogen, in each case optionally durch halogen-substituted alkyl, cycloalkyl, alkenyl, alkoxy, alkoxyalkyl, represent optionally substituted phenyl, represent optionally substituted benzyl, or together with the nitrogen atom to which they are attached represent a cycle which is optionally interupted by oxygen or sulphur.

2. Compounds of the formula (I) according to Claim 1 in which
J represents trifluoromethoxy,
X represents hydrogen, halogen, C₁-C₆-alkyl, C₁-C₄- haloalkyl, C₁-C₆-alkoxy or C₁-C₄-haloalkoxy,
Y represents hydrogen, C₁-C₆-alkyl, C₁-C₆-alkoxy or halogen,
with the proviso, that at least one of the radicals J, X and Y is located in the 2-position of the phenyl radical and is not hydrogen, resulting in the phenyl substitution patterns below 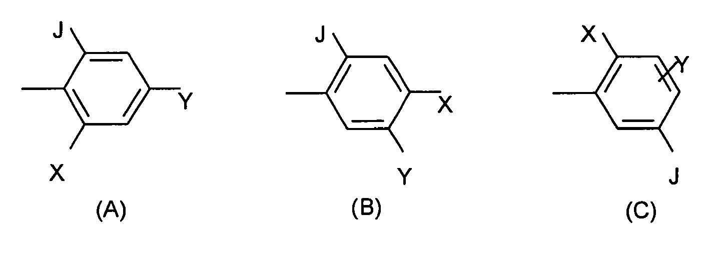 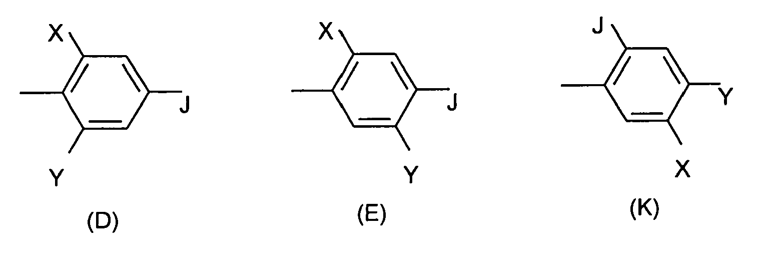 where in the phenyl substitution patterns (C), (D), (E) and (K) X and Y are simultaneously not hydrogen,
A represents hydrogen or in each case optionally halogen-substituted C₁-C₁₂-alkyl, C₃-C₈-alkenyl, C₁- C₁₀-alkoxy-C₁-C₈-alkyl, C₁-C₁₀-alkylthio-C₁-C₆-alkyl, optionally halogen-, C₁-C₆-alkyl- or C₁-C₆-alkoxy-substituted C₃-C₈-cycloalkyl in which optionally one or two not directly adjacent ring members are replaced by oxygen and/or sulphur or represents in each case optionally halogen-, C₁-C₆-alkyl-, C₁- C₆-haloalkyl-, C₁-C₆-alkoxy-, C₁-C₆-haloalkoxy-, cyano- or nitro-substituted phenyl, naphthyl, hetaryl having 5 to 6 ring atoms, phenyl-C₁-C₆- alkyl or naphthyl-C₁-C₆-alkyl,
B represents hydrogen, C₁-C₁₂-alkyl or C₁-C₈-alkoxy- C₁-C₆-alkyl or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₈-alkyl, C₁-C₆-alkoxy-C₁-C₄-alkyl, C₁-C₆- alkoxy-C₁-C₄-alkoxy, C₃-C₆-cycloalkyl-C₁-C₂- alkoxy, C₃-C₁₀-cycloalkyl, C₁-C₈-haloalkyl, C₁-C₈- alkoxy, C₁-C₈-alkylthio, halogen or phenyl or
A, B and the carbon atom to which they are attached represent C₃-C₆-cycloalkyl which is substituted by an alkylenediyl group which is optionally substituted by C₁-C₄-alkyl and optionally contains one or two not directly adjacent oxygen and/or sulphur atoms, or by an alkylenedioxyl or by an alkylenedithioyl group which, together with the carbon atom to which it is attached, forms a further five- to eight-membered ring or
A, B and the carbon atom to which they are attached represent C₃-C₈-cycloalkyl or C₅-C₈-cycloalkenyl in which two substituents together with the carbon atoms to which they are attached represent in each case optionally C₁-C₆-alkyl-, C₁-C₆-alkoxy- or halogen-substituted C₂-C₆-alkanediyl, C₂-C₆- alkenediyl or C₄-C₆-alkanedienediyl in which optionally one methylene group is replaced by oxygen or sulphur,
D represents hydrogen, in each case optionally halogen-substituted C₁-C₁₂-alkyl, C₃-C₈-alkenyl, C₃-C₈-alkynyl, C₁-C₁₀-alkoxy-C₂-C₈-alkyl, optionally halogen-, C₁-C₄-alkyl-, C₁-C₄-alkoxy- or C₁-C₄-haloalkyl-substituted C₃-C₈-cycloalkyl in which optionally one ring member is replaced by oxygen or sulphur or in each case optionally halogen-, C₁-C₆-alkyl-, C₁-C₆-haloalkyl-, C₁-C₆- alkoxy-, C₁-C₆-haloalkoxy-, cyano- or nitro- substituted phenyl, hetaryl having 5 or 6 ring atoms, phenyl-C₁-C₆-alkyl or hetaryl-C₁-C₆-alkyl having 5 or 6 ring atoms or
A and D together represent in each case optionally substituted C₃-C₆-alkanediyl or C₃-C₆-alkenediyl in which optionally one methylene group is replaced by a carbonyl group, oxygen or sulphur,
possible substituents being in each case:
halogen, hydroxyl, mercapto or in each case optionally halogen-substituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl grouping, C₃-C₆-alkenediyl grouping or a butadienyl grouping which is optionally substituted by C₁-C₆-alkyl or in which optionally two adjacent substituents together with the carbon atoms to which they are attached form a further saturated or unsaturated cycle having 5 or 6 ring atoms which may contain oxygen or sulphur or which optionally contains one of the following groups 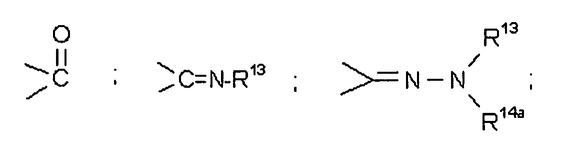 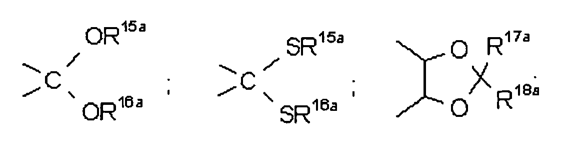 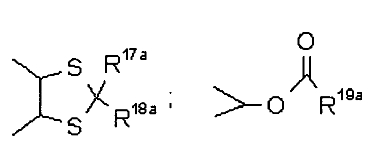 or 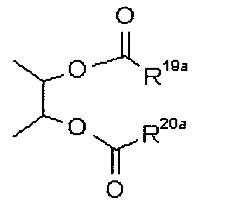 G represents hydrogen (a) or represents one of the groups 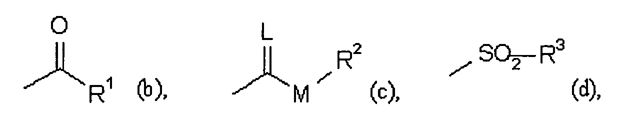 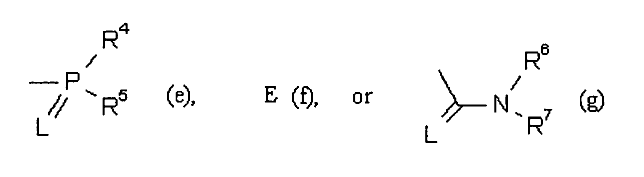 in which
E represents a metal ion equivalent or an ammonium ion,
L represents oxygen or sulphur and
M represents oxygen or sulphur,
R¹ represents in each case optionally halogen- substituted C₁-C₂₀-alkyl, C₂-C₂₀-alkenyl, C₁-C₈- alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur, represents optionally halogen-, cyano-, nitro-, C₁-C₆-alkyl-, C₁-C₆-alkoxy-, C₁-C₆-haloalkyl-, C₁- C₆-haloalkoxy-, C₁-C₆-alkylthio-or C₁-C₆-alkyl- sulphonyl-substituted phenyl, represents optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl, represents optionally halogen- or C₁-C₆-alkyl- substituted 5- or 6-membered hetaryl, represents optionally halogen- or C₁-C₆-alkyl- substituted phenoxy-C₁-C₆-alkyl or represents optionally halogen-, amino- or C₁-C₆- alkyl-substituted 5- or 6-membered hetaryloxy-C₁- C₆-alkyl,
R² represents in each case optionally halogen- substituted C₁-C₂₀-alkyl, C₂-C₂₀-alkenyl, C₁-C₈- alkoxy-C₂-C₈-alkyl, poly-C₁-C₈-alkoxy-C₂-C₈-alkyl, represents optionally halogen-, C₁-C₆-alkyl- or C₁-C₆-alkoxy-substituted C₃-C₈-cycloalkyl or represents in each case optionally halogen-, cyano-, nitro-, C₁-C₆-alkyl-, C₁-C₆-alkoxy-, C₁- C₆-haloalkyl- or C₁-C₆-haloalkoxy-substituted phenyl or benzyl,
R³ represents optionally halogen-substituted C₁-C₈- alkyl or represents in each case optionally halogen-, C₁-C₆-alkyl-, C₁-C₆-alkoxy-, C₁-C₄-halo- alkyl-, C₁-C₄-haloalkoxy-, cyano- or nitro- substituted phenyl or benzyl,
R⁴ and R⁵ independently of one another represent in each case optionally halogen-substituted C₁-C₈- alkyl, C₁-C₈-alkoxy, C₁-C₈-alkylamino, di-(C₁-C₈- alkyl)amino, C₁-C₈-alkylthio, C₂-C₈-alkenylthio, C₃-C₇-cycloalkylthio or represent in each case optionally halogen-, nitro-, cyano-, C₁-C₄-alkoxy, C₁-C₄-haloalkoxy-, C₁-C₄-alkylthio-, C₁-C₄-halo- alkylthio-, C₁-C₄-alkyl- or C₁-C₄-haloalkyl- substituted phenyl, phenoxy or phenylthio,
R⁶ and R⁷ independently of one another represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈- alkyl, represent optionally halogen-, C₁-C₈- haloalkyl-, C₁-C₈-alkyl- or C₁-C₈-alkoxy- substituted phenyl, optionally halogen-, C₁-C₈- alkyl-, C₁-C₈-haloalkyl- or C₁-C₈-alkoxy- substituted benzyl or together represent an optionally C₁-C₄-alkyl-substituted C₃-C₆-alkylene radical in which optionally one carbon atom is replaced by oxygen or sulphur, R¹³ represents hydrogen, represents in each case optionally halogen-substituted C₁-C₈-alkyl or C₁- C₈-alkoxy, represents optionally halogen-, C₁-C₄- alkyl- or C₁-C₄-alkoxy-substituted C₃-C₈- cycloalkyl in which optionally one methylene group is replaced by oxygen or sulphur, or represents in each case optionally halogen-, C₁-C₆-alkyl-, C₁- C₆-alkoxy-, C₁-C₄-haloalkyl-, C₁-C₄-haloalkoxy-, nitro- or cyano-substituted phenyl, phenyl-C₁-C₄- alkyl or phenyl-C₁-C₄-alkoxy,
R^{14a} represents hydrogen or C₁-C₈-alkyl or
R¹³ and R^{14a} together represent C₄-C₆-alkanediyl,
R^{15a} and R^{16a} are identical or different and represent C₁-C₆-alkyl or
R^{15a} and R^{16a} together represent a C₂-C₄-alkanediyl radical which is optionally substituted by C₁-C₆- alkyl, C₁-C₆-haloalkyl or by optionally halogen-, C₁-C₆-alkyl-, C₁-C₄-haloalkyl-, C₁-C₆-alkoxy-, C₁- C₄-haloalkoxy-, nitro- or cyano-substituted phenyl,
R^{17a} and R^{18a} independently of one another preferably represent hydrogen, represent optionally halogen- substituted C₁-C₈-alkyl or represent optionally halogen-, C₁-C₆-alkyl-, C₁-C₆-alkoxy-, C₁-C₄- haloalkyl-, C₁-C₄-haloalkoxy-, nitro- or cyano- substituted phenyl or
R^{17a} and R^{18a} together with the carbon atom to which they are attached represent a carbonyl group or represent optionally halogen-, C₁-C₄-alkyl- or C₁- C₄-alkoxy-substituted C₅-C₇-cycloalkyl in which optionally one methylene group is replaced by oxygen or sulphur,
R^{19a} and R^{20a} independently of one another represent C₁-C₁₀-alkyl, C₂-C₁₀-alkenyl, C₁-C₁₀-alkoxy, C₁- C₁₀-alkylamino, C₃-C₁₀-alkenylamino, di-(C₁-C₁₀- alkyl)amino or di-(C₃-C₁₀-alkenyl)amino.

3. Compounds of the formula (I) according to Claim 1 in which
J represents trifluoromethoxy,
X represents hydrogen, fluorine, chlorine, bromine, C₁-C₄-alkyl, trifluoromethyl or C₁-C₄-alkoxy,
Y represents hydrogen, fluorine, chlorine, bromine, C₁-C₄-alkoxy or C₁-C₄-alkyl, with the proviso, that at least one of the radicals J, X and Y is located in the 2-position of the phenyl radical and is not hydrogen, resulting in the phenyl substitution patterns below 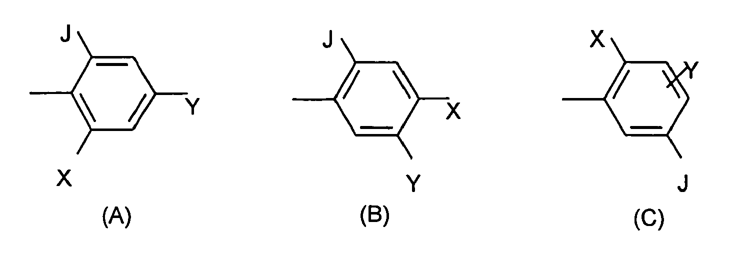 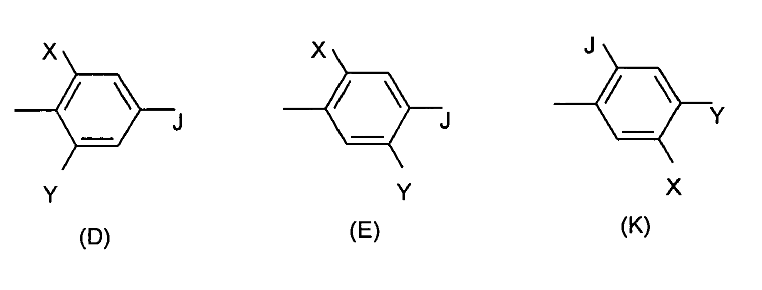
where in the phenyl substitution patterns (C), (D), (E) and (K) X and Y are simultaneously not hydrogen,
A represents hydrogen, represents C₁-C₆-alkyl or C₁- C₄-alkoxy-C₁-C₂-alkyl, each of which is optionally mono- to trisubstituted by fluorine or chlorine, represents C₃-C₆-cycloalkyl which is optionally mono- or disubstituted by C₁-C₂-alkyl or C₁-C₂- alkoxy and which may optionally be interrupted by an oxygen atom, represents phenyl or benzyl, each of which is optionally mono- or disubstituted by fluorine, chlorine, bromine, C₁-C₄-alkyl, C₁-C₂- haloalkyl, C₁-C₄-alkoxy, C₁-C₂-haloalkoxy, cyano or nitro,
B represents hydrogen, C₁-C₄-alkyl or C₁-C₂-alkoxyl-C₁- C₂-alkyl or
A, B and the carbon atom to which they are attached represent saturated or unsaturated C₅-C₇- cycloalkyl in which optionally one ring member is replaced by oxygen or sulphur and which is optionally mono- or disubstituted by C₁-C₆-alkyl, C₁-C₄-alkoxy-C₁-C₂-alkyl, C₁-C₄-alkoxy-C₁-C₂- alkoxy, C₃-C₆-cycloalkyl-methoxy, trifluoromethyl or C₁-C₆-alkoxy, or
A, B and the carbon atom to which they are attached C₅- C₆-cycloalkyl which is substituted by an alkylenediyl group which is optionally substituted by methyl or ethyl and optionally contains one or two not directly adjacent oxygen and/or sulphur atoms, or by an alkylenedioxyl or by an alkylenedithiol group which, together with the carbon atom to which it is attached, forms a further five- to eight-membered ring, or
A, B and the carbon atom to which they are attached represent C₃-C₆-cycloalkyl or C₅-C₆-cycloalkenyl in which two substituents together with the carbon atoms to which they are attached represent in each case optionally C₁-C₂-alkyl- or C₁-C₂-alkoxy- substituted C₂-C₄-alkanediyl, C₂-C₄-alkenediyl or butadiendiyl,
D represents hydrogen, represents C₁-C₆-alkyl, C₃-C₆- alkenyl or C₁-C₄-alkoxy-C₂-C₃-alkyl, each of which is optionally mono- to trisubstituted by fluorine, represents C₃-C₆-cycloalkyl which is optionally mono- or disubstituted by C₁-C₄-alkyl, C₁-C₄- alkoxy or C₁-C₂-haloalkyl and in which optionally one methylene group is replaced by oxygen, or
A and D together represent C₃-C₅-alkanediyl in which one methylene group may be replaced by a carbonyl group, oxygen or sulphur and which may optionally be mono- or disubstituted, possible substituents being C₁-C₂-alkyl or C₁-C₂-alkoxy or
A and D together with the atoms to which they are attached represent one of the groups AD-1 to AD- 10: 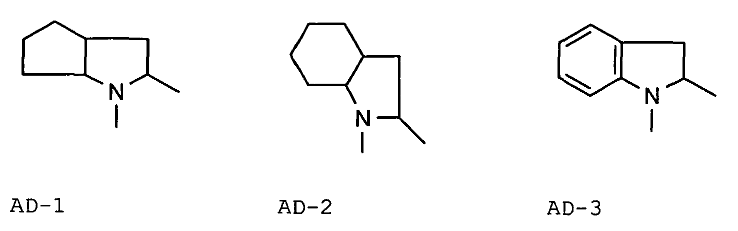 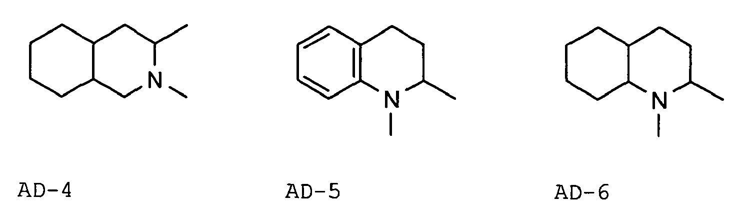 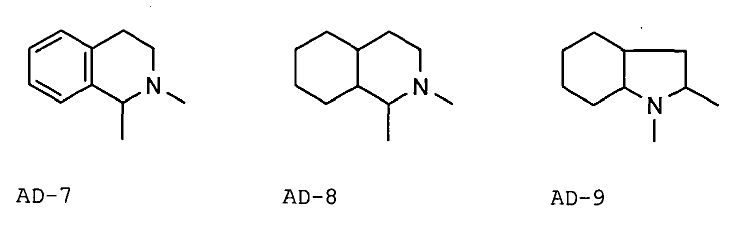 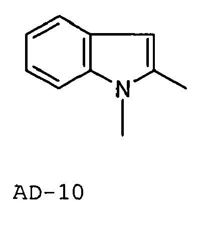
G represents hydrogen (a) or represents one of the groups 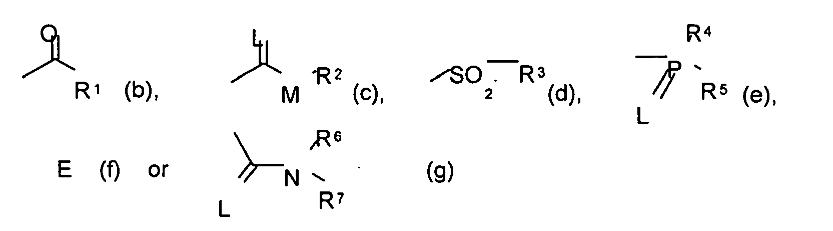 in which
E represents a metal ion equivalent or an ammonium ion,
L represents oxygen or sulphur and
M represents oxygen or sulphur,
R¹ represents C₁-C₈-alkyl, C₂-C₁₈-alkenyl, C₁-C₄-alk- oxy-C₁-C₂-alkyl or C₁-C₄-alkylthio-C₁-C₂-alkyl, each of which is optionally mono- to trisubstituted by fluorine or chlorine, or C₃-C₆- cycloalkyl which is optionally mono- or disubstituted by fluorine, chlorine, C₁-C₂-alkyl or C₁-C₂-alkoxy and in which optionally one or two not directly adjacent ring members are replaced by oxygen, represents phenyl which is optionally mono- or disubstituted by fluorine, chlorine, bromine, cyano, nitro, C₁-C₄-alkyl, C₁-C₄-alkoxy, C₁-C₂- haloalkyl or C₁-C₂-haloalkoxy,
R² represents C₁-C₈-alkyl, C₂-C₈-alkenyl or C₁-C₄- alkoxy-C₂-C₄-alkyl, each of which is optionally mono- to trisubstituted by fluorine, represents C₃-C₆-cycloalkyl which is optionally monosubstituted by C₁-C₂-alkyl or C₁-C₂-alkoxy or represents phenyl or benzyl, each of which is optionally mono- or disubstituted by fluorine, chlorine, bromine, cyano, nitro, C₁-C₄-alkyl, C₁- C₃-alkoxy, trifluoromethyl or trifluoromethoxy,
R³ represents C₁-C₆-alkyl which is optionally mono- to trisubstituted by fluorine or represents phenyl which is optionally monosubstituted by fluorine, chlorine, bromine, C₁-C₄-alkyl, C₁-C₄-alkoxy, trifluoromethyl, trifluoromethoxy, cyano or nitro,
R⁴ represents C₁-C₆-alkyl, C₁-C₆-alkoxy, C₁-C₆-alkyl- amino, di-(C₁-C₆-alkyl)amino, C₁-C₆-alkylthio, C₃- C₄-alkenylthio, C₃-C₆-cycloalkylthio or represents phenyl, phenoxy or phenylthio, each of which is optionally monosubstituted by fluorine, chlorine, bromine, nitro, cyano, C₁-C₃-alkoxy, C₁-C₃-halo- alkoxy, C₁-C₃-alkylthio, C₁-C₃-haloalkylthio, C₁- C₃-alkyl or trifluoromethyl,
R⁵ represents C₁-C₆-alkoxy or C₁-C₆-alkylthio,
R⁶ represents hydrogen, C₁-C₆-alkyl, C₃-C₆-cycloalkyl, C₁-C₆-alkoxy, C₃-C₆-alkenyl, C₁-C₆-alkoxy-C₁-C₄- alkyl, represents phenyl which is optionally monosubstituted by fluorine, chlorine, bromine, trifluoromethyl, C₁-C₄-alkyl or C₁-C₄-alkoxy, represents benzyl which is optionally monosubstituted by fluorine, chlorine, bromine, C₁-C₄-alkyl, trifluoromethyl or C₁-C₄-alkoxy,
R⁷ represents C₁-C₆-alkyl, C₃-C₆-alkenyl or C₁-C₆-alkoxy- C₁-C₄-alkyl,
R⁶ and R⁷ together represent an optionally methyl- or ethyl-substituted C₄-C₅-alkylene radical in which optionally one methylene group is replaced by oxygen or sulphur.

4. Compounds of the formula (I) according to Claim 1 in which
J represents trifluoromethoxy,
X represents hydrogen, fluorine, chlorine, bromine, methyl, ethyl, propyl, methoxy or ethoxy,
Y represents hydrogen, chlorine, bromine, methyl, ethyl or methoxy,
with the proviso, that at least one of the radicals J, X and Y is located in the 2-position of the phenyl radical and is not hydrogen, resulting in the phenyl substitution patterns below 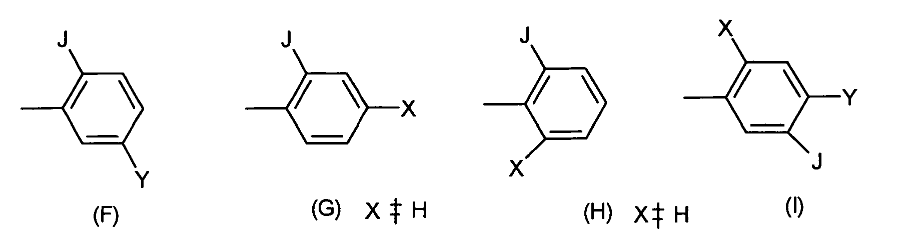 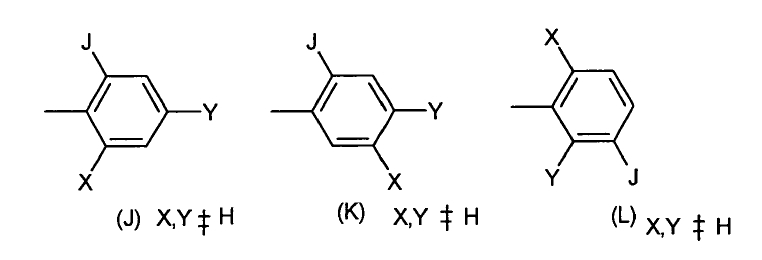 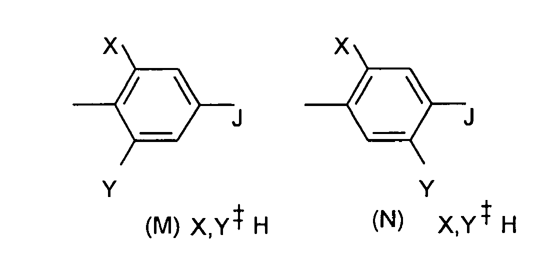
A represents hydrogen, represents C₁-C₄-alkyl or C₁- C₂-alkoxy-C₁-C₂-alkyl, each of which is optionally mono- to trisubstituted by fluorine, represents cyclopropyl, cyclopentyl or cyclohexyl, represents in each case optionally fluorine-, chlorine-, bromine-, methyl-, ethyl-, n-propyl-, isopropyl-, methoxy-, ethoxy-, trifluoromethyl-, trifluoromethoxy-, cyano- or nitro-substituted phenyl or benzyl,
B represents hydrogen, methyl or ethyl or
A, B and the carbon atom to which they are attached represent saturated C₅-C₆-cycloalkyl in which optionally one ring member is replaced by oxygen or sulphur and which is optionally monosubstituted by methyl, ethyl, propyl, isopropyl, methoxymethyl, ethoxymethyl, propoxymethyl, trifluoromethyl, methoxy, ethoxy, propoxy, butoxy, methoxyethyl, ethoxyethyl, methoxyethoxy, ethoxyethoxy, cyclopropylmethoxy, cyclopentylmethoxy or cyclohexylmethoxy, or
A, B and the carbon atom to which they are attached represent C₆-cycloalkyl which is optionally substituted by an alkylenedioxyl group which contains two not directly adjacent oxygen atoms, or
A, B and the carbon atom to which they are attached represent C₅-C₆-cycloalkyl or C₅-C₆-cycloalkenyl in which two substituents together with the carbon atoms to which they are attached represent C₂-C₄- alkanediyl or C₂-C₄-alkenediyl or butadienediyl,
D represents hydrogen, represents C₁-C₄-alkyl, C₃-C₄- alkenyl, C₁-C₄-alkoxy-C₂-C₃-alkyl, represents cyclopropyl, cyclopentyl or cyclohexyl, each of which is optionally mono- to trisubstituted by fluorine,
or
A and D together very particularly preferably represent C₃-C₅-alkanediyl which is optionally monosubstituted by methyl or methoxy and in which optionally one carbon atom is replaced by oxygen or sulphur, or represent the group AD-1,
G represents hydrogen (a) or represents one of the groups 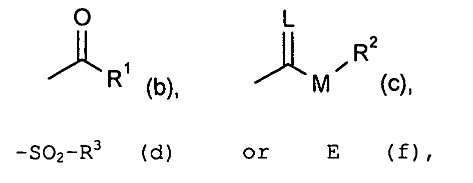 in which
L represents oxygen or sulphur,
M represents oxygen or sulphur and
E represents an ammonium ion,
R¹ represents C₁-C₆-alkyl, C₂-C₁₇-alkenyl, C₁-C₂-alk- oxy-C₁-alkyl, C₁-C₂-alkylthio-C₁-alkyl or represents cyclopropyl or cyclohexyl, each of which is optionally monosubstituted by fluorine, chlorine, methyl or methoxy, represents phenyl which is optionally monosubstituted by fluorine, chlorine, bromine, cyano, nitro, methyl, methoxy, trifluoromethyl or trifluoromethoxy,
R² represents C₁-C₈-alkyl, C₂-C₆-alkenyl or C₁-C₄- alkoxy-C₂-C₃-alkyl, phenyl or benzyl, each of which is optionally monosubstituted by fluorine,
R³ represents C₁-C₈-alkyl.

5. Compounds of the formula (I) according to Claim 1, in which
J represents trifluoromethoxy,
X represents hydrogen, chlorine, bromine, methyl, ethyl, or methoxy,
Y represents hydrogen, chlorine, bromine, methyl or methoxy,
with the proviso, that at least one of the radicals J, X and Y is located in the 2-position of the phenyl radical and is not hydrogen, resulting in the phenyl substitution patterns below 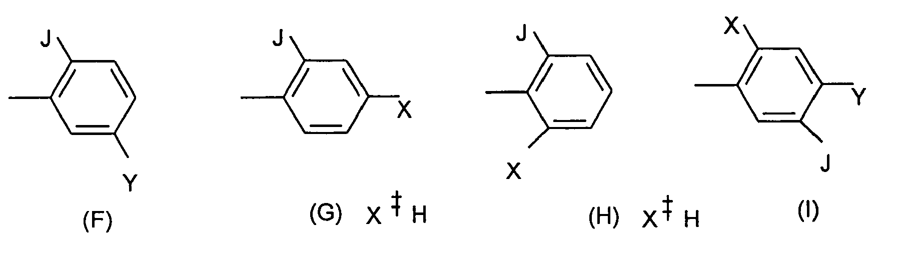 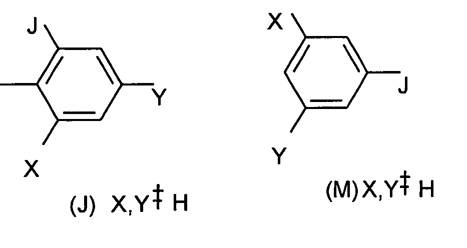
A represents C₁-C₄-alkyl or cyclopropyl,
B represents hydrogen or methyl,
A, B and the carbon atom to which they are attached represent saturated C₅-C₆-cycloalkyl in which optionally one ring member is replaced by oxygen and which is optionally monosubstituted by methoxymethyl, methoxy, ethoxy, propoxy or butoxy, or represent 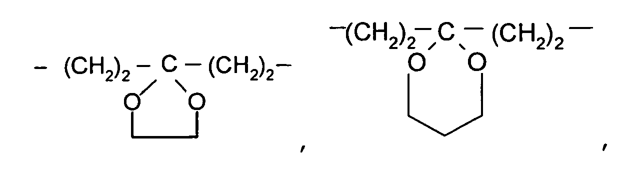
D represents hydrogen or cyclopropyl,
or
A and D together represent C₃-C₅-alkanediyl,
G represents hydrogen (a) or one of the groups 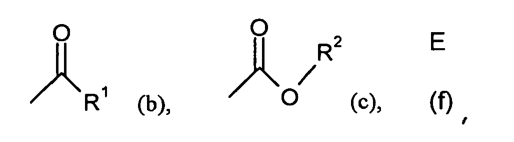
R¹ represents C₁-C₆-alkyl,
R² represents C₁-C₈-alkyl or benzyl.

6. Process for preparing compounds of the formula (I) according to Claim 1, **characterized in that**, to obtain (A) compounds of the formula (I-a) 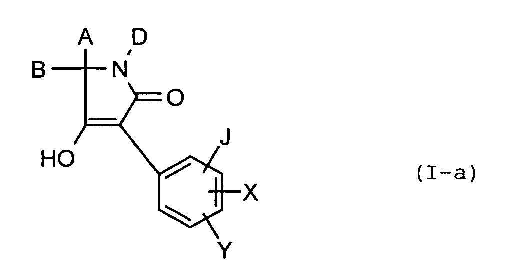 in which
A, B, D, J, X and Y have the meanings given above, N-acylamino acid esters of the formula (II) 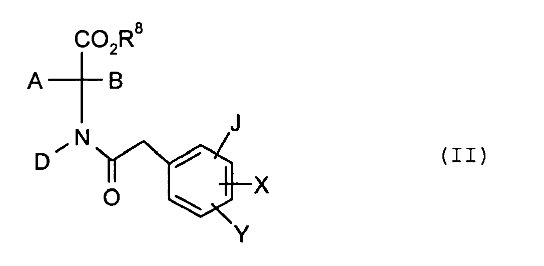 in which
A, B, D, J, X and Y have the meanings given above,
and
R⁸ represents alkyl, are condensed intramolecularly in the presence of a diluent and in the presence of a base,
(B) compounds of the formula (I-b) shown above in which A, B, D, J, R¹, X, and Y have the meanings given above, compounds of the formula (I-a) shown above in which A, B, D, J, X and Y have the meanings given above are in each case reacted
(α) with acid halides of the formula (III) 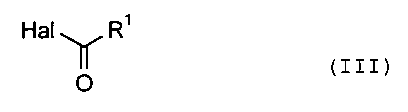
in which
R¹ has the meaning given above and
Hal represents halogen
or
(β) with carboxylic anhydrides of the formula (IV) R¹-CO-O-CO-R¹ (IV)
in which
R¹ has the meaning given above,
if appropriate in the presence of a diluent and if appropriate in the presence of an acid binder;
(C) compounds of the formula (I-c) shown above in which A, B, D, J, R², M, X and Y have the meanings given above and L represents oxygen, compounds of the formula (I-a) shown above in which A, B, D, J, X and Y have the meanings given above are in each case reacted
with chloroformic esters or chloroformic thioesters of the formula (V)
R²-M-CO-Cl (V)
in which
R² and M have the meanings given above,
if appropriate in the presence of a diluent and if appropriate in the presence of an acid binder;
(D) compounds of the formula (I-c) shown above in which A, B, D, J, R², M, X and Y have the meanings given above and L represents sulphur, compounds of the formula (I-a) shown above in which A, B, D, J, X and Y have the meanings given above are in each case reacted
with chlormonothioformic esters or chlordithioformic esters of the formula (VI) 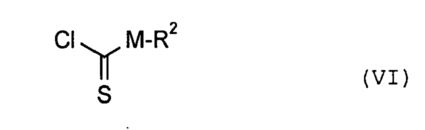 in which
M and R² have the meanings given above,
if appropriate in the presence of a diluent and if appropriate in the presence of an acid binder
and
(E) compounds of the formula (I-d) shown above in which A, B, D, J, R³, X and Y have the meanings given above, compounds of the formula (I-a) shown above in which A, B, D, J, X and Y have the meanings given above are in each case reacted
with sulphonyl chlorides of the formula (VII)
R³-SO₂-Cl (VII)
in which
R³ has the meaning given above,
if appropriate in the presence of a diluent and if appropriate in the presence of an acid binder,
(F) compounds of the formula (I-e) shown above in which A, B, D, J, L, R⁴, R⁵, X and Y have the meanings given above, compounds of the formula (I-a) shown above in which A, B, D, J, X and Y have the meanings given above are in each case reacted
with phosphorus compounds of the formula (VIII) 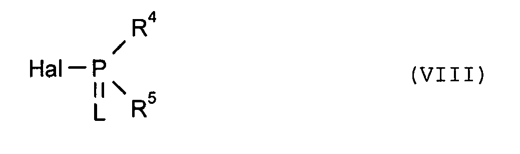 in which
L, R⁴ and R⁵ have the meanings given above and
Hal represents halogen,
if appropriate in the presence of a diluent and if appropriate in the presence of an acid binder,
(G) compounds of the formula (I-f) shown above in which A, B, D, E, J, X and Y have the meanings given above, compounds of the formula (I-a) in which A, B, D, J, X and Y have the meanings given above are in each case reacted
with metal compounds or amines of the formula (IX) and (X), respectively,
Me(OR¹⁰)ₜ (IX) 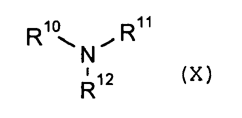
in which
Me represents a mono- or divalent metal, or represents an ammonium ion 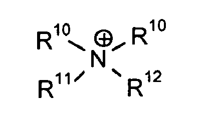
t represents the number 1 or 2 and
R¹⁰, R¹¹, R¹² independently of one another represent hydrogen or alkyl,
if appropriate in the presence of a diluent,
(H) compounds of the formula (I-g) shown above in which A, B, D, J, L, R⁶, R⁷, X and Y have the meanings given above, compounds of the formula (I-a) shown above in which A, B, D, J, X and Y have the meanings given above are in each case reacted
(α) with isocyanates or isothiocyanates of the formula (XI) R⁶-N=C=L (XI)
in which
R⁶ and L have the meanings given above,
if appropriate in the presence of a diluent and if appropriate in the presence of a catalyst or
(β) with carbamoyl chlorides or thiocarbamoyl chlorides of the formula 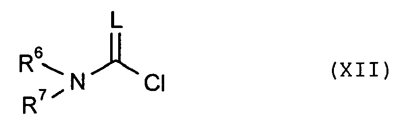
in which
L, R⁶ and R⁷ have the meanings given above,
if appropriate in the presence of a diluent and if appropriate in the presence of an acid binder.

7. Composition for controlling pests and/or unwanted vegetation, **characterized in that** it comprises at least one compound of the formula (I) according to Claim 1.

8. Method for controlling animal pests and/or unwanted vegetation, **characterized in that** compounds of the formula (I) according to Claim 1 are allowed to act on pests unwanted vegetation and/or their habitat.

9. Use of compounds of the formula (I) according to Claim 1 for controlling animal pests and/or unwanted vegetation.

10. Process for preparing compositions for controlling pests and/or unwanted vegetation, **characterized in that** compounds of the formula (I) according to Claim 1 are mixed with extenders and/or surfactants.

11. Use of compounds of the formula (I) according to Claim 1 for preparing compositions for controlling pests and/or unwanted vegetation.

12. Composition comprising an effective amount of an active compound combination comprising, as components,
(a') at least one trifluoromethoxyphenyl-substituted tetramic acid derivative of the formula (I) in which A, B, D, G, J, X and Y have the meaning given above
and
(b') at least one crop plant compatibility-improving
compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl - cf. also related compounds in EP-A-86750, EP-A-94349, EP-A-191736, EP-A-492366), 3-(2-chlorobenzyl)-1-(1-methyl-1-phenylethyl)urea (cumyluron), α-(cyanomethoximino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methylphenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichorophenyl)-4,5-dihydro-5-methyl-1H-pyrazole-3,5-dicarboxylate (mefenpyrdiethyl - cf. also related compounds in WO-A-91/07874), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate (cf. also related compounds in EP-A-269806 and EP-A-333131), ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate (cf. also related compounds in WO-A-91/08202), 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (cf. also related compounds in EP-A-582198), 4-carboxychroman-4-ylacetic acid (AC-304415, cf. EP-A-613618), 4-chlorophenoxyacetic acid, 3,3'-dimethyl-4-methoxybenzophenone, 1-bromo-4-chloromethylsulphonylbenzene, 1-[4-(N-2-methoxybenzoylsulphamoyl)phenyl]-3-methylurea (also known as N-(2-methoxybenzoyl)-4-[(methylaminocarbonyl)amino]benzenesulphonamide), 1-[4-(N-2-methoxybenzoylsulphamoyl)phenyl]-3,3-dimethylurea, 1-[4-(N-4,5-dimethylbenzoylsulphamoyl)phenyl]-3-methylurea, 1-[4-(N-naphthylsulphamoyl)phenyl]-3,3-dimethylurea, N-(2-methoxy-5-methylbenzoyl)-4-(cyclopropylaminocarbonyl)benzenesulphonamide,
and/or one of the following compounds, defined by general formulae
of the general formula (IIa) 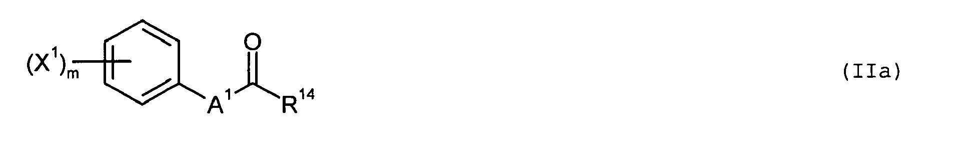 or of the general formula (IIb) 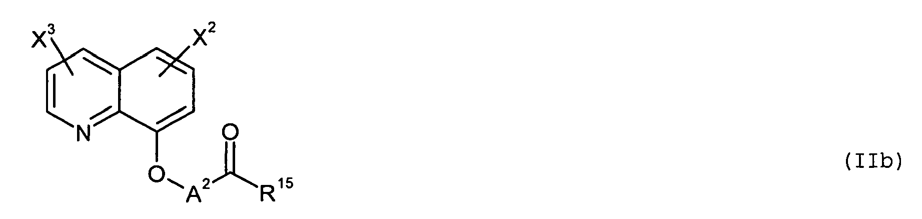 or of the formula (IIc) 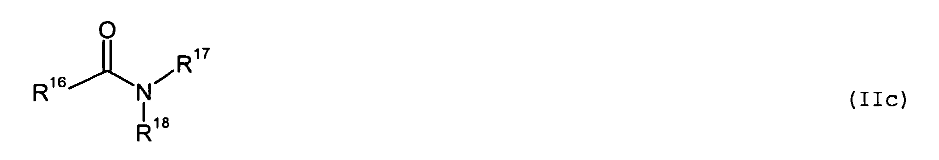 where
m represents a number 0, 1, 2, 3, 4 or 5,
A¹ represents one of the divalent heterocyclic groupings shown below 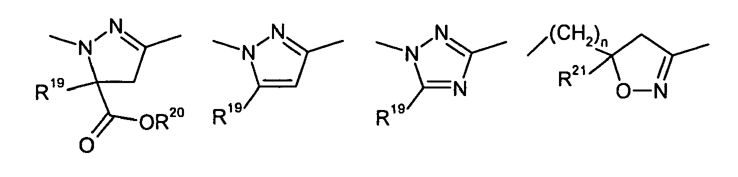
n represents a number 0, 1, 2, 3, 4 or 5,
A² represents optionally C₁-C₉-alkyl- and/or C₁-C₉- alkoxy-carbonyl- and/or C₁-C₉-alkenyloxycarbonyl- substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di(C₁-C₄- alkyl) amino,
R¹⁵ represents hydroxyl, mercapto, amino, C₁-C₇-alkoxy, C₁-C₆-alkenyloxy, C₁-C₆-alkenyloxy-C₁-C₆-alkoxy, C₁- C₆-alkylthio, C₁-C₆-alkylamino or di(C₁-C₄-alkyl)- amino,
R¹⁶ represents optionally fluorine-, chlorine- and/or bromine-substituted C₁-C₄-alkyl,
R¹⁷ represents hydrogen, in each case optionally fluorine-, chlorine- and/or bromine-substituted C₁- C₆-alkyl, C₂-C₆-alkenyl or C₂-C₆-alkynyl, C₁-C₄- alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁- C₆-alkyl, C₂-C₆-alkenyl or C₂-C₆-alkynyl, C₁-C₄- alkoxy-C₁-C₄-alkyl, dioxolanyl-C₁-C₄-alkyl, furyl,
R¹⁷ furyl-C₁-C₄-alkyl, thienyl, thiazolyl, piperidinyl, or optionally fluorine-, chlorine- and/or bromine- or C₁-C₄-alkyl-substituted phenyl, and R¹⁸ also together represent C₃-C₆-alkanediyl or C₂- C₅-oxaalkanediyl, each of which is optionally substituted by C₁-C₄-alkyl, phenyl, furyl, a fused benzene ring or by two substituents which, together with the C atom to which they are attached, form a 5- or 6-membered carbocycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy- substituted C₁-C₆-alkyl, C₃-C₆-cycloalkyl or tri-(C₁- C₉-alkyl)silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁- C₄-haloalkyl, C₁-C₄-alkoxy or C₁-C₄-haloalkoxy,
X² represents hydrogen, cyano, nitro, halogen, C₁-C₄- alkyl, C₁-C₄-haloalkyl, C₁-C₄-alkoxy or C₁-C₄- haloalkoxy,
X³ represents hydrogen, cyano, nitro, halogen, C₁-C₄- alkyl, C₁-C₄-haloalkyl, C₁-C₄-alkoxy or C₁-C₄- haloalkoxy,
and/or the following compounds, defined by general formulae
of the general formula (IId) 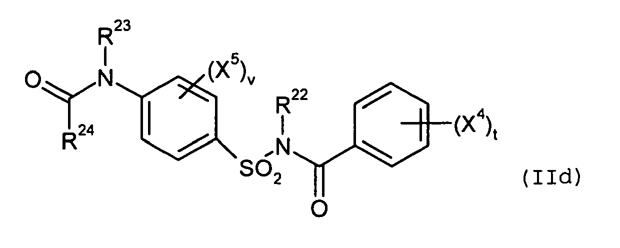 or of the general formula (IIe) 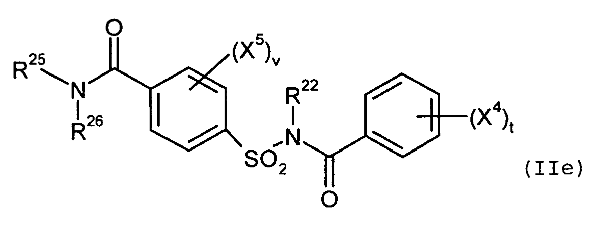 where
t represents a number 0, 1, 2, 3, 4 or 5,
v represents a number 0, 1, 2, 3, 4 or 5,
R²² represents hydrogen or C₁-C₄-alkyl,
R²³ represents hydrogen or C₁-C₄-alkyl,
R²⁴ represents hydrogen, in each case optionally cyano, halogen- or C₁-C₄-alkoxy-substituted C₁-C₆-alkyl, C₁- C₆-alkoxy, C₁-C₆-alkylthio, C₁-C₆-alkylamino or di(C₁-C₄-alkyl)amino, or in each case optionally cyano-, halogen- or C₁-C₄-alkyl-substituted C₃-C₆- cycloalkyl, C₃-C₆-cycloalkyloxy, C₃-C₆- cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano, halogen- or C₁-C₄-alkyl-substituted C₃-C₆- cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆- cycloalkyl, or optionally nitro-, cyano-, halogen-, C₁-C₄-alkyl-, C₁-C₄-haloalkyl-, C₁-C₄-alkoxy- or C₁- C₄-haloalkoxy-substituted phenyl, or together with R²⁵ represents in each case optionally C₁-C₄-alkyl- substituted C₂-C₆-alkanediyl or C₂-C₅-oxaalkanediyl,
X⁴ represents nitro, cyano, carboxyl, carbamoyl, formyl, sulphamoyl, hydroxyl, amino, halogen, C₁- C₄-alkyl, C₁-C₄-haloalkyl, C₁-C₄-alkoxy or C₁-C₄- haloalkoxy, and
X⁵ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy.

13. Composition according to Claim 12 in which the crop plant compatibility-improving compound is selected from the following group of compounds:
cloquintocet-mexyl, fenchlorazole-ethyl, isoxadifen-ethyl, mefenpyr-diethyl, furilazole, fenclorim, cumyluron, dymron or the compounds 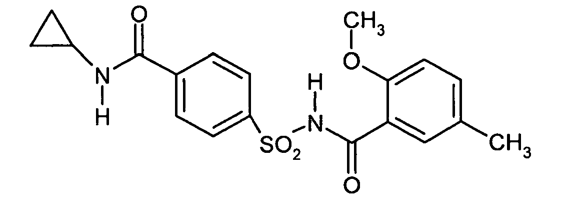 and

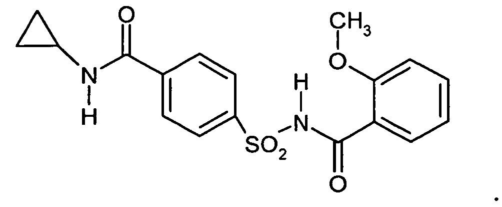

14. Composition according to one of Claims 12 and 13, in which the crop plant compatibility-improving compound is cloquintocet-mexyl.

15. Composition according to one of Claims 12 and 13, in which the crop plant compatibility-improving compound is mefenpyr-diethyl.

16. Method of controlling unwanted vegetation, **characterized in that** a composition according to Claim 12 is caused to act on the plants or their surroundings.

17. Use of a composition according to Claim 12 for controlling unwanted vegetation.

18. Method of controlling unwanted vegetation, **characterized in that** a compound of the formula (I) according to Claim 1 and the crop plant compatibility-improving compound according to Claim 12 are caused to act, separately in close temporal succession, on the plants or their surroundings.

19. Compounds of the formula (II) 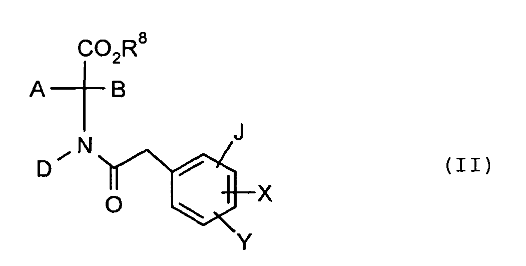 in which
A, B, D, J, X and Y and R⁸ have the meanings given above.

20. Compounds of the formula (XV) 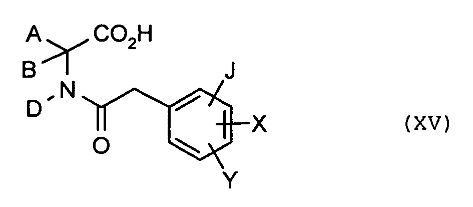 in which
A, B, D, J, X and Y have the meanings given above.

21. Compounds of the formula (XIV) 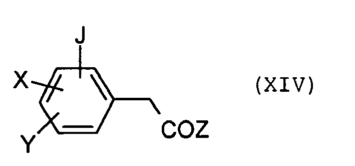 in which
Z represents a leaving group introduced by reagents for activating carboxylic acids, such as carbonyldiimidazole, carbonyldiimides (such as, for example, dicyclohexylcarbondiimide), phosphorylating reagents (such as, for example, POC13, BOP-C1), halogenating agents, such as, for example, thionyl chloride, oxalyl chloride, phosgene or chloroformic esters,
J, X and Y have the meanings given above.

22. Compounds of the formula (XIX) 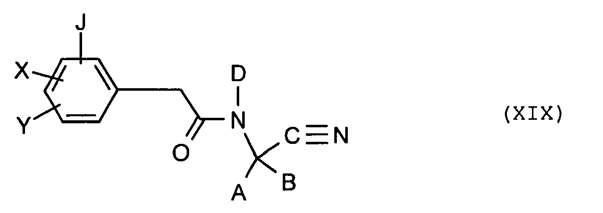 in which A, B, D, J, X and Y have the meanings given above.

23. Compounds of the formula (XVII) 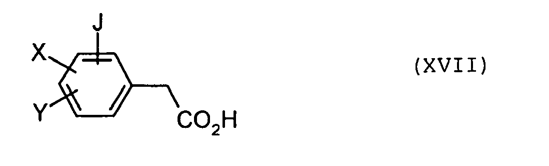 in which
J, X and Y have the meaning given in the table
| **J** | **X** | **Y** |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-Cl | 6-Cl |
| 4-OCF₃ | 2-OCH₃ | 6-Cl |
| 4-OCF₃ | 2-OCH₃ | 6-Br |
| 4-OCF₃ | 2-CH₃ | 6-CH₃ |
| 4-OCF₃ | 2-Cl | 6-CH₃ |
| 2-OCF₃ | 6-Cl | 4-Br |
| 2-OCF₃ | 6-CH₃ | 4-CH₃ |
| 2-OCF₃ | 6-OCH₃ | 4-Cl |
| 2-OCF₃ | 6-Cl | 4-CH₃ |
| 2-OCF₃ | 6-C₂H₅ | 4-Cl |

24. Compounds of the formula (XX) 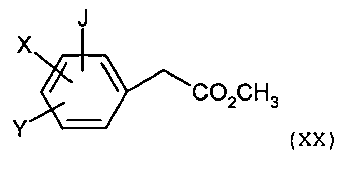 in which J, X and Y have the meanings given in the table
| **J** | **X** | **Y** |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-CH₃ | 6-CH₃ |
| 4-OCF₃ | 2-Br | 6-Cl |
| 4-OCF₃ | 2-Cl | 6-CH₃ |
| 2-OCF₃ | 6-Br | 4-Br |
| 2-OCF₃ | 6-CH₃ | 4-CH₃ |
| 2-OCF₃ | 6-OCH₃ | 4-Cl |
| 2-OCF₃ | 6-CH₃ | 4-Cl |
| 2-OCF₃ | 6-Cl | 4-CH₃ |
| 2-OCF₃ | 6-Cl | 4-Br |

25. Composition comprising
- at least one compound of the formula (I) according to Claim 1 or a composition according to Claim 12 and
- at least one salt of the formula (III') 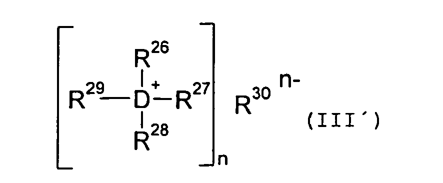 in which
D represents nitrogen or phosphorus,
R²⁶, R²⁷, R²⁸ and R²⁹ independently of one another represent hydrogen or in each case optionally substituted C₁-C₈-alkyl or mono- or polyunsaturated, optionally substituted C₁-C₈- alkylene, the substituents being selectable from halogen, nitro and cyano,
n represents 1, 2, 3 or 4,
R³⁰ represents an organic or inorganic anion.

26. Composition according to Claim 25, **characterized in that** it comprises at least one penetrant.

27. Method of increasing the action of pesticides and/or herbicides comprising an active compound of the formula (I) according to Claim 1 or a composition according to Claim 12, **characterized in that** the ready-to-use composition (spray liquor) is prepared using a salt of the formula (III') according to Claim 25.

28. Method according to Claim 27, **characterized in that** the spray liquor is prepared using a penetrant.

## Revendications

1. Composés de formule (I) 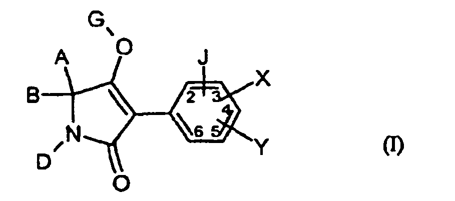 dans laquelle
J représente trifluorométhoxy,
X représente hydrogène, alkyle, halogène, halogénoalkyle, alcoxy ou halogénoalcoxy,
Y représente hydrogène, alkyle, alcoxy ou halogène,
à condition qu'au moins un des radicaux J, X ou Y se trouve en 2ème position du radical phényle et soit différent d'hydrogène,
A représente hydrogène ; alkyle, alcényle, alcoxyalkyle, alkylthioalkyle à chaque fois le cas échéant substitué par halogène ; ou cycloalkyle saturé ou insaturé, le cas échéant substitué, dans lequel le cas échéant au moins un atome de cycle est remplacé par un hétéroatome ; ou aryle, arylalkyle ou hétaryle à chaque fois le cas échéant substitué par halogène, alkyle, halogénoalkyle, alcoxy, halogénoalcoxy, cyano ou nitro,
B représente hydrogène, alkyle ou alcoxyalkyle, ou
A et B représentent, ensemble avec l'atome de carbone auquel ils sont liés, un cycle saturé ou insaturé, non substitué ou substitué, contenant le cas échéant au moins un hétéroatome,
D représente hydrogène ou un radical le cas échéant substitué de la série alkyle, alcényle, alcynyle, alcoxyalkyle, cycloalkyle saturé ou insaturé, dans lequel le cas échéant un ou plusieurs éléments de cycle sont remplacés par des hétéroatomes, arylalkyle, aryle, hétarylalkyle ou hétaryle ou
A et D représentent, ensemble avec les atomes auxquels ils sont liés, un cycle saturé ou insaturé et contenant le cas échéant au moins un hétéroatome, non substitué ou substitué dans la partie A, D,
G représente hydrogène (a) ou un des groupes 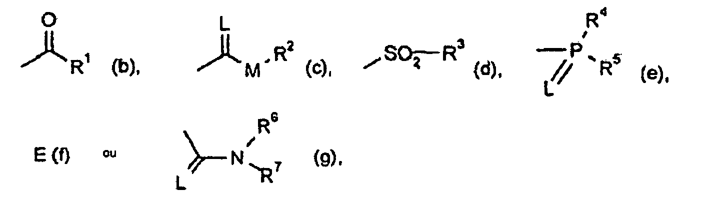
où
E représente un équivalent d'ion métallique ou un ion d'ammonium,
L représente oxygène ou soufre,
M représente oxygène ou soufre,
R¹ représente alkyle, alcényle, alcoxyalkyle, alkylthioalkyle, polyalcoxyalkyle à chaque fois le cas échéant substitué par halogène ; ou cycloalkyle le cas échéant substitué par halogène, alkyle ou alcoxy, qui peut être interrompu par au moins un hétéroatome ; phényle, phénylalkyle, hétaryle, phénoxyalkyle ou hétaryloxyalkyle à chaque fois le cas échéant substitué,
R² représente alkyle, alcényle, alcoxyalkyle, polyalcoxyalkyle à chaque fois le cas échéant substitué par halogène ; ou cycloalkyle, phényle ou benzyle à chaque fois le cas échéant substitué,
R³, R⁹ et R⁵ représentent, indépendamment l'un de l'autre, alkyle, alcoxy, alkylamino, dialkylamino, alkylthio, alcénylthio, cycloalkylthio à chaque fois le cas échéant substitué par halogène ; ou phényle, benzyle, phénoxy ou phénylthio à chaque fois le cas échéant substitué,
R⁶ et R⁷ représentent, indépendamment l'un de l'autre, hydrogène ; alkyle, cycloalkyle, alcényle, alcoxy, alcoxyalkyle à chaque fois le cas échéant substitué par halogène ; phényle le cas échéant substitué ; benzyle le cas échéant substitué ; ou ensemble avec l'atome de N auquel ils sont liés, un cycle le cas échéant interrompu par oxygène ou soufre.

2. Composés de formule (I) selon la revendication 1, où
J représente trifluorométhoxy,
X représente hydrogène, halogène, C₁-C₆-alkyle, C₁- C₄-halogénoalkyle, C₁-C₆-alcoxy ou C₁-C₄- halogénoalcoxy,
Y représente hydrogène, C₁-C₆-alkyle, C₁-C₆-alcoxy ou halogène,
à condition qu'au moins un des radicaux J, X ou Y se trouve en 2ème position du radical phényle et soit différent d'hydrogène, les schémas de substitution de phényle suivants étant obtenus 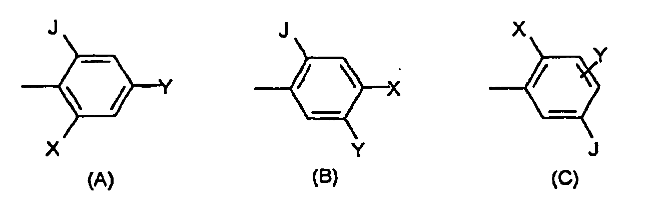 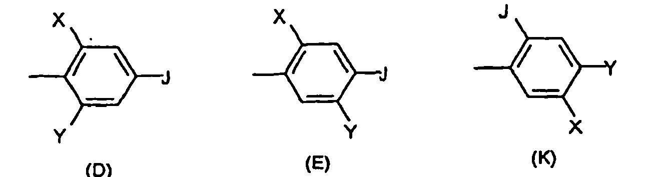 où, dans les schémas de substitution de phényle (C), (D), (E) et (K), X et Y sont simultanément différents d'hydrogène,
A représente hydrogène ; ou C₁-C₁₂-alkyle, C₃-C₈- alcényle, C₁-C₁₀-alcoxy-C₁-C₈-alkyle, C₁-C₁₀- alkylthio-C₁-C₆-alkyle à chaque fois le cas échéant substitué par halogène ; C₃-C₈-cycloalkyle le cas échéant substitué par halogène, C₁-C₆-alkyle ou C₁- C₆-alcoxy, dans lequel le cas échéant un ou deux éléments de cycle non directement adjacents sont remplacés par oxygène et/ou soufre ; ou phényle, naphtyle, hétaryle comprenant 5 à 6 atomes de cycle, phényl-C₁-C₆-alkyle ou naphtyl-C₁-C₆-alkyle à chaque fois le cas échéant substitué par halogène, C₁-C₆-alkyle, C₁-C₆-halogénoalkyle, C₁-C₆- alcoxy, C₁-C₆-halogénoalcoxy, cyano ou nitro,
B représente hydrogène, C₁-C₁₂-alkyle ou C₁-C₈-alcoxy- C₁-C₆-alkyle ou
A, B et l'atome de carbone auquel ils sont liés représentent C₃-C₁₀-cycloalkyle saturé ou C₅-C₁₀- cycloalkyle insaturé, dans lesquels le cas échéant un élément de cycle est remplacé par oxygène ou soufre et qui sont le cas échéant monosubstitués ou disubstitués par C₁-C₈-alkyle, C₁-C₆-alcoxy-C₁- C₄-alkyle, C₁-C₆-alcoxy-C₁-C₉-alcoxy, C₃-C₆- cycloalkyl-C₁-C₂-alcoxy, C₃-C₁₀-cycloalkyle, C₁-C₈- halogénoalkyle, C₁-C₈-alcoxy, C₁-C₈-alkylthio, halogène ou phényle ou
A, B et l'atome de carbone auquel ils sont liés représentent C₃-C₆-cycloalkyle, qui est substitué par un groupe alkylènediyle contenant le cas échéant un ou deux atomes d'oxygène et/ou de soufre non directement adjacents, le cas échéant substitué par C₁-C₄-alkyle ou par un groupe
A, B alkylènedioxyle ou par un groupe alkylènedithioyle, qui forme avec l'atome de carbone auquel il est lié un autre cycle de cinq à huit chaînons ou et l'atome de carbone auquel ils sont liés représentent C₃-C₈-cycloalkyle ou C₅-C₈- cycloalcényle, dans lesquels deux substituants, ensemble avec les atomes de carbone auxquels ils sont liés, représentent C₂-C₆-alcanediyle, C₂-C₆- alcènediyle ou C₄-C₆-alcanediènediyle à chaque fois le cas échéant substitué par C₁-C₆-alkyle, C₁-C₆- alcoxy ou halogène, dans lesquels le cas échéant un groupe méthylène est remplacé par oxygène ou soufre,
D représente hydrogène ; C₁-C₁₂-alkyle, C₃-C₈- alcényle, C₃-C₈-alcynyle, C₁-C₁₀-alcoxy-C₂-C₈-alkyle à chaque fois le cas échéant substitué par halogène ; C₃-C₈-cycloalkyle le cas échéant substitué par halogène, C₁-C₄-alkyle, C₁-C₄-alcoxy ou C₁-C₄-halogénoalkyle, dans lequel le cas échéant un élément de cycle est remplacé par oxygène ou soufre ; ou phényle, hétaryle comprenant 5 ou 6 atomes de cycle, phényl-C₁-C₆-alkyle ou hétaryle- C₁-C₆-alkyle comprenant 5 ou 6 atomes de cycle à chaque fois le cas échéant substitué par halogène, C₁-C₆-alkyle, C₁-C₆-halogénoalkyle, C₁-C₆-alcoxy, C₁-C₆-halogénoalcoxy, cyano ou nitro ; ou
A et D représentent ensemble C₃-C₆-alcanediyle ou C₃- C₆-alcènediyle à chaque fois le cas échéant substitué, dans lesquels le cas échéant un groupe méthylène est remplacé par un groupe carbonyle, oxygène ou soufre et où les substituants qui entrent en considération sont : halogène ; hydroxy ; mercapto ; ou C₁-C₁₀-alkyle, C₁-C₆-alcoxy, C₁-C₆-alkylthio, C₃-C₇-cycloalkyle, phényle ou benzyloxy à chaque fois le cas échéant substitué par halogène ; ou un autre groupe C₃-C₆- alcanediyle, C₃-C₆-alcènediyle ou butadiényle, qui est le cas échéant substitué par C₁-C₆-alkyle ou dans lequel le cas échéant deux substituants adjacents, avec les atomes de carbone auxquels ils sont liés, forment un autre cycle saturé ou insaturé, comprenant 5 ou 6 atomes de cycle, qui peut contenir oxygène ou soufre ou dans lequel le cas échéant un des groupes suivants 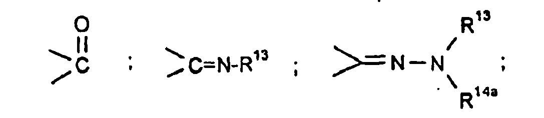 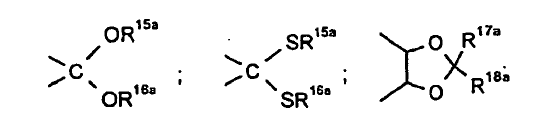 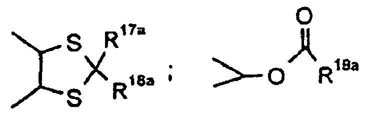
ou 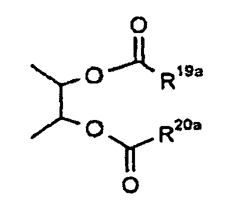 est contenu,
G représente hydrogène (a) ou un des groupes 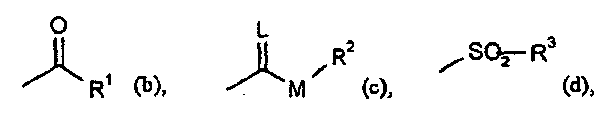 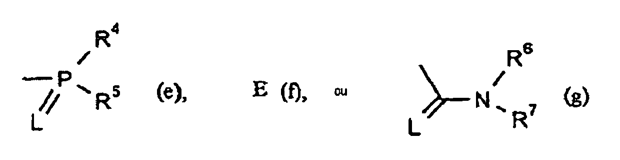
dans lesquels
E représente un équivalent d'ion métallique ou un ion d'ammonium,
L représente oxygène ou soufre, et
M représente oxygène ou soufre,
R¹ représente C₁-C₂₀-alkyle, C₂-C₂₀-alcényle, C₁-C₈- alcoxy-C₁-C₈-alkyle, C₁-C₈-alkylthio-C₁-C₈-alkyle, poly-C₁-C₈-alcoxy-C₁-C₈-alkyle à chaque fois le cas échéant substitué par halogène ; ou C₃-C₈- cycloalkyle le cas échéant substitué par halogène, C₁-C₆-alkyle ou C₁-C₆-alcoxy, dans lequel le cas échéant un ou plusieurs éléments de cycle non directement adjacents sont remplacés par oxygène et/ou soufre, représente phényle, le cas échéant substitué par halogène, cyano, nitro, C₁-C₆-alkyle, C₁-C₆-alcoxy, C₁-C₆-halogénoalkyle, C₁-C₆-halogénoalcoxy, C₁-C₆- alkylthio ou C₁-C₆-alkylsulfonyle, représente phényl-C₁-C₆-alkyle le cas échéant substitué par halogène, nitro, cyano, C₁-C₆-alkyle, C₁-C₆-alcoxy, C₁-C₆-halogénoalkyle ou C₁-C₆- halogénoalcoxy, représente hétaryle de 5 ou 6 chaînons, le cas échéant substitué par halogène ou C₁-C₆-alkyle, représente phénoxy-C₁-C₆-alkyle le cas échéant substitué par halogène ou C₁-C₆-alkyle, ou représente hétaryloxy-C₁-C₆-alkyle de 5 ou 6 chaînons, le cas échéant substitué par halogène, amino ou C₁-C₆-alkyle,
R² représente C₁-C₂₀-alkyle, C₂-C₂₀-alcényle, C₁-C₈- alcoxy-C₂-C₈-alkyle, poly-C₁-C₈-alcoxy-C₂-C₈-alkyle à chaque fois le cas échéant substitué par halogène, représente C₃-C₈-cycloalkyle le cas échéant substitué par halogène, C₁-C₆-alkyle ou C₁-C₆- alcoxy ou représente phényle ou benzyle à chaque fois le cas échéant substitué par halogène, cyano, nitro, C₁- C₆-alkyle, C₁-C₆-alcoxy, C₁-C₆-halogénoalkyle ou C₁- C₆-halogénoalcoxy,
R³ représente C₁-C₈-alkyle le cas échéant substitué par halogène ; ou phényle ou benzyle à chaque fois le cas échéant substitué par halogène, C₁-C₆- alkyle, C₁-C₆-alcoxy, C₁-C₄-halogénoalkyle, C₁-C₄- halogénoalcoxy, cyano ou nitro,
R⁴ et R⁵ représentent, indépendamment l'un de l'autre, C₁-C₈-alkyle, C₁-C₈-alcoxy, C₁-C₈-alkylamino, di- (C₁-C₈-alkyl)amino, C₁-C₈-alkylthio, C₂-C₈- alcénylthio, C₃-C₇-cycloalkylthio le cas échéant à chaque fois substitué par halogène ; ou phényle, phénoxy ou phénylthio le cas échéant à chaque fois substitué par halogène, nitro, cyano, C₁-C₄-alcoxy, C₁-C₄-halogénoalcoxy, C₁-C₄-alkylthio, C₁-C₄- halogénoalkylthio, C₁-C₄-alkyle ou C₁-C₄- halogénoalkyle,
R⁶ et R⁷ représentent, indépendamment l'un de l'autre, hydrogène ; C₁-C₈-alkyle, C₃-C₈-cycloalkyle, C₁-C₈- alcoxy, C₃-C₈-alcényle, C₁-C₈-alcoxy-C₁-C₈-alkyle à chaque fois le cas échéant substitué par halogène ; phényle le cas échéant substitué par halogène, C₁-C₈-halogénoalkyle, C₁-C₈-alkyle ou C₁- C₈-alcoxy ; benzyle le cas échéant substitué par halogène, C₁-C₈-alkyle, C₁-C₈-halogénoalkyle ou C₁- C₈-alcoxy ; ou ensemble un radical C₃-C₆-alkylène le cas échéant substitué par C₁-C₄-alkyle, dans lequel le cas échéant un atome de carbone est remplacé par oxygène ou soufre,
R¹³ représente hydrogène ; C₁-C₈-alkyle ou C₁-C₈-alcoxy à chaque fois le cas échant substitué par halogène ; C₃-C₈-cycloalkyle le cas échéant substitué par halogène, C₁-C₄-alkyle ou C₁-C₄- alcoxy, dans lequel le cas échéant un groupe méthylène est remplacé par oxygène ou soufre ; ou phényle, phényl-C₁-C₄-alkyle ou phényl-C₁-C₄-alcoxy
à chaque fois le cas échéant substitué par halogène, C₁-C₆-alkyle, C₁-C₆-alcoxy, C₁-C₄- halogénoalkyle, C₁-C₄-halogénoalcoxy, nitro ou cyano,
R^{14a} représente hydrogène ou C₁-C₈-alkyle, ou
R¹³ et R^{14a} représentent ensemble C₄-C₆-alcanediyle,
R^{15a} et R^{16a} sont identiques ou différents et représentent C₁-C₆-alkyle ou
R^{15a} et R^{16a} représentent ensemble un radical C₂-C₄- alcanediyle, qui est le cas échéant substitué par C₁-C₆-alkyle, C₁-C₆-halogénoalkyle ou par phényle, le cas échéant substitué par halogène, C₁-C₆- alkyle, C₁-C₄-halogénoalkyle, C₁-C₆-alcoxy, C₁-C₄- halogénoalcoxy, nitro ou cyano,
R^{17a} et R^{18a} représentent, indépendamment l'un de l'autre, hydrogène ; C₁-C₈-alkyle le cas échéant substitué par halogène ; ou phényle le cas échéant substitué par halogène, C₁-C₆-alkyle, C₁-C₆-alcoxy, C₁-C₄-halogénoalkyle, C₁-C₄-halogénoalcoxy, nitro ou cyano, ou
R^{17a} et R^{18a} représentent, ensemble avec l'atome de carbone auquel ils sont liés, un groupe carbonyle ; ou C₅-C₇-cycloalkyle le cas échéant substitué par halogène, C₁-C₄-alkyle ou C₁-C₄- alcoxy, dans lequel le cas échéant un groupe méthylène est remplacé par oxygène ou soufre,
R^{19a} et R^{20a} représentent, indépendamment l'un de l'autre, C₁-C₁₀-alkyle, C₂-C₁₀-alcényle, C₁-C₁₀- alcoxy, C₁-C₁₀-alkylamino, C₃-C₁₀-alcénylamino, di- (C₁-C₁₀-alkyl)amino ou di-(C₃-C₃-alcényl)amino.

3. Composés de formule (I) selon la revendication 1, où
J représente trifluorométhoxy,
X représente hydrogène, fluor, chlore, brome, C₁-C₄- alkyle, trifluorométhyle ou C₁-C₄-alcoxy,
Y représente hydrogène, fluor, chlore, brome, C₁-C₄- alkyle ou C₁-C₄-alcoxy,
à condition qu'au moins un des radicaux J, X ou Y se trouve en 2ème position du radical phényle et soit différent d'hydrogène, les schémas de substitution de phényle suivants étant obtenus 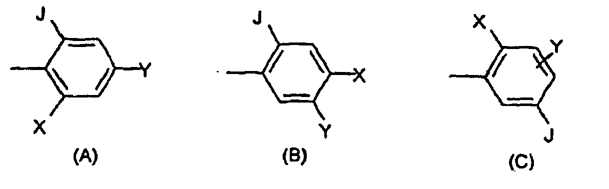 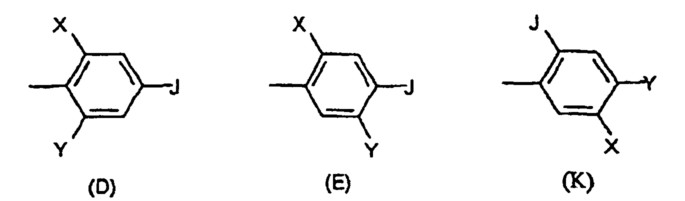 où, dans les schémas de substitution de phényle (C), (D), (E) et (K), X et Y sont simultanément différents d'hydrogène,
A représente hydrogène ; C₁-C₆-alkyle, C₁-C₄-alcoxy- C₁-C₂-alkyle à chaque fois le cas échéant monosubstitué à trisubstitué par fluor ou chlore ; C₃-C₆-cycloalkyle le cas échéant monosubstitué à disubstitué par C₁-C₂-alkyle ou C₁-C₂-alcoxy, qui peut le cas échéant être interrompu par un atome d'oxygène ; phényle ou benzyle à chaque fois le cas échéant monosubstitué à disubstitué par fluor, chlore, brome, C₁-C₄-alkyle, C₁-C₂-halogénoalkyle, C₁-C₄-alcoxy, C₁-C₂-halogénoalcoxy, cyano ou nitro,
B représente hydrogène, C₁-C₄-alkyle ou C₁-C₂-alcoxy- C₁-C₂-alkyle ou
A, B et l'atome de carbone auquel ils sont liés représentent C₅-C₇-cycloalkyle saturé ou insaturé, dans lequel le cas échéant un élément de cycle est remplacé par oxygène ou soufre et qui est le cas échéant monosubstitué à disubstitué par C₁-C₆- alkyle, C₁-C₄-alcoxy-C₁-C₂-alkyle, C₁-C₄-alcoxy-C₁- C₂-alcoxy, C₃-C₆-cycloalkylméthoxy, trifluorométhyle ou C₁-C₆-alcoxy, ou
A, B et l'atome de carbone auquel ils sont liés représentent C₅-C₆-cycloalkyle, qui est substitué par un groupe alkylènediyle contenant le cas échéant un ou deux atomes d'oxygène et/ou de soufre non directement adjacents, le cas échéant substitué par méthyle ou éthyle ou par un groupe alkylènedioxyle ou par un groupe alkylènedithioyle, qui forme avec l'atome de carbone auquel il est lié un autre cycle de cinq ou six chaînons ou
A, B et l'atome de carbone auquel ils sont liés représentent C₃-C₆-cycloalkyle ou C₅-C₆- cycloalcényle, dans lesquels deux substituants, ensemble avec les atomes de carbone auxquels ils sont liés, représentent C₂-C₉-alcanediyle, C₂-C₄- alcènediyle ou butadiènediyle à chaque fois le cas échéant substitué par C₁-C₂-alkyle ou C₁-C₂-alcoxy,
D représente hydrogène ; C₁-C₆-alkyle, C₃-C₆- alcényle, C₁-C₄-alcoxy-C₂-C₃-alkyle à chaque fois le cas échéant monosubstitué à trisubstitué par fluor ; C₃-C₆-cycloalkyle le cas échéant monosubstitué à disubstitué par C₁-C₄-alkyle, C₁- C₄-alcoxy ou C₁-C₂-halogénoalkyle, dans lequel le cas échéant un groupe méthylène est remplacé par oxygène, ou
A et D représentent ensemble C₃-C₅-alcanediyle le cas échéant monosubstitué à disubstitué, dans lequel un groupe méthylène peut être remplacé par un groupe carbonyle, oxygène ou soufre et où les substituants qui entrent en considération sont C₁- C₂-alkyle ou C₁-C₂-alcoxy,
A et D représentent, ensemble avec les atomes auxquels ils sont liés, un des groupes AD-1 à AD-10 : 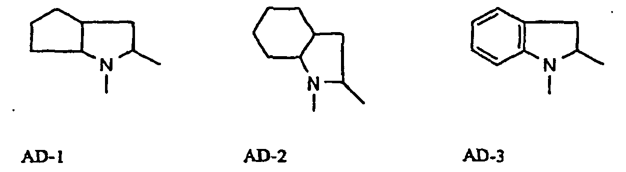 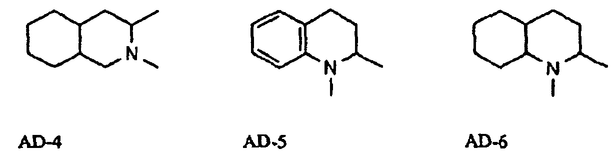 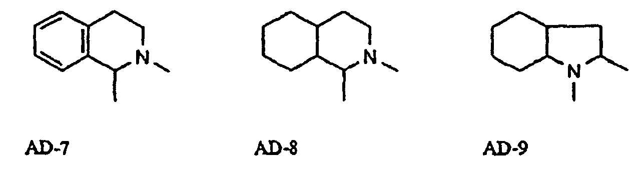 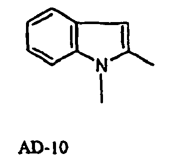
G représente hydrogène (a) ou un des groupes 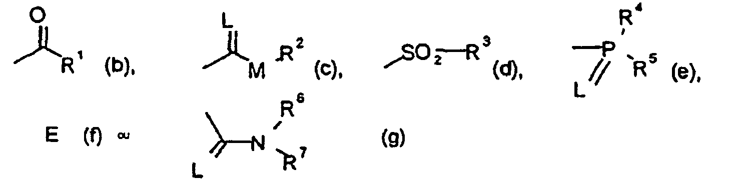
dans lesquels
E représente un équivalent d'ion métallique ou un ion d'ammonium,
L représente oxygène ou soufre, et
M représente oxygène ou soufre,
R¹ représente C₁-C₈-alkyle, C₂-C₁₈-alcényle, C₁-C₄- alcoxy-C₁-C₂-alkyle, C₁-C₄-alkylthio-C₁-C₂-alkyle à chaque fois le cas échéant monosubstitué à trisubstitué par fluor ou chlore ; ou C₃-C₆- cycloalkyle le cas échéant monosubstitué à disubstitué par fluor, chlore, C₁-C₂-alkyle ou C₁- C₂-alcoxy, dans lequel le cas échéant un ou deux éléments de cycle non directement adjacents sont remplacés par oxygène, représente phényle le cas échéant monosubstitué à disubstitué par fluor, chlore, brome, cyano, nitro, C₁-C₄-alkyle, C₁-C₄-alcoxy, C₁-C₂- halogénoalkyle ou C₁-C₂-halogénoalcoxy,
R² représente C₁-C₈-alkyle, C₂-C₈-alcényle ou C₁-C₄- alcoxy-C₂-C₄-alkyle à chaque fois le cas échéant monosubstitué à trisubstitué par fluor, représente C₃-C₆-cycloalkyle le cas échéant monosubstitué par C₁-C₂-alkyle ou C₁-C₂-alcoxy ou représente phényle ou benzyle à chaque fois le cas échéant monosubstitué à disubstitué par fluor, chlore, brome, cyano, nitro, C₁-C₄-alkyle, C₁-C₃- alcoxy, trifluorométhyle ou trifluorométhoxy,
R³ représente C₁-C₆-alkyle le cas échéant monosubstitué à trisubstitué par fluor ; ou phényle le cas échéant monosubstitué par fluor, chlore, brome, C₁-C₄-alkyle, C₁-C₄-alcoxy, trifluorométhyle, trifluorométhoxy, cyano ou nitro,
R⁴ représente C₁-C₆-alkyle, C₁-C₆-alcoxy, C₁-C₆- alkylamino, di-(C₁-C₆-alkyl)amino, C₁-C₆-alkylthio, C₃-C₄-alcénylthio, C₃-C₆-cycloalkylthio ; ou phényle, phénoxy ou phénylthio à chaque fois le cas échéant monosubstitué par fluor, chlore, brome, nitro, cyano, C₁-C₃-alcoxy, C₁-C₃- halogénoalcoxy, C₁-C₃-alkylthio, C₁-C₃- halogénoalkylthio, C₁-C₃-alkyle ou trifluorométhyle,
R⁵ représente C₁-C₆-alcoxy ou C₁-C₆-alkylthio,
R⁶ représente hydrogène ; C₁-C₆-alkyle, C₃-C₆- cycloalkyle, C₁-C₆-alcoxy, C₃-C₆-alcényle, C₁-C₆- alcoxy-C₁-C₄-alkyle ; phényle le cas échéant monosubstitué par fluor, chlore, brome, trifluorométhyle, C₁-C₄-alkyle ou C₁-C₄-alcoxy ; benzyle le cas échéant monosubstitué par fluor, chlore, brome, C₁-C₄-alkyle, trifluorométhyle ou C₁-C₄-alcoxy,
R⁷ représente C₁-C₆-alkyle, C₃-C₆-alcényle ou C₁-C₆- alcoxy-C₁-C₄-alkyle,
R⁶ et R⁷ représentent ensemble un radical C₄-C₅-alkylène le cas échéant substitué par méthyle ou éthyle, dans lequel le cas échéant un groupe méthylène est remplacé par oxygène ou soufre.

4. Composés de formule (I) selon la revendication 1, où
J représente trifluorométhoxy,
X représente hydrogène, fluor, chlore, brome, méthyle, éthyle, propyle, méthoxy ou éthoxy,
Y représente hydrogène, chlore, brome, méthyle, éthyle ou méthoxy, à condition qu'au moins un des radicaux J, X ou Y se trouve en 2ème position du radical phényle et soit différent d'hydrogène, les schémas de substitution de phényle suivants étant obtenus 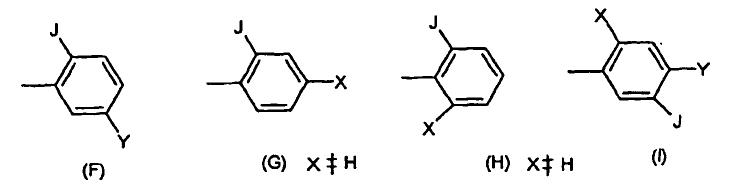 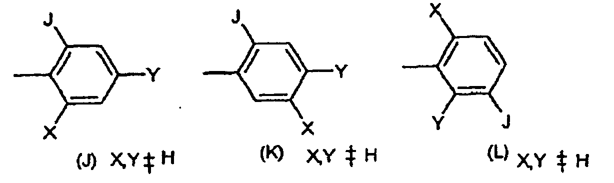 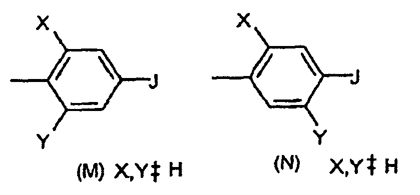
A représente hydrogène ; C₁-C₄-alkyle ou C₁-C₂- alcoxy-C₁-C₂-alkyle à chaque fois le cas échéant monosubstitué à trisubstitué par fluor ; cyclopropyle, cyclopentyle ou cyclohexyle ; phényle ou benzyle à chaque fois le cas échéant substitué par fluor, chlore, brome, méthyle, éthyle, n-propyle, iso-propyle, méthoxy, éthoxy, trifluorométhyle, trifluorométhoxy, cyano ou nitro,
B représente hydrogène, méthyle ou éthyle ou
A, B et l'atome de carbone auquel ils sont liés représentent C₅-C₆-cycloalkyle saturé, dans lequel le cas échéant un élément de cycle est remplacé par oxygène ou soufre et qui est le cas échéant monosubstitué par méthyle, éthyle, propyle, isopropyle, méthoxyméthyle, éthoxyméthyle, propoxyméthyle, trifluorométhyle, méthoxy, éthoxy, propoxy, butoxy, méthoxyéthyle, éthoxyéthyle, méthoxyéthoxy, éthoxyéthoxy, cyclopropylméthoxy, cyclopentylméthoxy ou cyclohexylméthoxy, ou
A, B et l'atome de carbone auquel ils sont liés, représentent C₆-cycloalkyle, qui est le cas échéant substitué par un groupe alkylènedioxyle contenant deux atomes d'oxygène non directement adjacents, ou
A, B et l'atome de carbone auquel ils sont liés représentent C₅-C₆-cycloalkyle ou C₅-C₆- cycloalcényle, dans lesquels deux substituants, ensemble avec les atomes de carbone auxquels ils sont liés, représentent C₂-C₄-alcanediyle, C₂-C₄- alcènediyle ou butadiènediyle,
D représente hydrogène ; C₁-C₄-alkyle, C₃-C₄- alcényle, C₁-C₄-alcoxy-C₂-C₃-alkyle à chaque fois le cas échéant monosubstitué à trisubstitué par fluor ; cyclopropyle, cyclopentyle ou cyclohexyle, ou
A et D représentent ensemble C₃-C₅-alcanediyle le cas échéant monosubstitué par méthyle ou méthoxy, dans lequel le cas échéant un atome de carbone est remplacé par oxygène ou soufre ; ou le groupe AD- 1,
G représente hydrogène (a) ou un des groupes 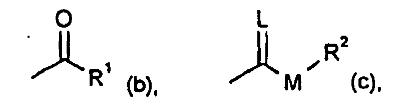
-SO₂-R³ (d) ou E (f),
dans lesquels
L représente oxygène ou soufre,
M représente oxygène ou soufre, et
E représente un ion d'ammonium,
R¹ représente C₁-C₆-alkyle, C₂-C₁₇-alcényle, C₁-C₂- alcoxy-C₁-alkyle, C₁-C₂-alkylthio-C₁-alkyle ; ou cyclopropyle ou cyclohexyle à chaque fois le cas échéant monosubstitué par fluor, chlore, méthyle ou méthoxy, représente phényle le cas échéant monosubstitué par fluor, chlore, brome, cyano, nitro, méthyle, méthoxy, trifluorométhyle ou trifluorométhoxy,
R² représente C₁-C₈-alkyle, C₂-C₆-alcényle ou C₁-C₄- alcoxy-C₂-C₃-alkyle à chaque fois le cas échéant monosubstitué à trisubstitué par fluor ; phényle ou benzyle,
R³ représente C₁-C₈-alkyle.

5. Composés de formule (I) selon la revendication 1, où
J représente trifluorométhoxy,
X représente hydrogène, chlore, brome, méthyle, éthyle ou méthoxy,
Y représente hydrogène, chlore, brome, méthyle ou méthoxy,
à condition qu'au moins un des radicaux J, X ou Y se trouve en 2ème position du radical phényle et soit différent d'hydrogène, les schémas de substitution de phényle suivants étant obtenus 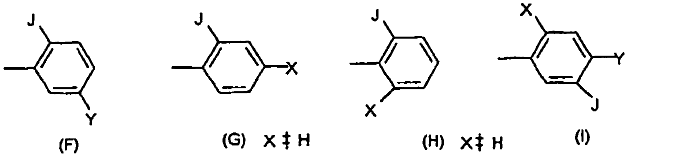 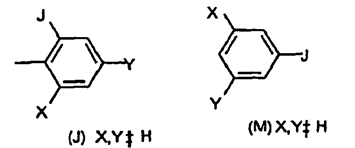
A représente C₁-C₄-alkyle ou cyclopropyle,
B représente hydrogène ou méthyle,
A, B et l'atome de carbone auquel ils sont liés représentent C₅-C₆-cycloalkyle saturé, dans lequel le cas échéant un élément de cycle est remplacé par oxygène et qui est le cas échéant monosubstitué par méthoxyméthyle, méthoxy, éthoxy, propoxy ou butoxy ; ou 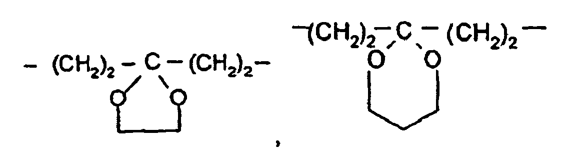
D représente hydrogène ou cyclopropyle, ou
A et D représentent ensemble C₃-C₅-alcanediyle,
G représente hydrogène (a) ou un des groupes 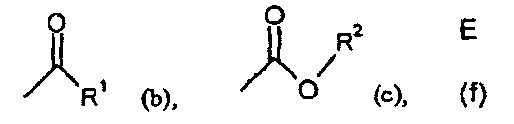
R¹ représente C₁-C₆-alkyle,
R² représente C₁-C₈-alkyle ou benzyle.

6. Procédé pour la préparation de composés de formule (I) selon la revendication 1, **caractérisé en ce que**, pour obtenir
(A) des composés de formule (I-a), 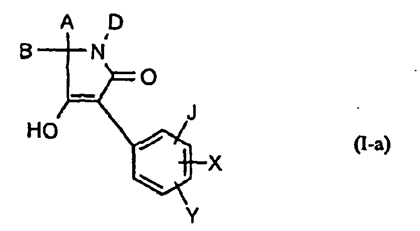 dans laquelle
A, B, D, J, X et Y présentent les significations indiquées ci-dessus, on condense de manière intramoléculaire des esters de N- acylaminoacides de formule (II) 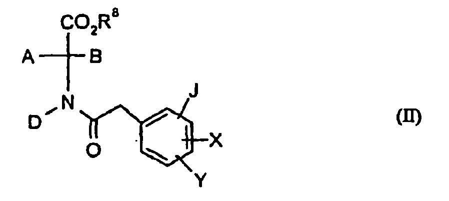
dans laquelle
A, B, D, J, X et Y présentent les significations indiquées ci-dessus, et
R⁸ représente alkyle, en présence d'un diluant et en présence d'une base,
(B) des composés de la formule (I-b) représentée ci- dessus, dans laquelle A, B, D, J, R¹, X et Y présentent les significations indiquées ci-dessus, on transforme des composés de la formule (I-a) représentée ci-dessus, dans laquelle A, B, D, J, X et Y présentent les significations indiquées ci- dessus, à chaque fois (α) avec des halogénures d'acide de formule (III) 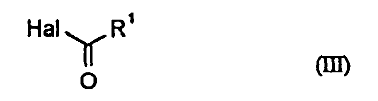
dans laquelle
R¹ présente la signification indiquée ci- dessus et
Hal représente halogène, ou
(β) avec des anhydrides d'acide carboxylique de formule (IV)
R¹-CO-O-CO-R¹ (IV)
dans laquelle
R¹ présente la signification indiquée ci- dessus,
le cas échéant en présence d'un diluant et le cas échéant en présence d'un liant d'acide ;
(C) des composés de la formule (I-c) représentée ci- dessus, dans laquelle A, B, D, J, R², M, X et Y présentent les significations indiquées ci-dessus et L représente oxygène, on transforme des composés de la formule (I-a) représentée ci- dessus, dans laquelle A, B, D, J, X et Y présentent les significations indiquées ci-dessus, à chaque fois
avec des esters de l'acide chloroformique ou des thioesters de l'acide chloroformique de formule (V)
R²-M-CO-C₁ (V)
dans laquelle
R² et M présentent les significations
indiquées ci-dessus,
le cas échéant en présence d'un diluant et le
cas échéant en présence d'un liant d'acide ;
(D) des composés de la formule (I-c) représentée ci- dessus, dans laquelle A, B, D, J, R², M, X et Y présentent les significations indiquées ci-dessus et L représente soufre, on transforme des composés de la formule (I-a) représentée ci-dessus, dans laquelle A, B, D, J, X et Y présentent les significations indiquées ci-dessus, à chaque fois avec des esters de l'acide chloromonothioformique ou des esters de l'acide chlorodithioformique de formule (VI) 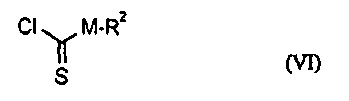
dans laquelle
M et R² présentent les significations indiquées ci-dessus,
le cas échéant en présence d'un diluant et le cas échéant en présence d'un liant d'acide et
(E) des composés de la formule (I-d) représentée ci- dessus, dans laquelle A, B, D, J, R³, X et Y présentent les significations indiquées ci-dessus, on transforme des composés de la formule (I-a) représentée ci-dessus, dans laquelle A, B, D, J, X et Y présentent les significations indiquées ci- dessus, à chaque fois avec des chlorures d'acide sulfonique de formule (VII)
R³-SO₂-Cl (VII)
dans laquelle
R³ présente la signification indiquée ci- dessus,
le cas échéant en présence d'un diluant et le cas échéant en présence d'un liant d'acide,
(F) des composés de la formule (I-e) représentée ci- dessus, dans laquelle A, B, D, J, L, R⁴, R⁵, X et Y présentent les significations indiquées ci-dessus, on transforme des composés de la formule (I-a) représentée ci-dessus, dans laquelle A, B, D, J, X et Y présentent les significations indiquées ci- dessus, à chaque fois avec des composés phosphorés de formule (VIII) 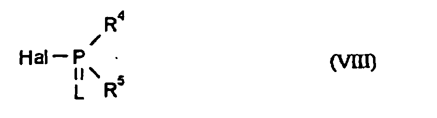
dans laquelle
L, R⁴ et R⁵ présentent les significations indiquées ci-dessus, et
Hal représente halogène,
le cas échéant en présence d'un diluant et le cas échéant en présence d'un liant d'acide,
(G) des composés de la formule (I-f) représentée ci- dessus, dans laquelle A, B, D, E, J, X et Y présentent les significations indiquées ci-dessus, on transforme des composés de la formule (I-a) représentée ci-dessus, dans laquelle A, B, D, J, X et Y présentent les significations indiquées ci- dessus, à chaque fois avec des composés métalliques ou des amines des formules (IX) ou (X) 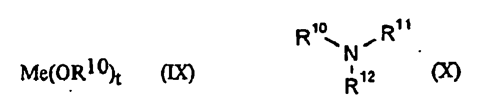
dans lesquelles
Me représente un métal monovalent ou divalent ou un ion d'ammonium 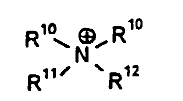
t représente le nombre 1 ou 2 et
R¹⁰, R¹¹, R¹² représentent, indépendamment l'un de l'autre, hydrogène ou méthyle,
le cas échéant en présence d'un diluant,
(H) des composés de la formule (I-g) représentée ci- dessus, dans laquelle A, B, D, J, L, R⁶, R⁷, X et Y présentent les significations indiquées ci-dessus, on transforme des composés de la formule (I-a) représentée ci-dessus, dans laquelle A, B, D, J, X et Y présentent les significations indiquées ci- dessus, à chaque fois
(α) avec des isocyanates ou des isothiocyanates de formule (XI)
R⁶-N=C=L (XI)
dans laquelle
R⁶ et L présentent les significations
indiquées ci-dessus,
le cas échéant en présence d'un diluant et le cas échéant en présence d'un catalyseur ou
(β) avec des chlorures d'acide carbamique ou des chlorures d'acide thiocarbamique de formule (XII) 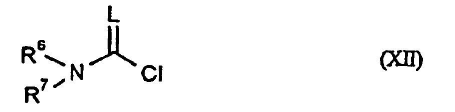 dans laquelle
L, R6 et R⁷ présentent les significations
indiquées ci-dessus, le cas échéant en présence d'un diluant et le cas échéant en présence d'un liant d'acide.

7. Agent pour lutter contre des organismes nuisibles et/ou une croissance non souhaitée de végétaux, **caractérisé par** une teneur en au moins un composé de formule (I) selon la revendication 1.

8. Procédé pour lutter contre des organismes nuisibles animaux et/ou une couverture végétale non souhaitée, **caractérisé en ce qu'**on laisse agir des composés de formule (I) selon la revendication 1 sur des organismes nuisibles, des végétaux non souhaités et/ou leur espace de vie.

9. Utilisation de composés de formule (I) selon la revendication 1 pour lutter contre des organismes nuisibles animaux et/ou une couverture végétale non souhaitée.

10. Procédé pour la préparation d'agents destinés à lutter contre des organismes nuisibles et/ou une couverture végétale non souhaitée, **caractérisé en ce qu'**on mélange des composés de formule (I) selon la revendication 1 avec des agents d'allongement et/ou des substances tensioactives.

11. Utilisation de composés de formule (I) selon la revendication 1 pour la préparation d'agents destinés à lutter contre des organismes nuisibles et/ou une croissance non souhaitée des végétaux.

12. Agent contenant une teneur active en une combinaison de substances actives comprenant, comme composants
(a') au moins un dérivé de l'acide tétramique substitué par trifluorométhoxyphényle de formule (I), dans laquelle A, B, D, G, J, X et Y présentent la signification indiquée ci-dessus et
(b') au moins un composé améliorant la tolérance des plantes de culture du groupe suivant de composés :
4-dichloroacétyl-1-oxa-4-azaspiro[4,5]-décane (AD-67, MON-4660), 1-dichloroacétylhexahydro-3,3,8a-triméthylpyrrolo[1,2-a]-pyrimidin-6(2H)-one (Dicyclonon, BAS-145138), 4-dichloroacétyl-3,4-dihydro-3-méthyl-2H-1,4-benzoxazine (Benoxacor), ester 1-méthylhexylique de l'acide 5-chloroquinoléin-8-oxyacétique (Cloquintocet-mexyl - Cf. également les composés apparentés dans les documents EP A 86 750, EP A 94 349, EP A 191 736, EP A 492 366), 3-(2-chlorobenzyl)-1-(1-méthyl-1-phényléthyl)-urée (Cumyluron), α-(cyanométhoximino)-phénylacétonitrile (Cyometrinil), acide 2,4-dichlorophénoxyacétique (2,4-D), acide 4-(2,4-dichlorophénoxy)-butyrique (2,4-DB), 1-(1-méthyl-1-phényléthyl)-3-(4-méthylphényl)-urée (Daimuron, Dymron), acide 3,6-dichloro-2-méthoxybenzoïque (Dicamba), ester S-1-méthyl-1-phényléthylique de l'acide pipéridine-1-thiocarboxylique (Dimepiperate), 2,2-dichloro-N-(2-oxo-2-(2-propénylamino)-éthyl)-N-(2-propényl)-acétamide (DKA-24), 2,2-dichloro-N,N-di-2-propénylacétamide (Dichlormid), 4,6-dichloro-2-phénylpyrimidine (Fenclorim), ester éthylique de l'acide 1-(2,4-dichlorophényl)-5-trichlorométhyl-1H-1,2,4-triazole-3-carboxylique (Fenchlorazol-ethyl - Cf. également les composés apparentés dans les documents EP A 174 562 et EP A 346 620), ester phénylméthylique de l'acide 2-chloro-4-trifluorométhylthiazole-5-carboxylique (Flurazole), 4-chloro-N-(1,3-dioxolan-2-ylméthoxy)-α-trifluoroacétophénonoxime (Fluxofenim), 3-dichloroacétyl-5-(2-furannyl)-2,2-diméthyloxazolidine (Furilazole, MON-13900), 4,5-dihydro-5,5-diphényl-3-isoxazole-carboxylate d'éthyle (Isoxadifen-ethyl - Cf. également les composés apparentés dans le document WO A 95/07 897), 3,6-dichloro-2-méthoxybenzoate de 1-(éthoxycarbonyl)-éthyle (Lactidichlor), acide (4-chloro-o-toluyloxy)-acétique (MCPA), acide 2-(4-chloro-o-toluyloxy)-propionique (Mecoprop), 1-(2,4-dichlorophényl)-4,5-dihydro-5-méthyl-1H-pyrazole-3,5-dicarboxylate de diéthyle (Mefenpyrdi-ethyl - Cf. également les composés apparentés dans le document WO A 91/07 874), 2-dichlorométhyl-2-méthyl-1,3-dioxolane (MG-191), 2-propényl-1-oxa-4-azaspiro[4,5]décane-4-carbodithioate (MG-838), anhydride de l'acide 1,8-naphtalénique, α-(1,3-dioxolan-2-ylméthoximino)-phénylacétonitrile (Oxabetrinil), 2,2-dichloro-N-(1,3-dioxolan-2-ylméthyl)-N-(2-propényl)-acétamide (PPG-1292), 3-dichloroacétyl-2,2-diméthyloxazolidine (R-28725), 3-dichloroacétyl-2,2,5-triméthyloxazolidine (R-29148), acide 4-(4-chloro-o-toluyl)-butyrique, acide 4-(4-chlorophénoxy)-butyrique, acide diphénylméthoxyacétique, ester méthylique de l'acide diphénylméthoxyacétique, ester éthylique de l'acide diphénylméthoxyacétique, ester méthylique de l'acide 1-(2-chlorophényl)-5-phényl-1H-pyrazole-3-carboxylique, ester éthylique de l'acide 1-(2,4-dichlorophényl)-5-méthyl-1H-pyrazole-3-carboxylique, ester éthylique de l'acide 1-(2,4-dichlorophényl)-5-isopropyl-1H-pyrazole-3-carboxylique, ester éthylique de l'acide 1-(2,4-dichlorophényl)-5-(1,1-diméthyléthyl)-1H-pyrazole-3-carboxylique, ester éthylique de l'acide 1-(2,4-dichlorophényl)-5-phényl-1H-pyrazole-3-carboxylique (Cf. également les composés apparentés dans les documents EP A 269 806 et EP A 333 131), ester éthylique de l'acide 5-(2,4-dichlorobenzyl)-2-isoxazoline-3-carboxylique, ester éthylique de l'acide 5-phényl-2-isoxazoline-3-carboxylique, ester éthylique de l'acide 5-(4-fluorophényl)-5-phényl-2-isoxazoline-3-carboxylique (Cf. également les composés apparentés dans le document WO A 91/08 202), ester 1,3-diméthylbut-1-ylique de l'acide 5-chloroquinoléin-8-oxyacétique, ester 4-allyloxybutylique de l'acide 5-chloroquinoléin-8-oxyacétique, ester 1-allyloxyprop-2-ylique de l'acide 5-chloroquinoléin-8-oxyacétique, ester méthylique de l'acide 5-chloroquinoxalin-8-oxyacétique, ester éthylique de l'acide 5-chloroquinoléin-8-oxyacétique, ester allylique de l'acide 5-chloroquinoxalin-8-oxyacétique, ester 2-oxoprop-1-ylique de l'acide 5-chloroquinoléin-8-oxyacétique, ester diéthylique de l'acide 5-chloroquinoléin-8-oxymalonique, ester diallylique de l'acide 5-chloroquinoxalin-8-oxymalonique, ester diéthylique de l'acide 5-chloroquinoléin-8-oxymalonique (Cf. également les composés apparentés dans le document EP A 582 198), acide 4-carboxychroman-4-ylacétique (AC-304415, Cf. EP A 613 618), acide 4-chlorophénoxyacétique, 3,3'-diméthyl-4-méthoxybenzophénone, 1-bromo-4-chlorométhylsulfonylbenzène, 1-[4-(N-2-méthoxybenzoylsulfamoyl)-phényl]-3-méthylurée (alias N-(2-méthoxybenzoyl)-4-[(méthylaminocarbonyl)-amino]-benzènesulfonamide), 1-[4-(N-2-méthoxybenzoylsulfamoyl)-phényl]-3,3-diméthylurée, 1-[4-(N-4,5-diméthylbenzoylsulfamoyl)-phényl]-3-méthylurée, 1-[4-(N-naphtylsulfamoyl)-phényl]-3,3-diméthylurée, N-(2-méthoxy-5-méthylbenzoyl)-4-(cyclopropylaminocarbonyl)-benzènesulfonamide,
et/ou un des composés suivants, définis par les formules générales
de formule générale (IIa) 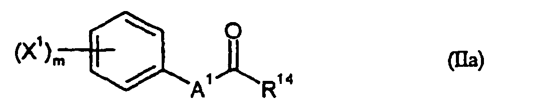
ou de formule générale (IIb) 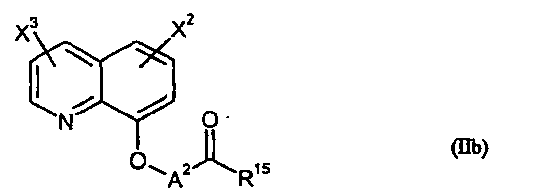
ou de formule (IIc) 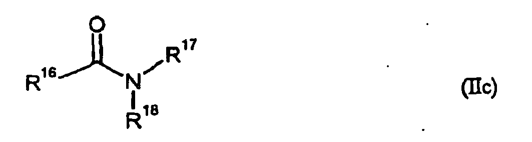 où
m représente le nombre 0, 1, 2, 3, 4 ou 5,
A¹ représente un des groupes hétérocycliques divalents esquissés ci-après, 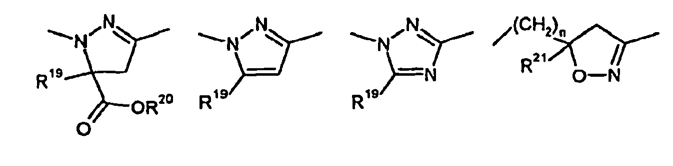
n représente le nombre 0, 1, 2, 3, 4 ou 5,
A² représente alcanediyle, comprenant 1 ou 2 atomes de carbone, le cas échéant substitué par C₁-C₄- alkyle et/ou C₁-C₄-alcoxycarbonyle et/ou C₁-C₄- alcényloxycarbonyle,
R¹⁴ représente hydroxy, mercapto, amino, C₁-C₆-alcoxy, C₁-C₆-alkylthio, C₁-C₆-alkylamino ou di-(C₁-C₄- alkyl)-amino,
R¹⁵ représente hydroxy, mercapto, amino, C₁-C₇-alcoxy, C₁-C₆-alcényloxy, C₁-C₆-alcényloxy-C₁-C₆-alcoxy, C₁- C₆-alkylthio, C₁-C₆-alkylamino ou di-(C₁-C₄-alkyl)- amino,
R¹⁶ représente C₁-C₄-alkyle le cas échéant substitué par fluor, chlore et/ou brome,
R¹⁷ représente hydrogène ; C₁-C₆-alkyle, C₂-C₆-alcényle ou C₂-C₆-alcynyle à chaque fois le cas échéant substitué par fluor, chlore et/ou brome ; C₁-C₄- alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou C₁-C₄-alkyle,
R¹⁸ représente hydrogène ; C₁-C₆-alkyle, C₂-C₆-alcényle ou C₂-C₆-alcynyle à chaque fois le cas échéant substitué par fluor, chlore et/ou brome ; C₁-C₄- alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou C₁-C₄-alkyle,
R¹⁷ et R¹⁸ représentent également, ensemble, C₃-C₆- alcanediyle ou C₂-C₅-oxaalcanediyle à chaque fois le cas échéant substitué par C₁-C₄-alkyle, phényle, furyle, un cycle benzène annelé ou par deux substituants, qui forment, ensemble avec l'atome de carbone auquel ils sont liés, un carbocyclique de 5 ou 6 chaînons,
R¹⁹ représente hydrogène ; cyano ; halogène ; ou C₁-C₄- alkyle, C₃-C₆-cycloalkyle ou phényle à chaque fois le cas échéant substitué par fluor, chlore et/ou brome,
R²⁰ représente hydrogène ; C₁-C₆-alkyle, C₃-C₆- cycloalkyle ou tri-(C₁-C₄-alkyl)-silyle le cas échéant substitué par hydroxy, cyano, halogène ou C₁-C₄-alcoxy,
R²¹ représente hydrogène ; cyano ; halogène ; ou C₁-C₄- alkyle, C₃-C₆-cycloalkyle ou phényle à chaque fois le cas échéant substitué par fluor, chlore et/ou brome,
X¹ représente nitro, cyano, halogène, C₁-C₄-alkyle, C₁-C₄-halogénoalkyle, C₁-C₄-alcoxy ou C₁-C₄- halogénoalcoxy,
X² représente hydrogène, cyano, nitro, halogène, C₁- C₄-alkyle, C₁-C₄-halogénoalkyle, C₁-C₄-alcoxy ou C₁- C₄-halogénoalcoxy,
X³ représente hydrogène, cyano, nitro, halogène, C₁- C₄-alkyle, C₁-C₄-halogénoalkyle, C₁-C₄-alcoxy ou C₁- C₄-halogénoalcoxy,
et/ou les composés suivants définis par les formules générales de formule générale (IId) 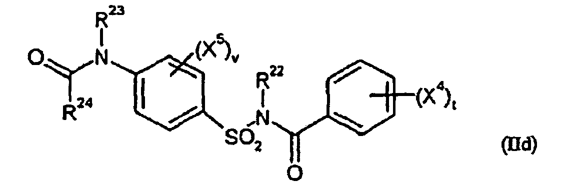
ou de formule générale (IIe) 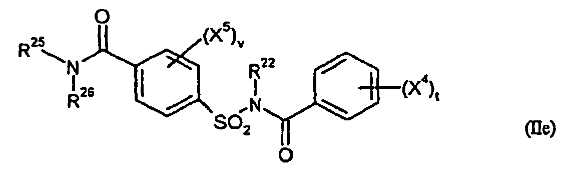
où
t représente le nombre 0, 1, 2, 3, 4 ou 5,
v représente le nombre 0, 1, 2, 3, 4 ou 5,
R²² représente hydrogène ou C₁-C₄-alkyle,
R²³ représente hydrogène ou C₁-C₄-alkyle,
R²⁴ représente hydrogène ; C₁-C₆-alkyle, C₁-C₆-alcoxy, C₁-C₆-alkylthio, C₁-C₆-alkylamino ou di-(C₁-C₄- alkyl)-amino à chaque fois le cas échéant substitué par cyano, halogène ou C₁-C₄-alcoxy ; ou C₃-C₆-cycloalkyle, C₃-C₆-cycloalkyloxy, C₃-C₆- cycloalkylthio ou C₃-C₆-cycloalkylamino à chaque fois substitué par cyano, halogène ou C₁-C₄-alkyle,
R²⁵ représente hydrogène ; C₁-C₆-alkyle le cas échéant substitué par cyano, hydroxy, halogène ou C₁-C₄- alcoxy ; C₃-C₆-alcényle ou C₃-C₆-alcynyle à chaque fois le cas échéant substitué par cyano ou halogène ; ou C₃-C₆-cycloalkyle le cas échéant substitué par cyano, halogène ou C₁-C₄-alkyle,
R²⁶ représente hydrogène ; C₁-C₆-alkyle le cas échéant substitué par cyano, hydroxy, halogène ou C₁-C₄- alcoxy ; C₃-C₆-alcényle ou C₃-C₆-alcynyle à chaque fois le cas échéant substitué par cyano ou halogène ; C₃-C₆-cycloalkyle le cas échéant substitué par cyano, halogène ou C₁-C₄-alkyle ; ou phényle le cas échéant substitué par nitro, cyano, halogène, C₁-C₄-alkyle, C₁-C₄-halogénoalkyle, C₁-C₄- alcoxy ou C₁-C₄-halogénoalcoxy ; ou, ensemble avec R²⁵, C₂-C₆-alcanediyle ou C₂-C₅-oxaalcanediyle à chaque fois le cas échéant substitué par C₁-C₄- alkyle,
X⁴ représente nitro, cyano, carboxy, carbamoyle, formyle, sulfamoyle, hydroxy, amino, halogène, C₁- C₄-alkyle, C₁-C₄-halogénoalkyle, C₁-C₄-alcoxy ou C₁- C₄-halogénoalcoxy, et
X⁵ représente nitro, cyano, carboxy, carbamoyle, formyle, sulfamoyle, hydroxy, amino, halogène, C₁- C₄-alkyle, C₁-C₄-halogénoalkyle, C₁-C₄-alcoxy ou C₁- C₄-halogénoalcoxy.

13. Agent selon la revendication 12, dans lequel le composé améliorant la tolérance des plantes de culture est choisi dans le groupe suivant de composés : Cloquintocet-mexyl, Fenchlorazole-ethyl, Isoxadifen-ethyl, Mefenpyr-diethyl, Furilazole, Fenclorim, Cumyluron, Dymron ou les composés 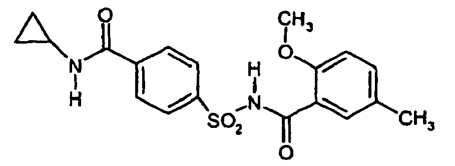 et

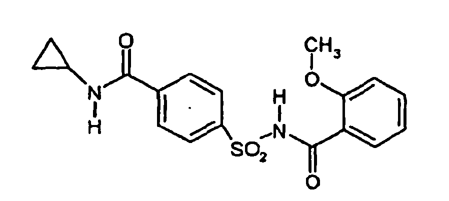

14. Agent selon l'une quelconque des revendications 12 ou 13, dans lequel le composé améliorant la tolérance des plantes de culture est le Cloquintocet-mexyl.

15. Agent selon l'une quelconque des revendications 12 ou 13, dans lequel le composé améliorant la tolérance des plantes de culture est le Mefenpyr-diethyl.

16. Procédé pour lutter contre une croissance non souhaitée de végétaux, **caractérisé en ce qu'**on laisse agir un agent selon la revendication 12 sur les végétaux ou leur environnement.

17. Utilisation d'un agent selon la revendication 12 pour lutter contre la croissance non souhaitée de végétaux.

18. Procédé pour lutter contre une croissance non souhaitée de végétaux, **caractérisé en ce qu'**on laisse agir un composé de formule (I) selon la revendication 1 et le composé améliorant la tolérance des plantes de culture selon la revendication 12 séparément, dans une succession proche dans le temps, sur les végétaux ou leur environnement.

19. Composés de formule (II) 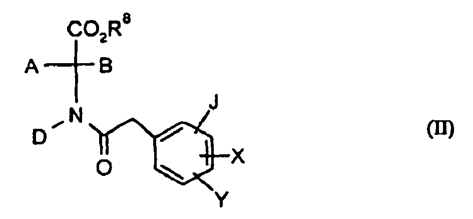 dans laquelle
A, B, D, J, X et Y et R⁸ présentent les significations indiquées ci-dessus.

20. Composés de formule (XV) 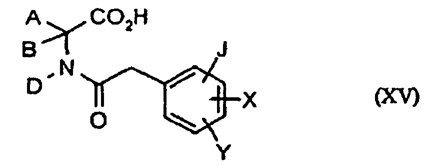 dans laquelle
A, B, D, J, X et Y présentent les significations indiquées ci-dessus.

21. Composés de formule (XIV) 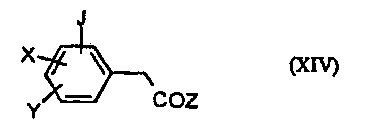 dans laquelle
Z représente un groupe partant introduit par un réactif d'activation d'acide carboxylique, tel que le carbonyldiimidazole, des carbonyldiimides (tels que par exemple le dicyclohexylcarbodiimide), des réactifs de phosphorylation (tels que par exemple POCl₃ BOP-Cl), des agents d'halogénation tels que par exemple le chlorure de thionyle, le chlorure d'oxalyle, le phosgène ou un ester de l'acide chloroformique,
J, X et Y présentent les significations indiquées ci- dessus.

22. Composés de formule (XIX) 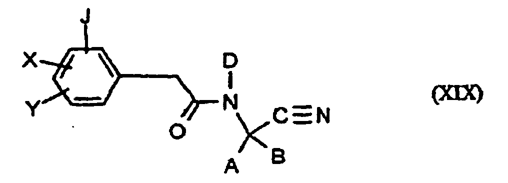 dans laquelle
A, B, D, J, X et Y présentent les significations indiquées ci-dessus.

23. Composés de formule (XVII) 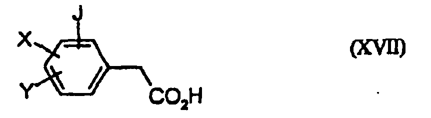 dans laquelle
J, X et Y présentent la signification indiquée dans le
tableau
| J | X | Y |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-Cl | 6-Cl |
| 4-OCF₃ | 2-OCH₃ | 6-Cl |
| 4-OCF₃ | 2-OCH₃ | 6-Br |
| 4-OCF₃ | 2-CH₃ | 6-CH₃ |
| 4-OCF₃ | 2-Cl | 6-CH₃ |
| 2-OCF₃ | 6-Cl | 4-Br |
| 2-OCF₃ | 6-CH₃ | 4-CH₃ |
| 2-OCF₃ | 6-OCH₃ | 4-Cl |
| 2-OCF₃ | 6-Cl | 4-CH₃ |
| 2-OCF₃ | 6-C₂H₅ | 4-Cl |

24. Composés de formule (XX) 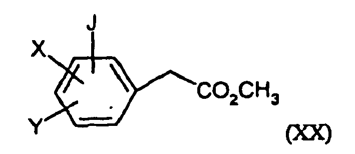 dans laquelle J, X et Y présentent les significations indiquées dans le tableau
| J | X | Y |
|---|---|---|
| 4-OCF₃ | 2-Cl | 6-Cl |
| 4-OCF₃ | 2-Br | 6-Cl |
| 4-OCF₃ | 2-Br | 6-Br |
| 4-OCF₃ | 2-OCH₃ | 6-Br |
| 4-OCF₃ | 2-OCH₃ | 6-Cl |
| 4-OCF₃ | 2-CH₃ | 6-CH₃ |
| 4-OCF₃ | 2-Br | 6-Cl |
| 4-OCF₃ | 2-Cl | 6-CH₃ |
| 2-OCF₃ | 6-Br | 4-Br |
| 2-OCF₃ | 6-CH₃ | 9-cl3 |
| 2-OCF₃ | 6-OCH₃ | 4-Cl |
| 2-OCF₃ | 6-CH₃ | 4-Cl |
| 2-OCF₃ | 6-Cl | 4-CH₃ |
| 2-OCF₃ | 6-Cl | 4-Br |

25. Composition comprenant
au moins un composé de formule (I) selon la revendication 1 ou un agent selon la revendication 12 et
au moins un sel de formule (III') 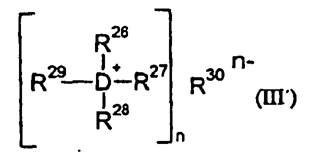 dans laquelle
D représente azote ou phosphore,
R²⁶, R²⁷, R²⁸ et R²⁹ représentent, indépendamment l'un de l'autre, hydrogène ; ou C₁-C₈-alkyle à chaque fois le cas échéant substitué ou C₁-C₈- alkylène monoinsaturé ou polyinsaturé, le cas échéant substitué, les substituants pouvant être choisis parmi halogène, nitro et cyano,
n représente 1, 2, 3 ou 4,
R³⁰ représente un anion inorganique ou organique.

26. Composition selon la revendication 25, **caractérisée en ce qu'**elle contient au moins un agent favorisant la pénétration.

27. Procédé pour augmenter l'action d'agents de lutte contre les organismes nuisibles et/ou d'herbicides contenant une substance active de formule (I) selon la revendication 1 ou un agent selon la revendication 12, **caractérisé en ce que** l'agent prêt à l'emploi (bouillon à pulvériser) est préparé avec utilisation d'un sel de formule (III') selon la revendication 25.

28. Procédé selon la revendication 27, **caractérisé en ce que** le bouillon à pulvériser est préparé avec utilisation d'un agent favorisant la pénétration.
